(19) Europäisches Patentamt
European Patent Office
Office européen des brevets

(11) Veröffentlichungsnummer: **0 213 080 B1**

# (12) EUROPÄISCHE PATENTSCHRIFT

(45) Veröffentlichungstag der Patentschrift: **20.05.92**

(51) Int. Cl.⁵: **C07D 211/60**, C07D 211/78, A61K 31/445

(21) Anmeldenummer: **86810375.5**

(22) Anmeldetag: **21.08.86**

## (54) Hydropyridin-Derivate.

(30) Priorität: **27.08.85 CH 3669/85**
**26.06.86 CH 2586/86**

(43) Veröffentlichungstag der Anmeldung:
**04.03.87 Patentblatt 87/10**

(45) Bekanntmachung des Hinweises auf die Patenterteilung:
**20.05.92 Patentblatt 92/21**

(84) Benannte Vertragsstaaten:
**AT BE CH DE FR GB IT LI LU NL SE**

(56) Entgegenhaltungen:
**EP-A- 0 011 447**
**EP-A- 0 018 417**
**EP-A- 0 066 456**
**GB-A- 932 487**

(73) Patentinhaber: **CIBA-GEIGY AG**
**Klybeckstrasse 141**
**CH-4002 Basel(CH)**

(72) Erfinder: **von Sprecher, Georg, Dr.**
**Bettenstrasse 64**
**CH-4123 Allschwil(CH)**
Erfinder: **Fröstl, Wolfgang, Dr.**
**St. Johanns-Vorstadt 6/2**
**CH-4056 Basel(CH)**
Erfinder: **Züst, Armin, Dr.**
**Sternengasse 27/408**
**CH-4051 Basel(CH)**

EP 0 213 080 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue Hydropyridin-Derivate der Formel

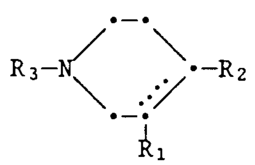 (I),

worin $R_1$ Carboxy, Niederalkoxycarbonyl, Carbamoyl, N-Niederalkylcarbamoyl oder N,N-Diniederalkylcarbamoyl bedeutet, $R_2$ Wasserstoff, eine gegebenenfalls verätherte oder acylierte Hydroxygruppe oder eine gegebenenfalls acylierte Aminogruppe bedeutet, $R_3$ einen Rest der Formeln R- (Ia), R-$alk_1$- (Ib) oder R'=$alk_2$- (Ic) bedeutet, worin R einen unsubstituierten oder im Benzoteil ein- oder mehrfach durch Hydroxy, Niederalkoxy, Niederalkanoyloxy, Halogen, Niederalkyl und/oder Trifluormethyl und/oder in $\alpha$-Stellung zum Benzoteil durch Niederalkyl substituierten, über ein gesättigtes C-Atom gebundenen Benzocycloalkenylrest mit insgesamt 8 bis 12 Ring-C-Atomen bedeutet, R' einen unsubstituierten oder im Benzoteil ein- oder mehrfach durch Hydroxy, Niederalkoxy, Niederalkanoyloxy, Halogen, Niederalkyl und/oder Trifluormethyl substituierten Benzocycloalkenylidenrestmit insgesamt 8 bis 12 Ring-C-Atomen bedeutet, $alk_1$ Niederalkylen oder Niederalkyliden ist, $alk_2$ Niederalkyl-$\omega$-yliden bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und ihre Tautomeren und/oder Salze, die Verwendung dieser Verbindungen, Tautomeren und Salze, ein Verfahren zu ihrer Herstellung und pharmazeutische Zusammensetzungen, enthaltend eine Verbindung der Formel I oder ein Tautomeres und/oder ein pharmazeutisch verwendbares Salz davon.

Der Rest R bzw. R' kann einen oder mehrere, z.B. einen oder zwei oder drei, gleiche oder verschiedene der genannten Substituenten aufweisen.

Reste R gemäss der vorstehenden Definition sind beispielsweise Benzocyclobutenylreste, z.B. Benzocyclobuten-1-yl, Indanylreste, z.B. Indan1-yl- oder in zweiter Linie Indan-2-ylreste, oder 1,2,3,4-Tetrahydronaphthylreste, z.B. 1,2,3,4-Tetrahydronaphth-1-yl oder in zweiter Linie 1,2,3,4-Tetrahydronaphth-2-yl.

Reste R' gemäss der vorstehenden Definition sind beispielsweise Benzocyclobutenylidenreste, z.B. Benzocyclobuten-1-yliden.

Die Erfindung betrifft beispielsweise neue Hydropyridin-Derivate der Formel I, worin $R_3$ eine Gruppe Ia oder Ib ist, R einen unsubstituierten oder im Benzoteil ein- oder mehrfach durch Hydroxy, Niederalkoxy, Niederalkanoyloxy, Halogen, Niederalkyl und/oder Trifluormethyl und/oder in $\alpha$-Stellung zum Benzoteil durch Niederalkyl substituierten Benzocyclobuten-1-ylrest bedeutet, $R_1$ Carboxy, Niederalkoxycarbonyl, Carbamoyl, N-Niederalkylcarbamoyl oder N,N-Diniederalkylcarbamoyl bedeutet, $R_2$ Wasserstoff, eine gegebenenfalls verätherte oder acylierte Hydroxygruppe oder eine gegebenenfalls acylierte Aminogruppe bedeutet, $alk_1$ Niederalkylen oder Niederalkyliden bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und ihre Tautomeren und/oder Salze.

Veräthertes Hydroxy $R_2$ ist beispielsweise Niederalkoxy oder gegebenenfalls substituiertes Phenylniederalkoxy.

Acyl in acyliertem Hydroxy bzw. Amino $R_2$ ist beispielsweise von einer organischen Carbon- oder Sulfonsäure oder einem Halbester der Kohlensäure abgeleitetes Acyl.

Von organischen Carbonsäuren abgeleitetes Acyl ist beispielsweise der Rest einer aliphatischen oder monocyclisch-aromatischen Carbonsäure, wie Niederalkanoyl oder gegebenenfalls substituiertes Benzoyl, ferner Pyridoyl.

Von organischen Sulfonsäuren abgeleitetes Acyl ist beispielsweise Niederalkansulfonyl.

In von Halbestern der Kohlensäure abgeleitetem Acyl ist die zweite Hydroxygruppe beispielsweise mit einem aliphatischen oder aryl-, wie phenyl-aliphatischen Alkohol verestert. Als von Halbestern der Kohlensäure abgeleitete Acylgruppen seien beispielsweise Niederalkoxycarbonyl und gegebenenfalls substituiertes Phenylniederalkoxycarbonyl genannt.

Tautomere Formen von Verbindungen der Formel I existieren z.B. dann, wenn $R_2$ Hydroxy oder Amino bedeutet und die gestrichelte Linie zum Ausdruck bringen soll, dass zwischen den Kohlenstoffatomen, welche die Reste $R_1$ und $R_2$ tragen, eine Doppelbindung vorliegt. D.h., die Enole bzw. Enamine der Formel I stehen im Gleichgewicht mit den entsprechenden Keto- bzw. Ketimin-Tautomeren der Formel

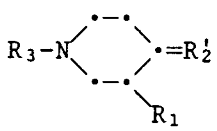

(I'),

worin $R_2'$ Oxo oder Imino bedeutet. Vertreter beider tautomerer Formen können isoliert werden.

Die erfindungsgemässen Verbindungen können ferner in Form von Stereoisomeren vorliegen. Wenn die Verbindungen der Formel I ein chirales C-Atom aufweisen (z.B. das C-Atom 3 eines 4-unsubstituierten Piperidinrestes), können sie z.B. als reine Enantiomere oder Enantiomerengemische, wie Racemate, und, sofern noch mindestens ein weiteres chirales Zentrum vorhanden ist (z.B. das C-Atom 4 eines 4-substituierten Piperidinrestes), als Diastereomere oder Diastereomerengemische vorliegen. So können z.B. im Hinblick auf $R_1$ und $R_2$ geometrische Isomere, wie cis- und trans-Isomere, gebildet werden, falls $R_2$ von Wasserstoff verschieden ist.

Salze von Verbindungen der Formel I bzw. deren Tautomeren sind insbesondere entsprechende Säureadditionssalze, vorzugsweise pharmazeutisch verwendbare Säureadditionssalze. Diese werden beispielsweise mit starken anorganischen Säuren, wie Mineralsäuren, z.B. Schwefelsäure, einer Phosphorsäure oder einer Halogenwasserstoffsäure, mit starken organischen Carbonsäuren, wie Niederalkancarbonsäuren, z.B. Essigsäure, wie gegebenenfalls ungesättigten Dicarbonsäuren, z.B. Malon-, Malein- oder Fumarsäure, oder wie Hydroxycarbonsäuren, z.B. Wein- oder Zitronensaure, oder mit Sulfonsäuren, wie Niederalkan- oder gegebenenfalls substituierten Benzolsulfonsäuren, z.B. Methan- oder p-Toluolsulfonsäure, gebildet. Bedeutet $R_1$ beispielsweise Carboxy, können entsprechende Verbindungen Salze mit Basen bilden. Geeignete Salze mit Basen sind beispielsweise entsprechende Alkalimetall- oder Erdalkalimetallsalze, z.B. Natrium-, Kalium- oder Magnesiumsalze, pharmazeutisch verwendbare Uebergangsmetallsalze, wie Zink- oder Kupfersalze, oder Salze mit Ammoniak oder organischen Aminen, wie cyclische Amine, wie Mono-, Di- bzw. Triniederalkylamine, wie Hydroxyniederalkylamine, z.B. Mono-, Di- bzw. Trihydroxyniederalkylamine, Hydroxyniederalkyl-niederalkyl-amine oder Polyhydroxyniederalkylamine. Cyclische Amine sind z.B. Morpholin, Thiomorpholin, Piperidin oder Pyrrolidin. Als Mononiederalkylamine kommen beispielsweise Aethyl- oder t-Butylamin, als Diniederalkylamine beispielsweise Diäthyl- oder Diisopropylamin, als Triniederalkylamine beispielsweise Trimethyl- oder Triäthylamin in Betracht. Entsprechende Hydroxyniederalkylamine sind z.B. Mono-, Di- bzw. Triäthanolamin, und Hydroxyniederalkyl-niederalkylamine sind z.B. N,N-Dimethylamino- oder N,N-Diäthylaminoäthanol; als Polyhydroxyniederalkylamin kommt z.B. Glucosamin in Frage.

Umfasst sind ferner für pharmazeutische Verwendungen ungeeignete Salze, da diese beispielsweise für die Isolierung bzw. Reinigung freier erfindungsgemässer Verbindungen sowie deren pharmazeutisch verwendbarer Salze verwendet werden können.

Vor- und nachstehend sind unter mit "Nieder" bezeichneten Resten oder Verbindungen, sofern nicht abweichend definiert, solche zu verstehen, die bis und mit 7, vor allem bis und mit 4, Kohlenstoffatome (C-Atome) enthalten.

Niederalkoxy ist z.B. Methoxy, Aethoxy, n-Propyloxy, Isopropyloxy, n-Butyloxy, Isobutyloxy oder tert.-Butyloxy.

Niederalkyl ist z.B. Methyl, Aethyl, n-Propyl, Isopropyl, n-Butyl, Isobutyl, sek-Butyl oder tert.-Butyl und umfasst ferner entsprechende Pentyl-, Hexyl- und Heptylreste.

Niederalkylen $alk_1$ überbrückt die beiden Ringsysteme in erster Linie durch 1 bis und mit 3 C-Atome und weist insbesondere 1 bis und mit 4, vor allem 2 bis und mit 3, C-Atome auf und ist z.B. geradkettig, wie Methylen, Aethylen oder 1,3-Propylen, oder verzweigt, wie 1,2-Propylen, 1,2- oder 1,3-(2-Methyl)propylen oder 1,2- oder 1,3-Butylen.

Niederalkyliden $alk_1$ überbrückt die beiden Ringsysteme durch 1 C-Atom und weist insbesondere 1 bis und mit 4, vor allem 1 bis und mit 3,C-Atome auf und ist z.B. Methylen, Aethyliden, 1,1- oder 2,2-Propyliden oder 1,1- oder 2,2-Butyliden.

Niederalkyl-ω-yliden $alk_2$ überbrückt die beiden Ringsysteme in erster Linie durch 2 bis und mit 3 C-Atome und weist insbesondere 2 bis und mit 4, vor allem 2 oder 3, C-Atome auf und ist z. B. Aethyl-2-yliden oder Propyl-3-yliden.

Niederalkanoyloxy ist z.B. Acetoxy, Propionyloxy, Butyryloxy, Isobutyryloxy oder Pivaloyloxy.

Niederalkansulfonyl ist z.B. Methan- oder Aethansulfonyl; Phenylniederalkoxy ist beispielsweise gegebenenfalls durch Niederalkyl, Niederalkoxy und/oder Halogen substituiertes α-Phenylniederalkoxy mit bis und mit 4 Alkyl-C-Atomen, insbesondere Benzyloxy.

Halogen ist insbesondere Halogen mit einer Atomnummer bis und mit 35, wie Fluor, Chlor oder Brom, und umfasst ferner Iod.

Aus der europäischen Patentanmeldung mit der Publikationsnummer 11,447 sind 1-Benzyl-1,2,5,6-tetrahydropyridin-3-carbonsäuren und -carbonsäurederivate bekannt, denen eine Hemmwirkung auf die Blutplättchen-Aggregation zugeschrieben wird. In der europäischen Patentanmeldung mit der Publikationsnummer 66,456 werden Piperidin- und 1,2,5,6-tetrahydropyridin-verbindungen offenbart, die innerhalb bestimmter Teilstrukturen eine gewisse Aehnlichkeit mit den erfindungsgemässen Verbindungen der Formel I aufweisen und als Inhibitoren der Aufnahme von gamma-Aminobuttersäure (GABA) sowie als Analgetika und als Sedativa beschrieben werden.

Die Verbindungen der Formel I, ihre Tautomeren und ihre pharmazeutisch verwendbaren Salze weisen beispielsweise wertvolle pharmakologische, insbesondere nootrope Eigenschaften auf. So bewirken sie z.B. im Two-Compartment Passive Avoidance Testmodell nach Mondadori und Classen, Acta Neurol. Scand. 69, Suppl. 99, S. 125-129 (1984) in Dosismengen ab etwa 0,1 mg/kg i.p. sowie p.o. bei der Maus eine Reduktion der amnesischen Wirkung eines zerebralen Elektroschocks.

Ebenso besitzen die erfindungsgemässen Verbindungen eine beträchtliche gedächtnisverbessernde Wirkung, die im Step-down Passive Avoidance Test nach Mondadori und Waser, Psychopharmacol. 63, S. 297-300 (1979) ab einer Dosis von etwa 0,1 mg/kg i.p. sowie p.o. an der Maus festzustellen ist.

Entsprechend können die Verbindungen der Formel I bzw. deren Tautomere und ihre pharmazeutisch verwendbaren Salze als Pharmazeutika, z.B. Nootropika, beispielsweise zur therapeutischen und prophylaktischen Behandlung von Gedächtnisstörungen, verwendet werden. Ein weiterer Gegenstand der Erfindung ist somit die Verwendung von Verbindungen der Formel I, ihrer Tautomeren und ihrer pharmazeutisch verwendbaren Salze zur Herstellung von Arzneimitteln, insbesondere von Nootropika, zur Behandlung von Gedächtnisstörungen. Dabei kann auch die gewerbsmässige Herrichtung der Wirksubstanzen eingeschlossen sein.

Die Erfindung betrifft insbesondere Verbindungen der Formel I, worin $R_3$ eine Gruppe Ia, Ib oder Ic ist, R einen unsubstituierten oder im Benzoteil ein- oder mehrfach, z.B. zwei- oder dreifach, durch Hydroxy, Niederalkoxy, Niederalkanoyloxy und/oder Halogen und/oder in α-Stellung zum Benzoteil durch Niederalkyl substituierten Benzocyclobuten-1-ylrest, Indan-1-yl- oder Indan-2-ylrest oder 1,2,3,4-Tetrahydronaphth-1-yl- oder 1,2,3,4-Tetrahydronaphth-2-ylrest bedeutet, R' einen unsubstituierten oder im Benzoteil ein- oder mehrfach, z.B. zwei- oder dreifach, durch Hydroxy, Niederalkoxy, Niederalkanoyloxy und/oder Halogen substituierten Benzocyclobuten-1-ylidenrest bedeutet, $R_1$ Carboxy, Niederalkoxycarbonyl oder Carbamoyl bedeutet, $R_2$ Wasserstoff, Hydroxy, Niederalkoxy, Niederalkanoyloxy, Niederalkansulfonyloxy, gegebenenfalls im Phenylteil durch Niederalkyl, Niederalkoxy und/oder Halogen substituiertes Phenylniederalkoxy oder Amino bedeutet, $alk_1$ Niederalkylen oder Niederalkyliden, das die beiden Ringsysteme durch 1 bis und mit 3 C-Atome bzw. durch 1 C-Atom verbindet, bedeutet, $alk_2$ Niederalkyl-ω-yliden, das die beiden Ringsysteme durch 2 oder 3 C-Atome verbindet, darstellt und die gestrichelte Linie zum Ausdruck bringen soll, dass zwischen den C-Atomen, welche die Reste $R_1$ und $R_2$ tragen, eine Einfach- oder eine Doppelbindung vorliegt, und ihre Tautomeren und/oder Salze.

Die Erfindung betrifft insbesondere beispielsweise Verbindungen der Formel I, worin $R_3$ eine Gruppe der Formel Ib darstellt, R einen unsubstituierten oder im Benzoteil ein- oder mehrfach, z.B. zwei-oder dreifach, durch Hydroxy, Niederalkoxy, Niederalkanoyloxy und/oder Halogen und/oder in 1-Stellung durch Niederalkyl substituierten Benzocyclobuten-1-ylrest bedeutet, $R_1$ Carboxy, Niederalkoxycarbonyl oder Carbamoyl bedeutet, $R_2$ Wasserstoff, Hydroxy, Niederalkoxy, Niederalkanoyloxy oder Amino bedeutet, $alk_1$ Niederalkylen oder Niederalkyliden bedeutet, das die beiden Ringsysteme durch 1 bis und mit 3 C-Atome bzw. durch 1 C-Atom verbindet, und die gestrichelte Linie zum Ausdruck bringen soll, dass zwischen den C-Atomen, welche die Reste $R_1$ und $R_2$ tragen, eine Einfach- oder eine Doppelbindung vorliegt, und ihre Tautomeren und/oder Salze.

Die Erfindung betrifft vor allem Verbindungen der Formel I, worin $R_3$ eine Gruppe Ia oder Ib darstellt, R einen unsubstituierten oder im Benzoteil durch Niederalkoxy mit bis und mit 4 C-Atomen, wie Methoxy, Niederalkyl mit bis und mit 4 C-Atomen, wie Methyl, oder durch Halogen der Atomnummer bis und mit 35, wie Chlor, monosubstituierten oder durch Niederalkyl mit bis und mit 4 C-Atomen, wie Methyl, oder durch Niederalkoxy mit bis und mit 4 C-Atomen, wie Methoxy, sowie Niederalkyl mit bis und mit 4 C-Atomen, wie Methyl, disubstituierten Benzocyclobuten-1-ylrest, Indan-1-ylrest, Indan-2-ylrest oder 1,2,3,4-Tetrahydronaphth-1-ylrest bedeutet, $R_1$ Niederalkoxycarbonyl, insbesondere mit 1 bis und mit 4 C-Atomen

im Niederalkoxyteil, wie Aethoxycarbonyl, oder Carbamoyl bedeutet, $R_2$ Wasserstoff oder Hydroxy bedeutet, $alk_1$ für Niederalkylen, welches die Ringsysteme durch 1 bis und mit 3 C-Atome überbrückt, insbesondere mit bis und mit 3 C-Atomen, wie Methylen, steht und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und ihre Tautomeren und/oder Salze.

Die Erfindung betrifft vor allem beispielsweise Verbindungen der Formel I, worin $R_3$ eine Gruppe der Formel Ib darstellt, R einen unsubstituierten oder im Benzoteil durch Niederalkoxy mit bis und mit 4 C-Atomen, wie Methoxy, substituierten Benzocyclobuten-1-ylrest bedeutet, $R_1$ Niederalkoxycarbonyl, insbesondere mit 1 bis und mit 4 C-Atomen im Niederalkoxyteil, wie Aethoxycarbonyl, oder Carbamoyl bedeutet, $R_2$ Wasserstoff oder Hydroxy bedeutet, $alk_1$ für Niederalkylen, welches die Ringsysteme durch 1 bis und mit 3 C-Atome überbrückt, insbesondere mit bis und mit 3 C-Atomen, wie Methylen, steht und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und ihre Tautomeren und/oder Salze.

Die Erfindung betrifft in erster Linie Verbindungen der Formel I, worin $R_3$ eine Gruppe der Formel Ib darstellt, R einen unsubstituierten oder im Benzoteil, insbesondere in 5-Stellung, durch Niederalkoxy mit bis und mit 4 C-Atomen, wie Methoxy, monosubstituierten Benzocyclobuten-1-ylrest bedeutet, $R_1$ Niederalkoxycarbonyl mit 1 bis und mit 4 C-Atomen im Niederalkoxyteil, wie Aethoxycarbonyl, bedeutet, $R_2$ Hydroxy bedeutet, $alk_1$ Methylen oder Aethylen bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und ihre Tautomeren und/oder Salze.

Die Erfindung betrifft vorzugsweise die in den Beispielen genannten neuen Verbindungen der Formel I und ihre Salze.

Ebenfalls Gegenstand der vorliegenden Erfindung ist ein Verfahren zur Herstellung von Verbindungen der Formel I oder ihrer Tautomeren und/oder Salze, z.B. dadurch gekennzeichnet, dass man

a) eine Verbindung der Formel

$R_3$-$X_1$ (IIa)

oder ein Salz davon, worin $X_1$ Hydroxy oder reaktionsfähiges verestertes Hydroxy bedeutet, mit einer Verbindung der Formel

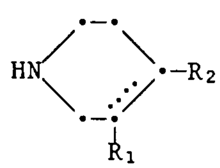

(IIb)

oder einem Tautomeren und/oder Salz davon umsetzt oder

b) in einer Verbindung der Formel

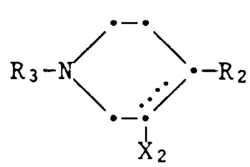

(III)

oder einem Tautomeren und/oder Salz davon, worin $X_2$ einen in $R_1$ überführbaren Rest bedeutet, $X_2$ in $R_1$ überführt oder

c) zur Herstellung von Verbindungen der Formel I, worin $R_2$ Hydroxy oder Amino bedeutet, und worin $R_1$ insbesondere Niederalkoxycarbonyl bedeutet, oder ihrer Tautomeren und/oder Salze eine Verbindung der Formel

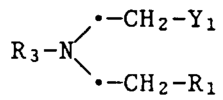

(IV),

worin $Y_1$ eine Gruppe der Formel $-CH=R_2'$ , $-C(Y_2)=R_2'$ oder Cyano und $R_2'$ Oxo oder Imino darstellt, wobei $Y_2$ einen abspaltbaren Rest darstellt, oder ein Salz davon cyclisiert oder

d) zur Herstellung von Verbindungen der Formel I, worin $R_2$ Hydroxy oder Amino bedeutet und die gestrichelte Linie zum Ausdruck bringen soll, dass zwischen den C-Atomen, welche die Reste $R_1$ und $R_2$ tragen, eine Doppelbindung vorliegt, und worin $R_1$ insbesondere Niederalkoxycarbonyl bedeutet, oder ihren Tautomeren und/oder Salzen eine Verbindung der Formel

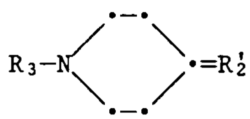

(Va),

worin $R_2'$ Oxo oder Imino ist, oder ein Tautomeres oder Salz davon mit einer Verbindung der Formel

$X_3$-$R_1$ (Vb),

worin $X_3$ Halogen oder Niederalkoxy bedeutet, oder einem Salz davon, umsetzt oder

e) zur Herstellung von Verbindungen der Formel I, worin $R_2$ von Wasserstoff verschieden ist, oder ihrer Tautomeren und/oder Salze in einer Verbindung der Formel

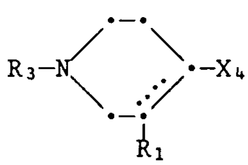

(VI)

oder einem Salz davon, worin $X_4$ einen in $R_2$ überführbaren Rest bedeutet, $X_4$ in $R_2$ überführt oder

f) zur Herstellung von Verbindungen der Formel I, worin $R_2$ Wasserstoff bedeutet, oder ihrer Tautomeren und/oder Salze in einer Verbindung der Formel

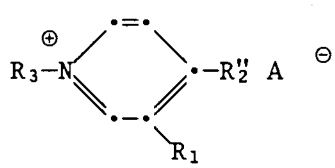

(VII),

worin $A^{\ominus}$ für das Anion einer Säure steht, und $R_2''$ Wasserstoff bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, ein verfahrensgemäss erhältliches Isomerengemisch in die Komponenten auftrennt, ein verfahrensgemäss erhältliches Enantiomeren-bzw. Diastereomerengemisch in die Enantiomeren bzw. Diastereomeren aufspaltet und/oder eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

Die vor- und nachstehend beschriebenen Umsetzungen werden in an sich bekannter Weise durchgeführt, z.B. in Ab- oder üblicherweise in Anwesenheit eines geeigneten Lösungs- oder Verdünnungsmittels oder eines Gemisches derselben, wobei man je nach Bedarf unter Kühlen, bei Raumtemperatur oder unter

Erwärmen z.B. in einem Temperaturbereich von etwa -10° bis zur Siedetemperatur des Reaktionsmediums, vorzugsweise von etwa 20° bis etwa 150°C, und, falls erforderlich, in einem geschlossenen Gefäss, unter Druck, in einer Inertgasatmosphäre und/oder unter wasserfreien Bedingungen arbeitet.

Ausgangsmaterial mit basischen Zentren kann z.B. in Form von Säureadditionssalzen, beispielsweise mit den vorstehend aufgeführten Säuren, vorliegen, während Ausgangsverbindungen mit sauren Gruppen, Salze mit Basen, z.B. der vorstehend genannten Art, bilden können. Weiter können Ausgangsverbindungen in Form von Tautomeren vorliegen, insbesondere Verbindungen der Formel IIb, worin $R_2$ Hydroxy bedeutet und die gestrichelte Linie zum Ausdruck bringen soll, dass zwischen den C-Atomen, welche $R_1$ und $R_2$ tragen, eine Doppelbindung vorliegt.

Variante a):

Reaktionsfähiges verestertes Hydroxy $X_1$ bedeutet insbesondere mit einer starken anorganischen Säure oder organischen Sulfonsäure verestertes Hydroxy, beispielsweise Halogen, wie Chlor, Brom oder Iod, Sulfonyloxy, wie Hydroxysulfonyloxy, Halogensulfonyloxy, z.B. Fluorsulfonyloxy, gegebenenfalls, z.B. durch Halogen, substituiertes Niederalkansulfonyloxy, z.B. Methan- oder Trifluormethansulfonyloxy, Cycloalkansulfonyloxy, z.B. Cyclohexansulfonyloxy, oder gegebenenfalls, z.B. durch Niederalkyl oder Halogen, substituiertes Benzolsulfonyloxy, z.B. p-Bromphenyl- oder p-Toluolsulfonyloxy.

Die Umsetzung wird insbesondere in Gegenwart eines Kondensationsmittels, wie einer geeigneten Base, durchgeführt. Als Basen kommen beispielsweise Alkalimetallhydroxide, -hydride, -amide, -alkanolate, -carbonate, -triphenylmethylide, -diniederalkylamide, -aminoniederalkylamide oder -niederalkylsilylamide, Naphthalinamine, Niederalkylamine, basische Heterocyclen, Ammoniumhydroxide sowie carbocyclische Amine in Frage. Beispielhaft seien Natriumhydroxid, -hydrid, -amid, -äthylat, Kalium-tert-butylat, -carbonat, Lithiumtriphenylmethylid, Lithium-diisopropylamid, Kalium-3-(aminopropyl)-amid, -bis-(trimethylsilyl)-amid, Dimethylaminonaphthalin, Di- oder Triäthylamin, Pyridin, Benzyl-trimethylammoniumhydroxid, 1,5-Diaza-bicyclo[4.3.0]non-5-en (DBN) sowie 1,8-Diaza-bicyclo[5.4.0]undec-7-en (DBU) genannt.

Die Ausgangsstoffe der Formel IIa sind teilweise bekannt. Neue Verbindungen IIa, worin $R_3$ eine Gruppe Ib bedeutet, werden z.B. erhalten, indem man eine entsprechende $\omega$-R-Alkancarbonsäure mit Diboran oder einen $\omega$-R-Alkancarbonsäureester mit Lithiumaluminiumhydrid jeweils zum entsprechenden Alkanol (IIa; $X_1$ = Hydroxy) reduziert und erforderlichenfalls die alkoholische Hydroxygruppe reaktionsfähig verestert, z.B. mittels Methansulfonsäurechlorid.

Die Ausgangsmaterialien der Formel IIb sind bekannt bzw. in an sich bekannter Weise herstellbar.

Variante b):

Ein in $R_1$ überführbarer Rest $X_2$ ist beispielsweise von $R_1$ verschiedenes funktionell abgewandeltes Carboxy, wie Cyano, anhydridisiertes Carboxy, gegebenenfalls substituiertes Amidino, gegebenenfalls verestertes oder anhydridisiertes Carboximidoyl, von verestertem oder amidiertem Carboxy $R_1$ verschiedenes, verestertes oder amidiertes Carboxy, Triniederalkoxy- oder Trihalogenmethyl.

Anhydridisiertes Carboxy ist beispielsweise mit einer Mineralsäure, wie Halogenwasserstoffsäure, oder mit einer Carbonsäure, wie einer gegebenenfalls substituierten Niederalkan- bzw. Benzoesäure oder einem Halogenameisensaure-niederalkylhalbester anhydridisiertes Carboxy. Als Beispiele seien Halogencarbonyl, wie Chlorcarbonyl, Niederalkanoyloxycarbonyl, wie Acetyloxycarbonyl, oder Niederalkoxycarbonyloxycarbonyl, wie Aethoxycarbonyloxycarbonyl, genannt.

Substituiertes Amidino ist beispielsweise mit einem aliphatischen Rest, z.B. Niederalkyl, substituiertes Amidino, wie Niederalkylamidino, z.B. Aethylamidino.

Unter verestertem oder anhydridisiertem Carboximidoyl ist z.B. Alkoxy- bzw. Halogencarbimidoyl, beispielsweise Niederalkoxy-, wie Aethoxy- bzw. Chlor-carbimidoyl, zu verstehen.

Triniederalkoxy- bzw. Trihalogenmethyl ist z.B. Trimethoxymethyl bzw. Trichlormethyl.

$X_2$ kann beispielsweise durch Solvolyse in $R_1$ überführt werden. Solvolysemittel sind beispielsweise Wasser, dem gewünschten Niederalkoxycarbonylrest $R_1$ entsprechende Niederalkanole, Ammoniak oder der gewünschten amidierten Carboxygruppe $R_1$ entsprechende Amine. Die Behandlung mit einem entsprechenden Solvolysemittel wird gegebenenfalls in Gegenwart einer Säure oder Base durchgeführt. Als Säuren kommen beispielsweise anorganische oder organische Protonsäuren, wie Mineralsäuren, z.B. Schwefel- oder Halogenwasserstoffsäure, beispielsweise Chlorwasserstoffsäure, wie Sulfonsäuren, z.B. Niederalkan- oder gegebenenfalls substituierte Benzolsulfonsäure, beispielsweise Methan- oder p-Toluolsulfonsäure, oder

wie Carbonsäuren, z.B. Niederalkancarbonsäuren, beispielsweise Essigsäure, in Frage, während als Basen beispielsweise die unter Variante a) genannten verwendet werden können, insbesondere Natrium- oder Kaliumhydroxid.

Bei der Solvolyse wird anhydridisiertes Carboxy, gegebenenfalls substituiertes Amidino, gegebenenfalls verestertes oder anhydridisiertes Carboximidoyl, von verestertem oder amidiertem Carboxy $R_1$ verschiedenes verestertes oder amidiertes Carboxy, Triniederalkoxy oder Trihalogenmethyl zu Carboxy bzw. Cyano zu Carbamoyl oder Carboxy hydrolysiert. Dabei können gegebenenfalls am Ring R befindliche Niederalkanoyloxyreste und/oder verätherte oder acylierte Hydroxygruppen bzw. acylierte Aminogruppen $R_2$ im Verlauf der Hydrolyse zu Hydroxy bzw. Amino hydrolysiert werden.

Cyano, anhydridisiertes Carboxy bzw. von verestertem oder amidiertem Carboxy $R_1$ verschiedenes verestertes oder amidiertes Carboxy werden beispielsweise mit einem geeigneten Niederalkanol zu Niederalkoxycarbonyl $R_1$ alkoholysiert und Cyano und anhydridisiertes Carboxy beispielsweise mit Ammoniak oder einem dem amidierten Carboxy $R_1$ entsprechenden Amin ammono-bzw. aminolysiert.

Das Ausgangsmaterial der Formel III kann in Analogie zu der unter Variante a) beschriebenen Weise durch Umsetzung einer Verbindung der Formel $R_3$-$X_1$ (IIa) mit einer Verbindung der Formel

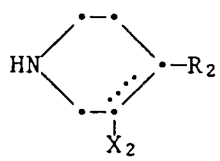 (IIIa)

oder einem Tautomeren und/oder Salz davon in Gegenwart einer der genannten Basen hergestellt werden.

Variante c):

Die Cyclisierung kann beispielsweise in Analogie zur Dieckmann-Reaktion, insbesondere in Gegenwart einer der unter der Variante a) genannten Basen und unter anschliessender hydrolytischer Aufarbeitung, durchgeführt werden.

In einer bevorzugten Ausführungsform kann man beispielsweise eine Verbindung der Formel

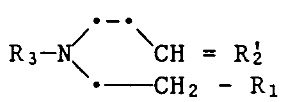 (IVa),

worin $R_2$' Oxo oder Imino bedeutet, der Behandlung mit einer der genannten Basen, insbesondere mit einem Alkalimetallniederalkanolat, z.B. mit Natriummethanolat oder Natriumäthanolat, unterwerfen. Dabei cyclisiert die Verbindung IVa zu einer Verbindung der Formel I, worin die gestrichelte Linie anzeigt, dass zwischen den C-Atomen, welche $R_1$ und $R_2$ tragen, eine Einfachbindung vorliegt, und worin $R_2$ Hydroxy oder Amino bedeutet. Ausgangsstoffe IVa werden z.B. erhalten, indem man einen reaktionsfähigen Benzocycloalkenyl(iden)alkanolester der Formel

$R_3$-$X_1$ (IIa),

worin $X_1$ reaktionsfähiges verestertes Hydroxy ist, mit einer $\beta$-Aminosäureverbindung der Formel $H_2N$-$CH_2$-$CH_2$-$R_1$ (IVb) und das erhaltene 3-Aminopropionsäurederivat der Formel

$$R_3 - \underset{H}{N} - CH_2CH_2 - R_1 \quad (IVc)$$

mit Acrolein oder einem gegebenenfalls funktionell abgewandelten Aldehyd der Formel $Y_1$-$CH_2$-$CH_2$-$CH=R_2$' (IVd; $Y_1$ = reaktionsfähiges verestertes Hydroxy; $R_2$' = Oxo oder Imino) umsetzt.

In einer anderen bevorzugten Ausführungsform der Variante c) cyclisiert man eine Verbindung der Formel IV, worin $Y_1$ und $R_1$ Niederalkoxycarbonyl bedeuten, zu der entsprechenden Verbindung der Formel I, worin die gestrichelte Linie anzeigt, dass zwischen den C-Atomen, welche $R_1$ und $R_2$ tragen, eine Doppelbindung vorliegt, und worin $R_2$ Hydroxy ist, bzw. zum entsprechenden Tautomeren der Formel I', worin $R_2'$ Oxo ist.

Zur Herstellung der letztgenannten Ausgangsverbindungen der Formel IV kann man beispielsweise von Verbindungen der Formel $R_3$-$NH_2$ (IVe) oder deren Salzen ausgehen, welche z.B. durch Reduktion der entsprechenden Nitrile erhältlich sind, und diese mit mindestens 2 Mol einer Verbindung der Formel $CH_2$ = CH-$R_1$ (IVf) zur Reaktion bringen.

Verbindungen IVe, worin $R_3$ eine Gruppe Ib ist, werden beispielsweise erhalten, indem man eine Verbindung der Formel R-$CH_2$-$X_1$ (IVea; $X_1$ = reaktionsfähiges verestertes Hydroxy) mit einem Alkalimetallazid zur entsprechenden Verbindung der Formel R-$CH_2$-$N_3$ (IVeb) oder mit einem Alkalimetallcyanid zur entsprechenden Verbindung der Formel R-$CH_2$-CN (IVec) umsetzt und das Reaktionsprodukt zum Amin (IVe; $R_3$ = R-$CH_2$- bzw. R-$CH_2CH_2$-) reduziert, z.B. mittels Lithiumaluminiumhydrid oder im Falle von Zwischenprodukten IVec mit Wasserstoff in Gegenwart von Raney-Nickel oder mittels Boran/Dimethylsulfid.

Verbindungen IVe, worin $R_3$ eine Gruppe Ia ist, werden z.B. hergestellt, indem man eine entsprechende Carbonsäure der Formel R-COOH (IVed) mit Thionylchlorid und anschliessend mit einem Alkalimetallazid in die entsprechende Verbindung der Formel R-$CON_3$ (IVee) überführt und diese, z.B. durch Behandeln mit Trifluoressigsäure und anschliessend mit einem Alkalimetallhydroxid, zum entsprechenden Amin IVe ($R_3$ = R) abbaut.

Variante d):

Die verfahrensgemässe C-Acylierung kann insbesondere in Gegenwart einer der unter der Variante a) genannten Basen erfolgen.

Die Umsetzung von Verbindungen der Formel $R_3$-$X_1$ (IIa) mit Verbindungen der Formel

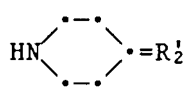

(Vc)

in Analogie zu der N-Substitution gemäss Variante a) in Gegenwart einer der erwähnten Basen führt zu dem Ausgangsmaterial der Formel Va.

Variante e):

In $R_2$ überführbare Reste $X_4$ sind beispielsweise durch Solvolyse, d.h. Umsetzung mit einer Verbindung der Formel $R_2H$ oder einem Salz davon, in $R_2$ überführbare Reste, beispielsweise reaktionsfähige veresterte Hydroxygruppen, wie Halogenatome, z.B. Chlor, Brom oder Iod. In Hydroxy überführbare Reste $X_4$ sind ferner Diazoniumgruppen, z.B. der Formel $-N_2^{\oplus}A^{\ominus}$,worin $A^{\ominus}$ das Anion einer starken Säure, wie einer Mineralsäure, z.B. das Chlorid- oder Sulfation, bedeutet.

Die Solvolyse erfolgt in üblicher Weise, beispielsweise in Gegenwart einer Base, wie eines Alkalimetall- oder Erdalkalimetallhydroxides, z.B. von Natrium- oder Kaliumhydroxid, oder einer tertiären Stickstoffbase, z.B. eines Triniederalkylamins, wie Triäthylamin, oder einer heteroaromatischen Stickstoffbase, wie Pyridin, bzw. eines quaternären Ammoniumhydroxides, wie Benzyl-trimethyl-ammoniumhydroxid, oder indem man die Verbindung $R_2H$ in Form eines Metallsalzes, z.B. der Formel $R_2^{\ominus}M^{\oplus}$, worin $M^{\oplus}$ ein Alkalimetallkation, wie das Natriumion, bedeutet, einsetzt. Vorteilhaft arbeitet man in Gegenwart eines Lösungs-oder Verdünnungsmittels, z.B. in einem Ueberschuss der Reaktionskomponente $R_2H$ und/oder einem mit dieser mischbaren inerten Lösungsmittel, erforderlichenfalls unter Kühlen oder Erwärmen, z.B. im Temperaturbereich von etwa 0° bis 120°C, und/oder unter Inertgas, wie Stickstoff.

Zur Herstellung von Ausgangsverbindungen der Formel VI und deren Salzen geht man beispielsweise von Verbindungen der Formel $R_3$-$X_1$ (IIa) aus und setzt diese mit einer entsprechenden Verbindung der Formel

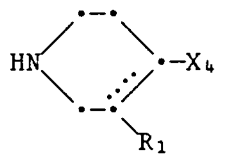

(VIa)

in Gegenwart einer der vorstehend genannten Basen um.

Variante f):

Das Anion $A^{\ominus}$ ist beispielsweise das Anion einer starken Protonsäure, z.B. ein Halogenidion, wie Chlorid-, Bromid- oder Iodidion, oder ein Sulfonation, wie ein gegebenenfalls substituiertes Niederalkan- oder Benzolsulfonation, z.B. das Methansulfonat-, Aethansulfonat- oder p-Brombenzolsulfonat- oder p-Toluolsulfonation.

Die Reduktion der überschüssigen Doppelbindungen erfolgt durch Behandeln mit einem geeigneten Reduktionsmittel, beispielsweise durch Hydrierung in Gegenwart eines Hydrierungskatalysators, durch Reduktion mit einem Hydrid-übertragenden Reagens oder durch Reduktion mit einem metallischen Reduktionssystem aus Metall und Protonen-abspaltendem Mittel.

Als Hydrierungskatalysatoren kommen z.B. Elemente der VIII. Nebengruppe des Periodensystems der Elemente oder deren Derivate in Betracht, wie Palladium, Platin, Platinoxid, Ruthenium, Rhodium, Tris-(triphenylphosphin)-rhodium-I-halogenid, z.B. -chlorid, oder Raney-Nickel, die gegebenenfalls auf einem Trägermaterial, wie Aktivkohle, Alkalimetallcarbonat bzw. -sulfat oder einem Kieselgel, aufgezogen sind. Als Hydridübertragende Reagentien kommen beispielsweise geeignete Leichtmetallhydride, insbesondere Alkalimetallaluminiumhydride bzw. -borhydride, wie Lithiumaluminiumhydrid, Lithiumtriäthylborhydrid, Natriumborhydrid, Natriumcyanoborhydrid, oder Zinnhydride, wie Triäthyl- oder Tributylzinnhydrid, oder Diboran in Frage. Der Metallbestandteil des metallischen Reduktionssystems ist beispielsweise ein unedles Metall, wie Alkali- oder Erdalkalimetall, z.B. Lithium, Natrium, Kalium, Magnesium oder Calcium, oder Uebergangsmetall, z.B. Zink, Zinn, Eisen oder Titan, während als Protonen-abspaltende Mittel z.B. Protonsäuren der vorstehend genannten Art, wie Salz- oder Essigsäure, Niederalkanole, wie Aethanol, und/oder Amine bzw. Ammoniak in Frage kommen. Solche Systeme sind beispielsweise Natrium/Ammoniak, Zink/Salzsäure, Zink/Essigsäure oder Zink/Aethanol.

Die Herstellung von Ausgangsverbindungen der Formel VII erfolgt beispielsweise durch Umsetzung von Verbindungen der Formel $R_3$-$X_1$ (IIa;$X_1$ = A) mit Verbindungen der Formel

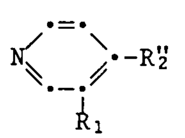

(VIIa)

oder einem Salz davon.

In den Ausgangsstoffen der Formeln IIb, III und IIIa kann eine Hydroxygruppe $R_2$ in verätherter bzw. eine Hydroxy- oder Aminogruppe $R_2$ auch in intermediär geschützter Form vorliegen. Geschütztes Hydroxy ist beispielsweise Silyloxy, wie Triniederalkylsilyloxy, z.B. Trimethylsilyloxy, kann aber auch Triphenylniederalkoxy, z.B. Trityloxy, sein. Geschütztes Amino ist beispielsweise Silylamino, wie Triniederalkylsilylamino, z.B. Trimethylsilylamino, kann aber auch Phenyl-, Diphenyl- oder Triphenylniederalkylamino, wie Benzylamino, Diphenylmethylamino oder Tritylamino, sein.

Die Freisetzung intermediär geschützter Reste, d.h. Abspaltung der intermediären Schutzgruppen, erfolgt in üblicher Weise, beispielsweise durch Solvolyse, wie milde Hydrolyse, z.B. Behandlung mit Wasser unter neutralen oder schwach sauren Bedingungen, z.B. durch Einwirkung verdünnt-wässriger Mineral- oder Carbonsäuren, z.B. von verdünnter Salz- oder Essigsäure.

Verfahrensgemäss oder anderweitig erhältliche erfindungsgemässe Verbindungen der Formel I können in üblicher Weise in andere erfindungsgemässe Verbindungen der Formel I übergeführt werden.

So kann man z.B. veresterte oder amidierte Carboxygruppen $R_1$ in üblicher Weise, beispielsweise in Gegenwart eines basischen oder sauren Hydrolysemittels, wie eines Alkalimetallhydroxides oder -carbonates, z.B. von Natriumhydroxid oder Kaliumcarbonat, oder einer Mineralsäure, z.B. von Salzsäure oder Schwefelsäure, zu Carboxy hydrolysieren. Veresterte Carboxygruppen können ferner durch Umesterung, d.h. Behandlung mit einem Alkohol in Gegenwart eines sauren oder basischen Solvolysemittels, wie einer Mineralsäure, z.B. von Schwefelsäure, bzw. eines entsprechenden Alkalimetallalkoholates oder eines Alkalimetallhydroxides, in andere veresterte Carboxygruppen $R_1$ oder durch Umsetzung mit Ammoniak oder einem entsprechenden Amin in amidiertes Carboxy $R_1$ überführt werden.

Freies Carboxy $R_1$ kann in üblicher Weise, beispielsweise durch Behandlung mit einem entsprechenden Alkohol in Gegenwart einer Mineralsäure, z.B. von Schwefelsäure, oder durch Ueberführung in ein Halogenid und anschliessende Umsetzung mit einem entsprechenden Alkohol, z.B. in Gegenwart von Pyridin oder Triethylamin, oder durch Ueberführung in ein Alkalimetallsalz und anschliessende Umsetzung mit einem reaktionsfähigen Ester des entsprechenden Alkohols, wie einem entsprechenden Halogenid, in verestertes Carboxy $R_1$ überführt werden. Ebenso kann eine Carboxyverbindung unter Verwendung eines Dehydratisierungsmittels, wie N,N'-Dicyclohexylcarbodiimid, mit einem entsprechenden Alkohol verestert werden. Freies oder verestertes Carboxy $R_1$ kann auch durch Umsetzung mit Ammoniak oder einem mindestens ein Wasserstoffatom aufweisenden Amin und Dehydratisierung des intermediär gebildeten Ammoniumsalzes, z.B. durch Erhitzen oder mittels eines Dehydratisierungsmittels, wie N,N'-Dicyclohexylcarbodiimid, oder durch Ueberführung in das Halogenid und anschliessende Umsetzung mit Ammoniak oder einem mindestens ein Wasserstoffatom aufweisenden Amin in amidiertes Carboxy $R_1$ überführt werden.

Ferner kann man gegebenenfalls vorhandenes Hydroxy am Rest R bzw. R' verestern, z.B. durch Behandeln mit einem Niederalkancarbonsäureanhydrid bzw. -halogenid in Niederalkanoyloxy überführen oder durch Umsetzung mit einem reaktionsfähigen Ester, insbesondere Brom- oder Chlorwasserstoffsäureester, eines Niederalkanols in entsprechede veräthertes Hydroxy überführen. In analoger Weise kann man auch eine gegebenenfalls vorhandene Hydroxy- bzw. Aminogruppe $R_2$ acylieren, z.B. durch Behandeln mit einem Niederalkancarbonsäureanhydrid bzw. -halogenid oder Niederalkansulfonsäurechlorid in Niederalkanoyloxy bzw. Niederalkensulfonyloxy $R_2$ überführen, sowie Hydroxy $R_2$ veräthern. Umgekehrt kann man aus veresterten oder verätherten Hydroxygruppen, wie Niederalkanoyloxy oder Niederalkoxy, die Hydroxygruppe solvolytisch freisetzen, vorzugsweise unter sauren Bedingungen. In analoger Weise kann man auch veräthertes oder acyliertes Hydroxy $R_2$ zu Hydroxy $R_2$ bzw. acyliertes Amino $R_2$ zu Amino $R_2$ hydrolysieren. Ferner kann man eine mit einem Phenylniederalkanol verätherte oder mit einem Kohlensäurehalbester veresterte Hydroxygruppe $R_2$ hydrogenolytisch freisetzen.

Liegt in den erfindungsgemässen Verbindungen I zwischen den C-Atomen, welche $R_1$ und $R_2$ tragen, eine Doppelbindung vor, so kann diese z.B. in an sich bekannter Weise mit Hilfe eines Reduktionsmittels, z.B. der unter Variante f) aufgeführten Art, insbesondere mit Natriumborhydrid oder durch katalytische Hydrierung, zu einer Einfachbindung hydriert werden.

Ferner kann man eine erfindungsgemässe Verbindung der Formel I, in der die gestrichelte Linie für eine Doppelbindung zwischen den C-Atomen, welche $R_1$ und $R_2$ tragen, steht und $R_2$ Wasserstoff bedeutet, z.B. in an sich bekannter Weise durch Addition einer Verbindung $R_2$-H, in der $R_2$ eine gegebenenfalls verätherte oder acylierte Hydroxygruppe oder eine Aminogruppe darstellt, in eine entsprechende erfindungsgemässe Piperidinverbindung überführen. Die Addition wird dabei insbesondere in Gegenwart einer geeigneten Base, z.B. der unter Variante a) aufgeführten Art, durchgeführt.

Erfindungsgemässe Verbindungen I, in denen die gestrichelte Linie für eine Einfachbindung zwischen den C-Atomen, welche, $R_1$ und $R_2$ tragen, steht, können umgekehrt z.B. durch Eliminierung einer Verbindung $R_2$-H, in der $R_2$ eine gegebenenfalls verätherte oder acylierte Hydroxygruppe oder eine gegebenenfalls acylierte Aminogruppe darstellt, in an sich bekannter Weise in entsprechende erfindungsgemässe Tetrahydro-Pyridin-Verbindungen, in denen $R_2$ Wasserstoff darstellt, überführt werden. Für eine Eliminierung schlechter geeignete Abgangsgruppen $R_2$, z.B. Hydroxy, können dabei zuvor, beispielsweise in situ, in besser geeignete Abgangsgruppen $R_2$, z.B. Niederalkansulfonyloxy, oder in Halogen, wie Chlor, Brom oder Iod, umgewandelt werden. Die Eliminierung erfolgt dabei insbesondere in Gegenwart einer geeigneten Base, z.B. der unter Variante a) aufgeführten Art.

Salze von Verbindungen der Formel I bzw. ihrer Tautomeren können in an sich bekannter Weise hergestellt werden. So erhält man beispielsweise Säureadditionssalze von Verbindungen der Formel I durch Behandeln mit einer Säure oder einem geeigneten Ionenaustauscherreagens. Salze können in üblicher Weise in die freien Verbindungen überführt werden, Säureadditionssalze z.B. durch Behandeln mit einem geeigneten basischen Mittel.

Je nach Verfahrensweise bzw. Reaktionsbedingungen können die erfindungsgemässen Verbindungen I mit salzbildenden, insbesondere basischen Eigenschaften, in freier Form oder in Form von Salzen erhalten werden.

Infolge der engen Beziehung zwischen der neuen Verbindung I in freier Form und in Form ihrer Salze sind im vorausgegangenen und nachfolgend unter der freien Verbindung I oder ihren Salzen sinn- und zweckgemäss gegebenenfalls auch die entsprechenden Salze bzw. die freie Verbindung I zu verstehen.

Die neuen Verbindungen I einschliesslich ihrer Salze von salzbildenden Verbindungen können auch in Form ihrer Hydrate erhalten werden oder andere zur Kristallisation verwendete Lösungsmittel einschliessen.

Die neuen Verbindungen I können, je nach der Wahl der Ausgangsstoffe und Arbeitsweisen, in Form eines der möglichen Isomeren oder als Gemische derselben, z.B. je nach der Anzahl der asymmetrischen Kohlenstoffatome, als reine optische Isomere, wie Antipoden, oder als Isomerengemische, wie Racemate, Diastereoisomerengemische oder Racematgemische, vorliegen.

Erhaltene Diastereomerengemische und Racematgemische können auf Grund der physikalisch-chemischen Unterschiede der Bestandteile in bekannter Weise in die reinen Isomeren, Diastereomeren oder Racemate aufgetrennt werden, beispielsweise durch fraktionierte Kristallisation.

Erhaltene Enantiomerengemische lassen sich nach bekannten Methoden in die optischen Antipoden zerlegen, beispielsweise durch Umkristallisation aus einem optisch aktiven Lösungsmittel, Chromatographie an chiralen Adsorbentien, mit Hilfe von geeigneten Mikroorganismen, durch Spaltung mit spezifischen, immobilisierten Enzymen, über die Bildung von Einschlussverbindungen, z.B. unter Verwendung chiraler Kronenäther, wobei nur ein Enantiomeres komplexiert wird, oder durch Ueberführung in diastereomere Salze, z.B. durch Umsetzung eines basischen Endstoffracemats mit einer optisch aktiven Säure, wie Carbonsäure, z.B. Wein- oder Aepfelsäure, oder Sulfonsäure, z.B. Camphersulfonsäure, und Trennung des auf diese Weise erhaltenen Diastereomerengemisches, z.B. auf Grund ihrer verschiedenen Löslichkeiten, in die Diastereomeren, aus denen das gewünschte Enantiomere durch Einwirkung geeigneter Mittel freigesetzt werden kann. Vorteilhaft isoliert man das wirksamere Enantiomere.

Die Erfindung betrifft auch diejenigen Ausführungsformen des Verfahrens, nach denen man von einer auf irgendeiner Stufe des Verfahrens als Zwischenprodukt erhältlichen Verbindung ausgeht und die fehlenden Schritte durchführt oder einen Ausgangsstoff in Form eines Derivates bzw. Salzes, und/oder seiner Racemate bzw. Antipoden, verwendet oder insbesondere unter den Reaktionsbedingungen bildet.

Beim Verfahren der vorliegenden Erfindung werden vorzugsweise solche Ausgangsstoffe verwendet, welche zu den eingangs als besonders wertvoll geschilderten Verbindungen I führen.

Die Erfindung betrifft ebenfalls die Verwendung von Verbindungen der Formel I bzw. deren Tautomeren oder von pharmazeutisch verwendbaren Salzen von solchen Verbindungen mit salzbildenden Eigenschaften als pharmakologische, in erster Linie nootrop wirksame, Wirksubstanzen. Dabei kann man sie, vorzugsweise in Form von pharmazeutisch verwendbaren Zubereitungen, in einem Verfahren zur prophylaktischen und/oder therapeutischen Behandlung des tierischen oder menschlichen Körpers, insbesondere als Nootropika, z.B. zur Behandlung von Gedächtnisstörungen, verwenden.

Die Erfindung betrifft gleichfalls pharmazeutische Präparate, die die erfindungsgemässen Verbindungen I oder pharmazeutisch verwendbare Salze derselben als Wirkstoffe enthalten, sowie Verfahren zu ihrer Herstellung.

Bei den erfindungsgemässen pharmazeutischen Präparaten, welche die erfindungsgemässen Verbindungen I oder pharmazeutisch verwendbare Salze davon enthalten, handelt es sich um solche zur enteralen, wie oralen, ferner rektalen, und parenteralen Verabreichung an Warmblüter, wobei der pharmakologische Wirkstoff allein oder zusammen mit einem pharmazeutisch anwendbaren Trägermaterial enthalten ist.

Die neuen pharmazeutischen Präparate enthalten z.B. von etwa 10 % bis etwa 80 %, vorzugsweise von etwa 20 % bis etwa 60 %, des Wirkstoffs. Erfindungsgemässe pharmazeutische Präparate zur enteralen bzw. parenteralen Verabreichung sind z.B. solche in Dosiseinheitsformen, wie Dragées, Tabletten, Kapseln oder Suppositorien, ferner Ampullen. Diese werden in an sich bekannter Weise, z.B. mittels konventioneller Misch-, Granulier-, Dragier-, Lösungs- oder Lyophilisierungsverfahren hergestellt. So kann man pharmazeutische Präparate zur oralen Anwendung erhalten, indem man den Wirkstoff mit festen Trägerstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphat, z.B. Tricalciumphosphat oder Calciumhydrogenphosphat, ferner Bindemittel, wie Stärkekleister, unter Verwendung z.B. von Mais-, Weizen-, Reis- oder Kartoffelstärke, Gelatine, Tragaganth, Methylcellulose und/oder Polyvinylpyrrolidon, wenn erwünscht,

Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier- und Schmiermitel, z.B. Kieselsäure, Talk, Stearinsäure oder Salze davon, wie Magnesium- oder Calciumstearat, und/oder Polyäthylenglykol. Dragée-Kerne werden mit geeigneten, gegebenenfalls Magensaft-resistenten Ueberzügen versehen, wobei man u.a. konzentrierte Zuckerlösungen, welche gegebenfalls arabischen Gummi, Talk, Polyvinylpyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare erfindungsgemässe pharmazeutische Präparate sind Steckkapseln aus Gelatine, sowie weiche, geschlossene Kapseln aus Gelatine und einem Weichmacher, wie Glycerin oder Sorbitol. Die Steckkapseln können den Wirkstoff in Form eines Granulats, z.B. im Gemisch mit Füllstoffen, wie Lactose, Bindemitteln, wie Stärken, und/oder Gleitmitteln, wie Talk oder Magnesiumstearat, und gegebenenfalls von Stabilisatoren, enthalten. In weichen Kapseln ist der Wirkstoff vorzugsweise in geeigneten Flüssigkeiten, wie fetten Oelen, Paraffinöl oder flüssigen Polyäthylenglykolen, gelöst oder suspendiert, wobei ebenfalls Stabilisatoren zugefügt sein können.

Als rektal anwendbare erfindungsgemässe pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyäthylenglykole oder höhere Alkanole. Ferner können auch Gelatine-Rektalkapseln verwendet werden, die eine Kombination des Wirkstoffs mit einem Grundmassenstoff enthalten. Als Grundmassenstoffe kommen z.B. flüssige Triglyceride, Polyäthylenglykole oder Paraffinkohlenwasserstoffe in Frage.

Zur parenteralen Verabreichung eignen sich in erster Linie wässrige Lösungen eines Wirkstoffs in wasserlöslicher Form, z.B. eines wasserlöslichen Salzes, ferner Suspensionen des Wirkstoffs, wie entsprechende ölige Injektionssuspensionen, wobei man geeignete lipophile Lösungsmittel oder Vehikel, wie fette Oele, z.B. Sesamöl, oder synthetische Fettsäureester, z.B. Aethyloleat oder Triglyceride, verwendet, oder wässerige Injektionssuspensionen, welche viskositätserhöhende Stoffe, z.B. Natriumcarboxymethylcellulose, Sorbit und/oder Dextran, und gegebenenfalls auch Stabilisatoren enthalten.

Die Dosierung des Wirkstoffs hängt von der Warmblüter-Spezies, dem Alter und dem individuellen Zustand sowie der Applikationsweise ab. Im Normalfall ist für einen etwa 75 kg schweren Warmblüter bei oraler Applikation eine ungefähre Tagesdosis von etwa 20 bis etwa 500 mg, insbesondere von etwa 25 bis etwa 250 mg, vorteilhaft mehreren gleichen Teildosen, zu veranschlagen.

Die nachfolgenden Beispiele illustrieren die oben beschriebene Erfindung; sie sollen jedoch diese in ihrem Umfang in keiner Weise einschränken. Temperaturen sind in Celsiusgraden angegeben.

Beispiel 1: Zu einer Suspension von 3 g (63 mMol) Natriumhydrid (50 % Dispersion in Mineralöl) in 300 ml Toluol wird 18 g (49 mMol) N-[(5-Methoxybenzocyclobuten-1-yl)methyl]-imino-di(3-propionsäure)-diäthylester, gelöst in 100 ml Toluol zugetropft. Das Reaktionsgemisch wird unter Rühren auf etwa 110° Innentemperatur erwärmt, wobei vom entstehenden Aethanol abdestilliert wird. Alsdann lässt man abkühlen, verdünnt mit Essigsäureäthylester und versetzt unter Rühren mit 100 ml Wasser. Die organische Phase wird abgetrennt, über Magnesiumsulfat getrocknet und eingedampft. Das so erhaltene Rohmaterial wird chromatographisch gereinigt und mit äthanolischer Salzsäure in das Hydrochlorid überführt. Kristallisation aus Aethanol ergibt 4-Hydroxy-1-[(5-methoxybenzocyclobuten-1-yl)methyl]-1,2,5,6-tetrahydro-pyridin-3-carbonsaure äthylester-hydrochlorid bzw. 1-[(5-Methoxybenzocyclobuten-1-yl)methyl]-4-oxopiperidin-3-carbonsäureäthylester-hydrochlorid vom Smp. 181-182°.

Das Ausgangsmaterial kann z.B. folgendermassen hergestellt werden:

Zu 123 g (752 mMol) 5-Methoxybenzocyclobuten-1-ylmethylamin in 1100 ml Aethanol werden unter Rühren 245 ml (2.26 Mol) Acrylsäureäthylester getropft. Dann wird 20 Stunden zum Rückfluss erhitzt. Das Reaktionsgemisch wird am Wasserstrahlvakuum eingeengt und anschliessend chromatographisch gereinigt. Das so gereinigte Material wird mit äthanolischer Salzsäure ins Hydrochlorid überführt und aus Aethanol-Diäthyläther kristallisiert. Man erhält N-[(5-Methoxybenzocyclobuten-1-yl)methyl]imino-di(3-propionsäure)-diäthylester-hydrochlorid vom Smp. 97-98. 5°C.

Beispiel 2: Analog zu Beispiel 1 kann man ausgehend von 3.6 g (74 mMol) Natriumhydrid (50 % Dispersion in Mineralöl) und 19.2 g (57 mMol) N-[(5-Methoxybenzocyclobuten-1-yl)methyl]imino-di(3-propionsäure)dimethylester in 300 ml Toluol 4-Hydroxy-1-[(5-methoxybenzocyclobuten-1-yl)methyl]-1,2,5,6-tetrahydro-pyridin-3-carbonsäuremethylester-hydrochlorid bzw. 1-[(5-Methoxybenzocyclobuten-1-yl)methyl]-4-oxo-piperidin-3-carbonsäuremethylester-hydrochlorid vom Smp. 114.2-116.4° herstellen.

Das Ausgangsmaterial, N-[(5-Methoxybenzocyclobuten-1-yl)methyl]-imino-di(3-propionsäure)-dimethylester-hydrochlorid vom Smp. 114-115,5° C, kann man analog wie in Beispiel 1 beschrieben ausgehend von 4.2 g (25 mMol) 5-Methoxybenzocyclobuten-1-ylmethylamin und 6.8 ml (76 mMol) Acrylsäuremethylester in 50 ml Methanol herstellen.

Beispiel 3: 11.7 g (33 mMol) 4-Hydroxy-1-[(5-methoxybenzocyclobuten-1-yl)methyl]-1,2,5,6-tetrahydro-pyridin-3-carbonsäureäthylester-hydrochlorid werden unter Zusatz von 1.2 g Platinoxid in 300 ml Aethanol bei $4 \cdot 10^5$ Pa und Raumtemperatur hydriert. Vom Katalysator wird abfiltriert und die Reaktionslösung eingeengt. Kristallisation aus Aethanol/Diäthyläther ergibt cis-4-Hydroxy-1-[(5-methoxybenzocyclobuten-1-yl)methyl]piperidin-3-carbonsäureäthylester-hydrochlorid vom Smp. 156-160°.

Beispiel 4: 10.6 g (33.4 mMol) 4-Hydroxy-1-[(5-methoxybenzocyclobuten-1-yl)methyl]-1,2,5,6-tetrahydro-pyridin-3-carbonsäureäthylester, gelöst in 40 ml Aethanol, werden in eine Lösung von 0.7 g (17 mMol) Natriumborhydrid in 160 ml Aethanol/Wasser (1:1) getropft. Das Reaktionsgemisch wird 3 Stunden bei Raumtemperatur gerührt, anschliessend mit 200 ml Wasser verdünnt und mit Dichlormethan extrahiert. Die vereinigten Extrakte werden über Natriumsulfat getrocknet und unter vermindertem Druck eingedampft. Chromatographische Reinigung an Kieselgel ergibt neben dem in Beispiel 3 beschriebenen cis-Derivat auch den trans-4-Hydroxy-1-[(5-methoxybenzocyclobuten-1-yl)methyl]-piperidin-3-carbonäureäthylester als viskoses Oel, Rf = 0.44, Hexan/Aethanol (4:1).

Beispiel 5: Zu 4 g (12.5 mMol) cis-4-Hydroxy-1-[(5-methoxybenzocyclobuten-1-yl)methyl]-piperidin-3-carbonsäureäthylester-hydrochlorid, 1,7 g (14 mMol) 4-Dimethylaminopyridin und 3.5 ml (25 mMol) Triäthylamin in 100 ml Dichlormethan wird bei Raumtemperatur unter Rühren 1.1 ml (14 mMol) Methansulfonsäurechlorid getropft. Es wird bis zum vollständigen Umsatz bei dieser Temperatur weitergerührt. Das Reaktionsgemisch wird eingeengt, mit 4 g Kaliumhydroxid in 50 ml Aethanol versetzt und 30 Minuten verrührt. Anschliessend wird mit Dichlormethan verdünnt und mit Wasser gewaschen. Die organische Phase wird abgetrennt, über Magnesiumsulfat getrocknet und unter vermindertem Druck eingeengt. Das resultierende Rohprodukt wird an Kieselgel chromatographisch gereinigt, mit äthanolischer Salzsäure in das Hydrochlorid überführt und aus Aethanol/Diäthyläther kristallisiert. Man erhält 1-[(5-Methoxybenzocyclobuten-1-yl)methyl]-1,2,5,6-tetrahydro-pyridin-3-carbonsäureäthylester-hydrochlorid vom Smp. 204.5-205.5°.

Beispiel 6: Analog zu Beispiel 1 kann man ausgehend von 26.5 g (70 mMol) N-[2-(5-Methoxybenzocyclobuten-1-yl)äthyl]imino-di(3-propionsäure)diäthylester und 4.4 g (91 mMol) Natriumhydrid (50 % Dispersion in Mineralöl) in 250 ml Toluol 4-Hydroxy-1-[2-(5-methoxy-benzocyclobuten-1-yl)äthyl]-1,2,5,6-tetrahydro-pyridin-3-carbonsäureäthylester-hydrochlorid bzw. 1-[2-(5-Methoxybenzocyclobuten-1-yl)-äthyl]-4-oxo-piperidin-3-carbonsäureäthylester-hydrochlorid vom Smp. 148-150° herstellen.

Das Ausgangsmaterial kann z.B. wie folgt hergestellt werden:

Zu 5 g (29 mMol) 2-(5-Methoxybenzocyclobuten-1-yl)acetonitril in 50 ml Tetrahydrofuran wird bei Raumtemperatur 4,3 ml (43 mMol) Borandimethylsulfid (10 m in Tetrahydrofuran) langsam zugetropft. Dann wird 30 Minuten zum Rückfluss erwärmt. Anschliessend wird unter guter Kühlung mit 30 ml 6n-Salzsäure versetzt und dann weitere 30 Minuten zum Rückfluss erhitzt. Das Reaktionsgemisch wird abgekühlt, mit 4n-Natronlauge auf pH 10 gestellt und mit Dichlormethan extrahiert. Die organischen Phasen werden vereinigt, über Natriumsulfat getrocknet und unter vermindertem Druck eingeengt. Man erhält 2-(5-Methoxybenzocyclobuten-1-yl)äthylamin als viskoses Oel;
Rf = 0.1, Toluol/Aethanol/konz. wässriges Ammoniak, (90:20:1).

Aus 12 g (68 mMol) 2-(5-Methoxybenzocyclobuten-1-yl)äthylamin und 22 ml (200 mMol) Acrylsäureäthylester erhält man N-[2-(5-Methoxybenzocyclobuten-1-yl)äthyl]imino-di(3-propionsäure)diäthylester; Rf = 0.24, Hexan/Essigsäureäthylester (8:2) als viskoses Oel.

Beispiel 7: Analog zu Beispiel 3 kann man durch Hydrierung von 3.7 g (10 mMol) 4-Hydroxy-1-[2-(5-methoxybenzocyclobuten-1-yl)äthyl]-1,2,5,6-tetrahydro-pyridin-3-carbonsäureäthylester-hydrochlorid unter Zusatz von 400 mg Platinoxid in 100 ml Aethanol bei $4 \cdot 10^5$ Pa Wasserstoffüberdruck und Raumtemperatur cis-4-Hydroxy-1-(2-(5-methoxybenzocyclobuten-1-yl)äthyl]-piperidin-3-carbonsäureäthylester-hydrochlorid vom Smp. 144° herstellen.

Beispiel 8: Analog zu Beispiel 4 kann man ausgehend von 3,3 g (10 mMol) 4-Hydroxy-1-[2-(5-methoxybenzocyclobuten-1-yl)äthyl]-1,2,5,6-tetrahydropyridin-3-carbonsäurāthylester und 0.2 g (5 mMol) Natriumborhydrid in 80 ml 50%-wässrigem Aethanol trans-4-Hydroxy-1-[2-(5-methoxybenzocyclobuten-1-yl)äthyl]-piperidin-3-carbonsäureäthylester als viskoses Oel herstellen; Rf = 0.4, Hexan/Aethanol (8:2).

Beispiel 9: In Analogie zu Beispiel 5 kann man ausgehend von 8 g (24 mMol) cis-4-Hydroxy-1-[2-(5-methoxybenzocyclobuten-1-yl)-äthyl]piperidin-3-carbonsäureäthylester, 3,2 g (26 mMol) 4-Dimethylaminopyridin, 6,65 ml (48 mMol) Triäthylamin und 2 ml (27 mMol) Methansulfonsäurechlorid in 200 ml Dichlormethan 1-[2-(5-Methoxybenzocyclobuten-1-yl)äthyl]-1,2,5,6-tetrahydro-pyridin-3-carbonsäureäthylester-hydrochlorid vom Smp. 150.5-152.3° erhalten.

Beispiel 10: Analog wie in Beispiel 1 beschrieben kann man ausgehend von 18.6 g (53 mMol) N-[2-(5-Methoxybenzocyclobuten-1-yl)äthyl]-imino-di(3-propionsäure)dimethylester und 3.3 g (69 mMol) Natriumhydrid (50 % Dispersion in Mineralöl) in 350 ml Toluol 4-Hydroxy-1-[2-(5-methoxybenzocyclobuten-1-yl)-äthyl]-1,2,5,6-tetrahydropyridin3-carbonsäuremethylester-hydrochlorid bzw. 1-[2-(5-Methoxybenzocyclobuten-1-yl)äthyl]-4-oxo-piperidin-3-carbonsäuremethylester-hydrochlorid vom Smp. 157-158.5° herstellen.

Das Ausgangsmaterial, N-[2-(5-Methoxybenzocyclobuten-1-yl)äthyl]-imino-di(3-propionsäure)-dimethylester; Rf = 0.64, Hexan/Essigsäureäthylester (7:3) erhält man z.B. analog wie in Beispiel 1 beschrieben ausgehend von 14 g (77 mMol) 2-(5-Methoxybenzocyclobuten-1-yl)äthylamin und 21 ml (230 mMol) Acrylsäuremethylester in 150 ml Methanol.

Beispiel 11: 3.5 g (10 mMol) 4-Hydroxy-1-[(5-methoxybenzocyclobuten-1-yl)methyl]-1 ,2,5,6-tetrahydro-pyridin-3-carbonsäureäthylester-hydrochlorid werden in 50 ml Dioxan und 50 ml 25%-wässrigem Ammoniak im Bombenrohr unter Rühren 48 Stunden auf 80°C erwärmt. Das Reaktionsgemisch wird dann mit Trichlormethan extrahiert; die Extrakte werden mit Wasser gewaschen, über Natriumsulfat getrocknet und unter vermindertem Druck eingeengt. Das Rohmaterial wird chromatographisch gereinigt, mit Salzsäure in das Hydrochlorid überführt und aus Methanol/Diäthyläther kristallisiert. Man erhält 4-Amino-1-[(5-methoxybenzocyclobuten-1-yl)methyl]-1,2,5,6-tetrahydro-pyridin-3-carbonsäureäthylester-hydrochlorid bzw. 1-(5-Methoxybenzocyclobuten-1-yl)methyl]-4-imino-piperidin-3-carbonsäureäthylester-hydrochlorid vom Smp. 184-184.5°.

Beispiel 12: 2.42 g (10 mMol) Methansulfonsäure(5-methoxybenzocyclobuten-1-ylmethyl)ester, 1.7 g (10 mMol) 4-Piperidon-3-carbonsäureäthylester und 1.38 g (10 mMol) Kaliumcarbonat werden in 50 ml Aethanol 24 Stunden unter Rückfluss erwärmt. Dann wird filtriert, am Wasserstrahlvakuum eingeengt und an Kieselgel chromatographiert. Das so erhaltene Material wird mit äthanolischer Salzsäure ins Hydrochlorid überführt und aus Aethanol kristallisiert. Man erhält 4-Hydroxy-1-[(5-methoxybenzocyclobuten-1-yl)methyl]-1,2,5,6-tetrahydro-pyridin-3-carbonsäureäthylester-hydrochlorid bzw. 1-[2-(5-Methoxybenzocyclobuten-1-yl)-methyl]-4-oxo-piperidin-3-carbonsäureäthylester-hydrochlorid vom Smp. 181-182°.

Das Ausgangsmaterial kann z.B. folgendermassen erhalten werden: Zu 5.0 g (30.5 mMol) (5-Methoxybenzocyclobuten-1-yl)methanol in 50 ml Dichlormethan werden bei 0°C unter Rühren und Feuchtigkeitsausschluss 6.1 ml (76 mMol) Pyridin und 3.6 ml (46 mMol) Methansulfonsäurechlorid zugegeben. Dann wird noch 18 Stunden bei Raumtemperatur ausgerührt. Anschliessend wird mit 50 ml Eiswasser versetzt und 30 Minuten verrührt. Die organische Phase wird abgetrennt; je einmal mit 2n-Natronlauge, 2n-Salzsäure und Wasser gewaschen, über Natriumsulfat getrocknet und unter vermindertem Druck eingeengt. Man erhält Methansulfonsäure(5-methoxybenzocyclobuten-1-ylmethyl)ester als vikoses Oel, Rf = 0.4, Toluol/Essigsäureäthylester (9:1).

Beispiel 13: 4.5 g (20 mMol) 1-Brom-2-(indan-1-yl)-äthan werden in 20 ml Toluol gelöst und bei Raumtemperatur zu einer Lösung von 4.9 g Guvacolinhydrobromid (20 mMol) und 5.7 g (44 mMol) N,N-Diisopropyl-N-äthyl-amin in 15 ml Dimethylformamid gegeben. Das Gemisch wird 12 Stunden unter Stickstoff bei 45° gerührt. Anschliessend werden die Lösungsmittel unter vermindertem Druck weitgehend entfernt. Der Rückstand wird mit 2n-Salzsäure sauer gestellt und die wässrige Lösung mit Diäthyläther extrahiert. Die sauren wässrigen Phasen werden mit gesättigter Natriumhydrogencarbonatlösung basisch gestellt und mit Diäthyläther ausgeschüttelt. Die organischen Phasen werden abgetrennt, mit gesättigter Kochsalzlösung gewaschen und über Magnesiumsulfat getrocknet. Man erhält 1-[2-(Indan-1-yl)äthyl]-1,2,5,6-tetrahydro-pyridin-3-carbonsäuremethylester.

Beispiel 14: Die gemäss Beispiel 13 erhaltene Rohbase wird mit ätherischer Salzsäure versetzt und das erhaltene Hydrochlorid aus Isopropanol/Diäthyläther umkristallisiert. Man erhält 1-[2-(Indan-1-yl)äthyl]-1,2,5,6-tetrahydro-pyridin-3-carbonsäuremethylester-hydrochlorid vom Smp. 195-197°.

Beispiel 15: 5,13 g (20 mMol) Methansulfonsäure(6-methoxyindan-1-ylmethyl)ester werden in 20 ml Toluol gelöst und zu einer Lösung von 4.9 g (22 mMol) Guvacolin-hydrobromid in 15 ml Dimethylformamid und 5.65 g (44 mMol) N,N-Diisopropyl-N-äthyl-amin gegeben.

Das Reaktionsgemisch wird 24 Stunden unter Stickstoff bei 50° gerührt. Anschliessend werden die Lösungsmittel unter vermindertem Druck weitgehend entfernt. Der Rückstand wird in 2n-Salzsäure gelöst, die saure Lösung mit Diäthyläther ausgeschüttelt und die ätherischen Phasen abgetrennt. Die saure wässrige Lösung wird mit gesättigter Natriumhydrogencarbonatlösung basisch gestellt und mit Diäthyläther

extrahiert. Die ätherischen Lösungen werden mit gesättigter Kochsalzlösung nachgewaschen, über Magnesiumsulfat getrocknet und am Vakuum zur Trockene eingedampft. Der erhaltene Rückstand wird mit Dichlormethan/Methanol (19:1) an Kieselgel gereinigt und die erhaltenen Eluate zur Trockene eingedampft. Der erhaltene 1-[(6-Methoxyindan-1-yl)methyl]-1,2,5,6-tetrahydro-pyridin-3-carbonsäuremethylester wird in Diäthyläther gelöst und mit Oxalsäure versetzt. Das ausgefallene Oxalat wird aus Isopropanol/Wasser und aus Diäthyläther umkristallisiert. Man erhält 1-[(6-Methoxyindan-1-yl)methyl]-1,2,5,6-tetrahydro-pyridin-3-carbonsäuremethylester-oxalat vom Smp. 184-186°.

Die benötigten Ausgangsprodukte werden z.B. wie folgt erhalten:

30.9 g (0,15 Mol) 6-Methoxyindan-1-carbonsäuremethylester werden in 600 ml Diäthyläther gelöst und im Verlaufe von 1 Stunde zu einer Suspension von 8.5 g (0,22 Mol) Lithiumaluminiumhydrid in 300 ml Diäthyläther getropft, wobei das Gemisch zum Rückfluss kommt. Das Gemisch wird 15 Stunden am Rückfluss gehalten, mit einem Eisbad gekühlt und nacheinander mit 8,5 ml Wasser, 8,5 ml 15 %iger Natronlauge und 25 ml Wasser behandelt.

Die erhaltene weisse, flockige Suspension wird abfiltriert, der Rückstand 2-mal mit Diäthyläther aufgekocht und erneut abfiltriert. Die organischen Phasen werden über Magnesiumsulfat getrocknet und am Vakuum eingedampft. Der erhaltene Rückstand, ein farbloses viskoses Oel, ist das rohe 6-Methoxyindan-1-methanol.

5,7 g (32 mMol) des rohen 6-Methoxyindan-1-methanol werden in 50 ml Dichlormethan gelöst und mit einer Lösung von 4,3 g (38 mMol) Methansulfonsäurechlorid in 10 ml Dichlormethan versetzt. Das Gemisch wird mit einem Eisbad auf +5° gekühlt und im Verlaufe von 20 Minuten eine Lösung von 3,9 g (38 mMol) Triäthylamin in 10 ml Dichlormethan zugetropft. Das Gemisch wird 3 Stunden bei Raumtemperatur gerührt, vom ausgfallenen Triäthylammoniumchlorid abfiltriert und das Filtrat mit Eiswasser ausgeschüttelt. Die organische Phase wird über Magnesiumsulfat getrocknet und unter vermindertem Druck zur Trockene eingedampft. Das erhaltene Oel besteht aus rohem Methansulfonsäure(6-methoxyindan-1-ylmethyl)ester, der sofort weiter umgesetzt wird.

Beispiel 16: 5,23 g (15 mMol) N-[(6-Methoxyindan-1-yl)methyl]-imino-di(3-propionsäure)dimethylester werden in 150 ml Toluol gelöst und mit 0,72 g (15 mMol) Natriumhydrid (50%ige Dispersion in Mineralöl) versetzt. Das Reaktionsgemisch wird 3 Stunden auf 80° erwärmt und anschliessend bei einer Innentemperatur von 110° ein Gemisch von Methanol und Toluol abdestilliert.

Der erhaltene Rückstand wird auf Eis/Salzsäure gegossen und die organische Phase abgetrennt. Die saure wässerige Lösung wird mit Natriumhydrogencarbonatlösung basisch gestellt und mit Diäthyläther/Essigester ausgeschüttelt. Die organischen Phasen werden mit gesättigter Kochsalzlösung gewaschen, über Magnesiumsulfat getrocknet und unter vermindertem Druck zur Trockene eingedampft. Der erhaltene Rückstand besteht aus rohem 4-Hydroxy-1-[(6-methoxyindan-1-yl)methyl]-1,2,5,6-tetrahydro-pyridin- bzw. 1-[(6-Methoxyindan-1-yl)methyl]-4-oxo-piperidin-3-carbonsäuremethylester. Die erhaltene Rohbase wird an 6 g Kieselgel mit Cyclohexan als Laufmittel gereinigt und mit ätherischer Salzsäure in das Hydrochlorid überführt. Das Hydrochlorid wird aus Isopropanol/Diäthyläther umkristallisiert. Das reine 4-Hydroxy-1-[(6-methoxyindan-1-yl)methyl]-1,2,5,6-tetrahydro-pyridin- bzw. 1-[(6-Methoxy-indan-1-yl)methyl]-4-oxo-piperidin-3-carbonsäuremethylester-hydrochlorid schmilzt bei 175-178°.

Das Ausgangsmaterial kann z.B. wie folgt erhalten werden:

12,8 g (50 mMol) Methansulfonsäure(6-methoxyindan-1-ylmethyl)ester und 5,2 g (80 mMol) Natriumazid werden in 130 ml Dimethylsulfoxyd gelöst und und 1 Stunde bei 80° gerührt. Das Reaktionsgemisch wird auf Eiswasser gegossen und mit Diäthyläther ausgeschüttelt. Die organischen Phasen werden 2-mal mit gesättigter Kochsalzlösung gewaschen, über Magnesiumsulfat getrocknet und unter vermindertem Druck zu Trockene eingedampft. Als Rückstand werden 9,7 g rohes, öliges 6-Methoxyindan-1-ylmethylazid erhalten.

20 g (0,1 Mol) 6-Methoxyindan-1-ylmethylazid werden in 400 ml Diäthyläther gelöst und bei 25-30° zu einer Suspension von 5,0 g (0,13 Mol) Lithiumaluminiumhydrid in 200 ml Diäthyläther zugetropft. Nach beendigter Zugabe wird das Gemisch 6 Stunden am Rückfluss gehalten und unter Eiskühlung mit je 5 ml Wasser, 5 ml 15%iger Natronlauge und 15 ml Wasser versetzt. Der ausgefallene, weisse Niederschlag wird abfiltriert und 3 mal mit Dichlormethan ausgekocht. Die organischen Filtrate werden vereinigt, über Magnesiumsulfat getrocknet und am Vakuum zur Trockene eingedampft. Das als öliger Rückstand erhaltene N-(6-Methoxyindan-1-ylmethyl)amin wird mit ätherischer Salzsäure in das Hydrochlorid überführt und aus Isopropanol/Diäthyläther umkristallisiert. Das reine 6-Methoxyindan-1-ylmethylammoniumchlorid schmilzt bei 129-131°.

5,1 g (28,7 mMol) daraus hergestelltes 6-Methoxyindan-1-ylmethylamin werden mit 7,4g (86 mMol) Acrylsäuremethylester in 60 ml Methanol gelöst und 12 Stunden zum Rückfluss erhitzt. Das Reaktionsgemisch wird eingedampft und der Rückstand mit Cyclohexan über 10 g Kieselgel gereinigt. Das erhaltene rohe N-[(6-Methoxyindan-1-yl)methyl]imino-di(3-propionsäure)dimethylester wird roh weiterverwendet.

Beispiel 17: Aus 9,9 g (26 mMol) N-[(6-Methoxyindan-1-yl)methyl]-imino-di(3-propionsäure)diäthylester und 1,63 g (34 mMol) Natriumhydridsuspension wird analog Beispiel 16 4-Hydroxy-1-[(6-methoxyindan-1-yl)methyl]-1,2,5,6-tetrahydro-pyridin- bzw. 1-[(6-Methoxyindan-1-yl)methyl]-4-oxo-piperidin-3-carbonsäure-äthylester erhalten.

Aus der rohen Base wird mit ätherischer Salzsäure das Hydrochlorid gefällt und dieses aus Isopropanol/Diäthyläther umkristallisiert. Das reine 4-Hydroxy-1-[(6-methoxyindan-1-yl)methyl]-1,2,5,6-tetrah ydro-pyridin-bzw. 1-[(6-Methoxyindan-1-yl)methyl]-4-oxo-piperidin-3-carbonsäureäthylester-hydrochlorid schmilzt bei 155-156°.

Das Ausgangsprodukt wird wie z.B. folgt erhalten:

10,2 g (56 mMol) 6-Methoxyindan-1-ylmethylamin und 17,2 g (172 mMol) Acrylsäureäthylester werden in 120 ml Aethanol gelöst und 24 Stunden am Rückfluss gehalten. Das Reaktionsprodukt wird im Vakuum zur Trockne eingedampft und der erhaltene ölige N-[(6-Methoxyindan-1-yl)methyl]-imino-di(3-propionsäure)-diäthylester mit Cyclohexan an 20 g Kieselgel gereinigt.

Beispiel 18: 11,6 g (30 mMol) N-[2-(6-Methoxyindan-1-yl)äthyl]-imino-di(3-propionsäure)diäthylester werden in 100 ml Toluol gelöst werden bei Raumtemperatur zu einer Suspension von 1,85 g (39 mMol) einer 50%igen Natriumhydrid-Dispersion in 50 ml Toluol zugetropft. Das Reaktionsgemisch wird 3 Stunden auf 80° erwärmt. Dann wird die Aussentemperatur auf 110-120° gesteigert und ein Gemisch von Aethanol und Toluol abdestilliert. Der Rückstand wird abgekühlt und auf 100 ml eiskalte n-Salzsäure gegossen. Die wässrige Phase wird abgetrennt, die organische Phase 2-mal mit je 30 ml n-Salzsäure ausgeschüttelt und die sauren wässrigen Anteile vereinigt und mit gesättigter Natriumhydrogencarbonatlösung alkalisch gestellt. Nach Ausschütteln mit Diäthyläther/Essigester werden die organischen Phasen mit gesättigter Kochsalzlösung gewaschen und über Magnesiumsulfat getrocknet. Nach Entfernen der Lösungsmittel wird der erhaltene, ölige Rückstand mit Cyclohexan über 10 g Kieselgel filtriert. 9,8 g der gereinigten Base werden mit ätherischer Salzsäure in das Hydrochlorid überführt und dieses aus Isopropanol/Diäthyläther kristallisiert. Das erhaltene 4-Hydroxy-1-[2-(6-methoxyindan-1-yl)äthyl]-1,2,5,6-tetrahydro-pyridin- bzw. 1-[2-(6-Methoxyindan-1-yl)äthyl]-4-oxo-piperidin-3-carbonsäureäthylester-hydrochlorid schmilzt bei 140-145°.

Die Ausgangsprodukte werden z.B. wie folgt erhalten:

12,8 g (50 mMol) Methansulfonsäure(6-methoxyindan-1-ylmethyl)ester und 3,7 g (75 mMol) Natriumcyanid werden in 70 ml Dimethylsulfoxyd gelöst und 6 Stunden auf 80° erwärmt. Das Reaktionsgemisch wird auf 200 ml Eiswasser gegossen und 3 mal mit je 100 ml Toluol ausgeschüttelt. Die organischen Phasen werden vereinigt, mit gesättigter Kochsalzlösung nachgewaschen und über Magnesiumsulfat getrocknet. Nach Entfernen des Lösungsmittels am Vakuum wird rohes 2-(6-Methoxyindan-1-yl)acetonitril erhalten.

9,3 g (49 mMol) rohes 2-(6-Methoxyindan-1-yl)acetonitril werden in 100 ml Methanol gelöst und nach Zusatz von 1 g Raney-Nickel und 20 g flüssigem Ammoniak bei 70-80° und 120 bar Druck hydriert. Nach 3 Stunden wird die methanolische Lösung vom Katalysator abfiltriert und dieser mit Methanol nachgewaschen. Die methanolischen Lösungen werden vereinigt und mit Salzsäure sauer gestellt. Die saure Lösung wird am Vakuum zur Trockene eingedampft und der Rückstand in 100 ml Wasser gelöst, mit konzentrierter Natronlauge alkalisch gestellt und mit Diäthyläther ausgeschüttelt. Die ätherischen Phasen werden mit gesättigter Kochsalzlösung gewaschen und über Magnesiumsulfat getrocknet. Nach Entfernen des Lösungsmittels wird rohes 2-(6-Methoxyindan-1-yl)äthylamin als gelbes Oel erhalten.

6,1 g (31,8 mMol) 2-(6-Methoxyindan-1-yl)äthylamin und 10 g (100 mMol) Acrylsäureäthylester werden in 60 ml Aethanol gelöst und 12 Stunden am Rückfluss gehalten. Anschliessend wird das Lösungsmittel am Vakuum entfernt und der Rückstand an 20 g Florisil mit Cyclohexan gereinigt. Man erhält rohen N-[2-(6-Methoxyindan-1-yl)äthyl]imino-di(3-propionsäure)diäthylester als gelbes, bewegliches Oel.

Beispiel 19: 11,8 g (32,4 mMol) N-(6-Methoxyindan-1-yl)-imino-di(3-propionsäure)diäthylester werden in 100 ml Toluol gelöst und bei Raumtemperatur zu einer Suspension von 2,1 g (42 mMol) 50 %iger Natriumhydrid-Dispersion in 50 ml Toluol getropft. Das Reaktionsgemisch wird 3 Stunden auf 80° erwärmt. Anschliessend wird die Aussentemperatur auf 110-120° erhöht und ein Gemisch von Aethanol und Toluol abdestilliert. Anschliessend wird das Reaktionsgemisch abgekühlt, auf ein Gemisch von 100 ml 2n-Salzsäure und Eis gegossen, mit Diäthyläther ausgeschüttelt und die sauren wässrigen Phasen mit Natriumhydrogencarbonatlösung basisch gestellt. Das erhaltene Gemisch wird mit Diäthyläther/Essigester (2:1) ausgeschüttelt, die organischen Phasen mit gesättigter Kochsalzlösung gewaschen und über Magnesiumsulfat getrocknet. Nach Entfernen des Lösungsmittels am Vakuum wird der Rückstand mit Cyclohexan an Kieselgel gereinigt. Man erhält 4-Hydroxy-1-(6-methoxyindan-1-yl)-1,2,5,6-tetrahydropyridin-bzw. 1-(6-Methoxyindan-1-yl)-4-oxo-piperidin-3-carbonsäureäthylester als Oel. Dieses wird mit ätherischer Salzsäure in das Hydrochlorid überführt und dieses aus Aethanol/Diäthyläther umkristallisiert. Das erhaltene 4-Hydroxy-1-(6-methoxyindan-1-yl)-1,2,5,6-tetrahydropyridin- bzw. 1-(6-Methoxyindan-1-yl)-4-oxo-piperidin-3-carbonsäureäthylester-hydrochlorid schmilzt bei 170-173°.

Das Ausgangsprodukt wird z.B. wie folgt enthalten:

9,5 g (50 mMol) 6-Methoxyindan-1-carbonsäure werden in 100 ml Trichlormethan gelöst, mit 8,9 g (75 mMol) Thionylchlorid und 3 Tropfen Dimethylformamid versetzt und 1,5 Stunden am Rückfluss gehalten.

Das Reaktionsgemisch wird am Vakuum zur Trockene eingedampft und anschliessend 2 mal mit je 50 ml Toluol versetzt und erneut eingedampft. Der erhaltene Rückstand (10,8 g) wird in 200 ml Dichlormethan gelöst, mit 0,32 g (1 mMol) Tetrabutylammoniumbromid und bei 0° tropfenweise mit einer Lösung von 5,0 g (77 mMol) Natriumazid in 70 ml Wasser versetzt und 2 Stunden bei 0° gerührt.

Anschliessend wird die organische Phase abgetrennt, die wässrigen Phasen mit Dichlormethan nachgewaschen und die vereinigten Dichlormethanphasen bei 0° über Magnesiumsulfat getrocknet.

Die vereinigten Dichlormethanlösungen werden mit 11,4 g (100 mMol) Trifluoressigsäure versetzt und 18 Stunden am Rückfluss gekocht. Das abgekühlte Reaktionsgemisch wird mit Eis versetzt, mit 100 ml gesättigter Natriumhydrogencarbonatlösung ausgeschüttelt und über Magnesiumsulfat getrocknet. Nach Entfernen des Lösungsmittels wird der erhaltene kristallene Rückstand aus Aceton/Cyclohexan/Petroläther kristallisiert und ergibt das reine N-(6-Methoxyindan-1-yl)trifluoracetamidvom Smp. 128-136°.

19 g (73 mMol) N-(6-Methoxyindan-1-yl)trifluoracetamid werden in 300 ml Methanol gelöst und bei 40° tropfenweise mit 100 ml n-Kalilauge versetzt. Die stark alkalische Lösung wird 12 Stunden bei Raumtemperatur gerührt, über Diatomeenerde filtriert, mit Wasser nachgewaschen und mit konzentrierter Salzsäure sauer gestellt. Die erhaltene Lösung wird im Vakuum weitgehend eingeengt, der Rückstand mit konzentrierter Natronlauge alkalisch gestellt und mit Diäthyläther ausgeschüttelt. Die ätherischen Lösungen werden mit gesättigter Kochsalzlösung gewaschen, über festem Kaliumhydroxyd getrocknet und unter vermindertem Druck eingeengt. Der erhaltene, ölige Rückstand wird mit ätherischer Salzsäure versetzt und das ausgefallene Hydrochlorid aus Aethanol/Diäthyläther umkristallisiert. Das erhaltene N-(6-Methoxyindan-1-yl)-ammoniumchlorid schmilzt bei 253-254°.

Aus 6,0 g (30 mMol) N-(6-Methoxyindan-1-yl)ammoniumchlorid wird mit Diäthyläther und 2n-Natronlauge die Base freigesetzt. 5,3 g (30 mMol) der erhaltenen Base werden in 50 ml Aethanol gelöst, mit 9,0 g (90 mMol) Acrylsäureäthylester versetzt und 24 Stunden am Rückfluss gekocht. Anschliessend wird das Gemisch 8 Tage bei Raumtemperatur stehengelassen. Das Lösungsmittel wird am Vakuum entfernt und der Rückstand mit Cyclohexan an Kieselgel gereinigt. Die erhaltenen Eluate werden zur Trockene eingedampft. Man erhält als öligen Rückstand N-(5-Methoxyindan-1-yl)imino-di(3-propionsäure)diäthylester das ohne weitere Reinigung vewendet wird.

Beispiel 20: Eine Lösung von 29,32 g (75 mMol) N-[(7-Methoxy-1,2,3,4-tetrahydro-naphth-1-yl)methyl]-imino-di(3-propionsäure)diäthylester in 200 ml Dimethylformamid wird unter Rühren bei Raumtemperatur portionweise mit 4,35 g (90 mMol) Natriumhydrid-Dispersion (50 % in Oel) versetzt. Anschliessend wird 1 Stunde bei Raumtemperatur nachgerührt. Das Reaktionsgemisch wird am Hochvakuum eingedampft und der Rückstand mit kalter 2n-Salzsäure versetzt und mit Diäthyläther extrahiert. Die vereinigten salzsauren Extrakte werden mit Dichlormethan extrahiert, die vereinigten organischen Phasen über Natriumsulfat getrocknet und erneut eingedampft. Der ölige Rückstand wird in Aceton heiss gelöst, worauf nach dem Abkühlen 4-Hydroxy-1-[(7-Methoxy-1,2,3,4-tetrahydro-naphth-1-yl)methyl]-1,2,5,6-tetrahydro-pyridin- bzw. 1-[(7-Methoxy-1,2,3,4-tetrahydro-naphth-1-yl)methyl]-4-oxo-piperidin-3-carbonsäureäthylester-hydrochlorid vom Smp. 132-134° auskristallisieren.

Das Ausgangsmaterial kann z.B. folgendermassen hergestellt werden:

Eine Lösung von 19,1 g (100 mMol) 1,2,3,4-Tetrahydro-7-methoxy-naphth-1-ylmethylamin in 300 ml Aethanol wird mit 22 g (220 mMol) Acrylsäureäthylester versetzt und 48 Stunden bei 65° gerührt. Nach Erkalten wird das Lösungsmittel unter vermindertem Druck abgedampft, wobei man N-[(7-Methoxy-1,2,3,4-tetrahydro-naphth-1-yl)methyl]imino-di(propionsäure)diäthylester als rötliches Oel erhält.

Beispiel 21: Eine Lösung von 11,45 g (30 mMol) 4-Hydroxy-1,2,5,6-tetrahydro-1-[(7-methoxy-1,2,3,4-tetrahydro-naphth-1-yl)methyl]-pyridin-3-carbonsäureäthylester-hydrochlorid in 150 ml Aethanol wird unter Rühren bei 0-5° portionsweise mit 2,28 g (60 mMol) Natriumborhydrid versetzt. Nach 1 Stunde Rühren bei 0-5° wird das Reaktionsgemisch unter vermindertem Druck eingeengt, der Rückstand mit Wasser versetzt und mit Dichlormethan extrahiert. Die vereinigten organischen Phasen werden mit Wasser gewaschen, über Natriumsulfat getrocknet und total eingedampft. Das Rohprodukt, 8,0 g gelbes Oel wird über 320 g Kieselgel (0,040-0,063) mit einem Gemisch von Toluol und Essigester (1:1) chromatographiert. Man erhält so ein 1:1-Gemisch von cis- und trans-4-Hydroxy-1-[(7-methoxy-1,2,3,4-tetrahydro-naphth-1-yl)methyl]-piperidin-3-carbonsäureäthylester als farbloses Oel.

Beispiel 22: Eine Lösung von 5,2 g (15 mMol) cis-/trans-Gemisch von 4-Hydroxy-1-[(7-methoxy-1,2,3,4-tetrahydro-naphth-1-yl)methyl]-piperidin-3-carbonsäureäthylester und 11,4 g (75 mMol) 1,8-Diazabicyclo-[5.4.0]-undec-7-en in 100 ml Toluol wird unter Rühren bei 0-5° tropfenweise mit einer Lösung von 2,06 g (18 mMol) Methansulfochlorid in 20 ml Toluol versetzt. Anschliessend lässt man auf Raumtemperatur

erwärmen und rührt weitere 18 Stunden bei Raumtempratur. Das Reaktionsgemisch wird mit Eis-wasser versetzt und die organische Phase mit 2n-Salzsäure extrahiert. Die vereinigten salzsauren Extrakte werden mit konzentrierter Natronlauge alkalisch gestellt und mit Dichlormethan extrahiert. Die vereinigten organischen Phasen werden mit Wasser gewaschen, über Natriumsulfat getrocknet und eingedampft. Der ölige Rückstand (4,85 g) wird über 240 g Kieselgel (0,040-0,063) mit Toluol/Essigester (9:1) chromatographiert, wobei man 1-[(7-Methoxy-1,2,3,4-tetrahydro-naphth-1-yl)methyl]-1,2,5,6-tetrahydro-pyridin-3-carbonsäureäthylester als hellgelbes Oel erhält. Das mit Salzsäure in Diäthyläther hergestellte Hydrochlorid kristallisiert aus Aethanol/Diäthyläther Smp. 177-179° (Zers.).

Beispiel 23: In eine Lösung von 5,45 g (15 mMol) N-[(7-Methoxy-1,2,3,4-tetrahydronaphth-2-yl)methyl]-imino-di(3-propionsäure)dimethylester` in 55 ml Dimethylformamid, werden 0,86 g (18 mMol) Natriumhydrid-Dispersion (50 % in Oel) unter Rühren bei Raumtemperatur in 2 Portionen zugegeben. Das Reaktionsgemisch wird nach 2 stündigem Rühren im Hochvakuum total eingedampft. Der erhaltene Rückstand wird mit kalter 2n-Salzsäure versetzt und mit Diäthyläther extrahiert. Die wässrigen salzsauren Extrakte werde vereinigt und mit Dichlormethan extrahiert. Die organischen Phasen werden vereinigt, über Natriumsulfat getrocknet und eingedampft. Der ölige Rückstand wird in heissem Aceton gelöst, mit Diäthyläther bis zur Trübung versetzt, worauf beim Abkühlen 4-Hydroxy-1-[(7-methoxy-1,2,3,4-tetrahydronaphth-2-yl)methyl]-1,2,5,6-tetrahydropyridin-3-carbonsäuremethylester-hydrochlorid bzw. 1-[(7-Methoxy-1,2,3,4-tetrahydro-naphth-2-yl)methyl]-4-oxo-piperidin -3-carbonsäuremethylester-hydrochlorid vom Smp. 167-169° auskristallisiert.

Das Ausgangsmaterial kann man z.B. folgendermassen herstellen:
Zu einer Suspension von 3,79 g (100 mMol) Lithiumaluminiumhydrid in 160 ml absolutem Diäthyläther wird innerhalb von 30 Minuten eine Lösung von 23,43 g (100 mMol) 7-Methoxy-1,2,3,4-tetrahydro-naphthalin-2-carbonsäureäthylester in 160 ml absolutem Tetrahydrofuran unter Rühren, bei Raumtemperatur zugetropft. Nach 1 Stunde Rühren bei Raumtemperatur wird das Reaktionsgemisch mit 3,8 ml Wasser, 3,8 ml Natronlauge (15 %) und 11,4 ml Wasser versetzt. Der ausgefallene Niederschlag wird abgenutscht und das Filtrat am Vakuum zur Trockene eingedampft. Der ölige Rückstand wird in Diäthyläther gelöst, mit Wasser gewaschen, mit Natriumfulfat getrocknet und zur Trockene eingedampft. Man erhält 7-Methoxy-1,2,3,4-tetrahydro-naphthalin-2-methanol als gelbes Oel.

Eine Lösung von 19.2 g (100 mMol) 7-Methoxy-1,2,3,4-tetrahydro-naphthalin-2-methanol in 80 ml absolutem Pyridin wird unter Rühren bei Raumtemperatur mit 20,96 g (110 mMol) p-Toluolsulfonsäurechlorid versetzt, wobei die leicht exotherme Reaktion mit einem Eisbad bei Raumtemperatur gehalten wird. Das Reaktionsgemisch wird 1 Stunde bei Raumtemperatur ausgerührt und nun mit Eiswasser verdünnt und mit Diäthyläther extrahiert. Die organische Phase wird eiskalt mit 2n-Salzsäure und Wasser gewaschen, über Natriumsulfat getrocknet, filtriert und unter vermindertem Druck eingedampft. Das erhaltene Oel wird aus Diäthyläther/Pentan kristallisiert, wobei man p-Toluolsulfonsäure(7-methoxy-1,2,3,4-tetrahydro-naphth-2-yl-methyl)ester vom Smp. 64-66° erhält.

Eine Lösung von 13,86 g (40 mMol) p-Toluolsulfonsäure(7-methoxy-1,2,3,4-tetrahydro-naphth-2-ylmethyl)ester in 200 ml Aethanol wird mit einer Lösung von 3,9 g Natriumazid in 10 ml Wasser versetzt und während 18 Stunden am Rückfluss gekocht. Nach Erkalten wird der Alkohol unter vermindertem Druck abgedampft, der Rückstand mit Wasser versetzt und mit Dichlormethan extrahiert. Die organische Phase wird mit Wasser gewaschen, über Natriumsulfat getrocknet und eingedampft. Man erhält (7-Methoxy-1,2,3,4-tetrahydro-naphth-2-yl)methylazid als gelbes Oel.

Eine Lösung von 6,51 g (30 mMol) (7-Methoxy-1,2,3,4-tetrahydro-naphth-2-yl)methylazid in 100 ml absolutem Tetrahydrofuran wird innerhalb von 30 Minuten bei Raumtemperatur zu einer gerührten Suspension von 1,14 g (30 mMol) Lithiumaluminiumhydrid in 100 ml absolutem Diäthyläther, zugetropft. Nach 2 Stunden Rühren bei Raumtemperatur wird das Reaktionsgemisch mit 1,14 ml Wasser, 1,14 ml Natronlauge (15 %) und 3,4 ml Wasser hydrolysiert. Der entstandene Niederschlag wird abgenutscht und das Filtrat unter vermindertem Druck eingedampft. Das erhaltene Oel wird in Diäthyläther gelöst und mit 2n-Salzsäure aufgenommen. Die vereinigten salzsauren Extrakte werden unter Eiskühlung mit konzentrierter Natronlauge alkalisch gestellt und mit Dichlormethan extrahiert. Die organischen Phasen werden vereinigt, über Natriumsulfat getocknet und unter vermindertem Druck eingedampft. Man erhält 7-Methoxy-1,2,3,4-tetrahydro-naphth-2-ylmethylamin als gelbes Oel, Smp. des Hydrochlorides 205-206°.

Einer Lösung von 3,82 g (20 mMol) 7-Methoxy-1,2,3,4-tetrahydro-naphth-2-ylmethylamin in 60 ml Methanol wird mit 3,78 g Acrylsäuremethylester versetzt und 18 Stunden bei 65° gerührt. Nach dem Erkalten wird das Reaktionsgemisch unter vermindertem Druck eingedampft. Man erhält N-[(7-Methoxy-1,2,3,4-tetrahydro-naphth-2-yl)methyl]imino-di(3-propionsäure)dimethylester als rötliches Oel.

Beispiel 24: Eine Lösung von 5,19 g (15 mMol) p-Toluolsulfonsäure(7-methoxy-1,2,3,4-tetrahydro-naphth-2-ylmethyl)ester in 75 ml Dimethylformamid wird zuerst mit 3,66 g (16,5 mMol) 1,2,5,6-Tetrahydro-pyridin-3-carbonsäuremethylester-hydrobromid (Guvacolin-hydrobromid) und anschliessend mit 6,78 g (52,5 mMol) N-Aethyl-N,N-diisopropyl-amin versetzt. Das Gemisch wird 18 Stunden bei 60° gerührt und dann im Hochvakuum eingedampft. Der Rückstand wird mit Wasser versetzt und mit Diäthyläther extrahiert. Die organischen Phasen werden mit Wasser gewaschen und mit 2n-Salzsäure ausgezogen. Die Salzsäure-Extrakte werden kalt mit Natronlauge (30%ig) alkalisch gestellt, mit Dichlormethan extrahiert, worauf die vereinigten organischen Phasen über Natriumsulfat getrocknet und unter vermindertem Druck eingedampft werden. Man erhält 1-[(7-Methoxy-1,2,3,4-tetrahydro-naphth-2-yl)methyl]-1,2,5,6-tetrahydro-pyridin-3-carbonsäuremethylester als hellgelbes Oel. Das mit Salzsäure in Däthyläther hergestellte Hydrochlorid kristallisiert aus Aceton/Diäthyläther mit dem Smp. 161-163°.

Beispiel 25: Eine Lösung von 2,81 g Diisopropylamin in 30 ml trockenem Tetrahydrofuran wird bei 0°-5° mit 17,4 ml n-Butyllithium in Hexan versetzt. Man lässt 30 Minuten bei Raumtemperatur rühren, kühlt erneut auf -15° und versetzt mit einer Lösung von 6,13 g (25 mMol) 1-[(5-Methoxybenzocyclobuten-1-yl)-methyl]piperidin-4-on in 30 ml Tetrahydrofuran. Nach 15 Minuten tropft man eine Lösung von 3,05 g (28 mMol) Trimethylchlorsilan in 15 ml Tetrahydrofuran ein. Man lässt über Nacht bei Raumtemperatur rühren, filtriert und dampft unter Vakuum zur Trockne ein. Man erhält so 1-[(5-Methoxybenzocyclobuten-1-yl)-methyl]-4-trimethylsilyloxy-1,2,5,6-tetrahydro-pyridin als hellgelbes Oel.

Eine auf 0° gekühlte Lösung von 2,3 g (24 mMol) Chlorameisensäureäthylester und 60 mg (2,4 mMol) wasserfreiem Zinkbromid in 50 ml trockenem Dichlormethan versetzt man tropfenweise mit 6,39 g (20 mMol) 1-[(5-Methoxybenzocyclobuten-1-yl)methyl]-4-trimethylsilyloxy-1,2,5,6-tetrahydro-pyridin, gelöst in 50 ml Dichlormethan. Nach Erwärmen auf Raumtemperatur wird 1 Stunde nachgerührt hierauf auf 150 ml gesättigte Natriumhydrogencarbonatlösung gegossen und mit Dichlormethan extrahiert. Die vereinigten organischen Phasen werden über Natriumsulfat getrocknet und eingedampft. Der Rückstand wird in 70 ml Aethanol gelöst und mit äthanolischer Salzsäurelösung angesäuert. Nach Versetzen mit Diäthyläther und Abkühlen kristallisiert 4-Hydroxy-1-[(5-methoxybenzocyclobuten-1-yl)methyl]-1,2,5,6-tetrahydro-pyridin-3-carbonsäureäthylester-hydrochlorid bzw. 1-[(5-Methoxybenzocyclobuten-1-yl)methyl]-4-oxo-piperidin-3-carbonsäureäthylester-hydrochlorid vom Smp. 181°-182° aus.

Das Ausgangsmaterial kann man z.B. folgendermassen herstellen:

Eine Lösung von 12,11 g (50 mMol) Methansulfonsäure(5-methoxybenzocyclobuten-1-ylmethyl)ester in 100 ml Dimethylformamid wird zuerst mit 8,45 g (55 mMol) Piperidon-hydrochlorid-monohydrat und anschliessend mit 22,62 g (175 mMol) N-Aethyl-N,N-diisopropyl-amin versetzt. Das Gemisch wird 18 Stunden bei 80°C gerührt und nach Erkalten unter vermindertem Druck zur Trockne eingedampft. Der Rückstand wird in Diäthyläther gelöst und mit Wasser gewaschen. Die organische Phase wird abgetrennt und mit 2n-Salzsäure extrahiert. Die Salzsäure-Extrakte werden vereinigt, kalt mit konzentrierter Natronlauge alkalisch gestellt und mit Dichlormethan extrahiert. Die Auszüge werden vereinigt, über Natriumsulfat getrocknet und unter vermindertem Druck zur Trockne eingedampft. Man erhält ein dunkelbraunes Harz, welches mit Toluol/Aethylacetat (1:1) an 350 g Kieselgel (0,040-0,063 mm) chromatographisch gereinigt wird. Man erhält 1-[(5-Methoxybenzocyclobuten-1-yl)methyl]piperidin-4-on als hellgelbes Oel.

Das mit Salzsäure in Diäthyläther hergestellte Hydrochlorid kristallisiert aus Aceton/Diäthyläther mit dem Smp. 162°-163°.

Beispiel 26: Zu einer Lösung von 10,48 g (30 mMol) N-(7-Methoxy-1,2,3,4-tetrahydro-naphth-2-yl)imino-di(3-propionsäure)dimethylester in 100 ml absolutem Dimethylformamid werden bei Raumtemperatur unter Rühren innerhalb von 30 Minuten 1,73 g (36 mMol) Natriumhydrid eingetragen. Das Reaktionsgemisch wird 2 Stunden bei Raumtemperatur ausgerührt und unter vermindertem Druck zur Trockne eingedampft. Der Rückstand wird mit kalter 2n-Salzsäure versetzt und mit Diäthyläther extrahiert. Die vereinigten wässrigensalzsauren Extrakte werden mit Dichlormethan extrahiert und die vereinigten Dichlormethanextrakte über Natriumsulfat getrocknet und eingedampft. Der ölige Rückstand wird heiss in Acetonitril gelöst, bis zur Trübung mit Diäthyläther versetzt, worauf beim Abkühlen das 4-Hydroxy-1-(7-methoxy-1,2,3,4-tetrahydro-naphth-2-yl)-1,2,5,6-tetrahydro-pyridin- bzw. 1-(7-Methoxy-1,2,3,4-tetrahydro-naphth-2-yl)-4-oxo-piperidin-3-carbonsäuremethylester-hydrochlorid vom Smp. 188-190° (Zers.) aus kristallisiert.

Das Ausgangsmaterial kann z.B. folgendermassen hergestellt werden:

Eine Lösung von 4,43 g (25 mMol) 1,2,3,4-Tetrahydro-7-methoxy-naphth-2-ylamin in 150 ml Methanol wird mit 4,73 g (55 mMol) Acrylsäuremethylester versetzt und 24 Stunden zum Rückfluss erhitzt. Dann fügt man weitere 4,73 g (55 mMol) Acrylsäuremethylester zu und erhitzt weitere 48 Stunden zum Rückfluss. Nach Erkalten wird das Reaktionsgemisch eingedampft, wobei man N-(7-Methoxy-1,2,3,4-tetrahydro-naphth-2-yl)-imino-di(3-propionsäure)dimethylester als rötliches Oel erhält.

Beispiel 27: Eine Suspension von 5,3 g (15 mMol) 4-Hydroxy-1-(7-methoxy-1,2,3,4-tetrahydro-naphth-2-yl)-1,2,5,6-tetrahydro-pyridin-3-carbonsäuremethylester-hydrochlorid in 75 ml Methanol wird unter Rühren bei 0-5° portionsweise mit 1,14 g (30 mMol) Natriumborhydrid versetzt. Es wird 1 Stunde bei 0-5° ausgerührt und unter vermindertem Druck eingeengt. Das Reaktionsgemisch wird mit Wasser versetzt und mit Dichlormethan extrahiert. Die vereinigten organischen Phasen werden mit Wasser gewaschen, über Natriumsulfat getrocknet und eingedampft. Man erhält ein Gemisch von cis- und trans-4-Hydroxy-1-(7-methoxy-1,2,3,4-tetrahydro-naphth-2-yl)piperidin-3-carbonsäuremethylester als gelbes Oel.

Beispiel 28: Eine Lösung von 4,15 g (13 mMol) des Gemisches von cis und trans 4-Hydroxy-1-(7-methoxy-1,2,3,4-tetrahydro-naphth-2-yl)-piperidin-3-carbonsäuremethylester und 9,89 g (65 mMol) 1,8-Diazabicyclo[5.4.0]undec-7-en in 90 ml Toluol wird unter Rühren bei 0-5° tropfenweise mit einer Lösung von 1,78 g (15,5 mMol) Methansulfonsäurechlorid in 15 ml Toluol versetzt. Anschliessend lässt man auf Raumtemperatur erwarmen und weitere 18 Stunden rühren. Das Reaktionsgemisch wird sodann mit Eiswasser versetzt und die organische Phase mit 2n-Salzsäure extrahiert. Die vereinigten salzsauren Extrakte werden mit konzentrierter Natronlauge alkalisch gestellt und wiederholt mit Dichlormethan extrahiert. Die vereinigten Extrakte werden mit Wasser gewaschen, über Natriumsulfat getrocknet und eingedampft. Das erhaltene Rohprodukt wird an 190 g Kieselgel (0,040-0,063) mit Toluol/Aethylacetat (1:1) chromatographisch gereinigt. Man erhält 1-(7-Methoxy-1,2,3,4-tetrahydronaphth-2-yl)-1,2,5,6-tetrahydro-pyridin-3-carbonsäuremethylester als gelbes Oel, das aus Diäthyläther/Pentan kristallisiert; Smp. 65°-66°. Das mit Salzsäure in Diäthyläther hergestellte Hydrochlorid kristallisiert aus Aceton/Diäthyläther mit dem Smp. 210-212° (Zers.).

Beispiel 29: 7,7 g (26 mMol) Methansulfonsäure[2-(6-methoxyindan-1-yl)äthyl]ester werden in 20 ml Toluol gelöst und bei Raumtemperatur zu einer Lösung von 6,3 g (28.6 mMol) Guvacolin-hydrobromid und 12.7 g (57 mMol) N-Aethyl-N,N-diisopropyl-amin in 15 ml Dimethylformamid gegeben. Das Gemisch wird 3 Tage bei Raumtemperatur stehengelassen und anschliessend unter vermindertem Druck bei 40-50° vom Lösungsmittel befreit.

Der erhaltene Rückstand wird in 2n-Salzsäure gelöst, mit Diäthyläther ausgeschüttelt und die saure wässrige Lösung mit Natriumhydrogencarbonat unter Eiskühlung basisch gestellt. Das erhaltene Gemisch wird mit Diäthyläther ausgeschüttelt, die ätherischen Phasen mit gesättigter Kochsalzlösung gewaschen und über Magnesiumsulfat getrocknet. Der erhaltene 1-[2-(6-Methoxyindan-1-yl)äthyl]-1,2,5,6-tetrahydro-pyridin-3-carbonsäuremethylester wird mit Cyclohexan an 6 g Kieselgel chromatographisch gereinigt und ergibt ein gelbes Oel.

Die Rohbase wird mit ätherischer Salzsäure in das Hydrochlorid überführt und dieses aus Isopropanol/Diäthyläther umkristallisiert. Man erhält 1-[2-(6-Methoxyindan-1-yl)äthyl]-1,2,5,6-tetrahydro-pyridin-3-carbonsäuremethylester-hydrochlorid, das bei 145-148° schmilzt.

Die Ausgangsstoffe werden z.B. wie folgt erhalten:

15,7 g (76 mMol) 6-Methoxyindan-1-ylessigsäure werden in 150 ml Tetrahydrofuran gelöst. Dann wird unter Rühren bei 40-50° mit einem Stickstoffstrom Diboran eingeleitet, erzeugt durch Eintropfen von 21,6 g (152 mMol) Bortrifluorid-ätherat in eine Suspension von 4,3 g (114 mMol) Natriumborhydrid in 60 ml Diäthylenglykol bei 40-60° unter Rühren. Nach Beendigung der Diboranerzeugung (ca. 1 Stunde) wird die Reaktionslösung 8 Stunden am Rückfluss gehalten, mit einem Eisbad abgekühlt und langsam mit 140 ml einer 1 molaren Natriumdihydrogenphosphatlösung versetzt.

Das Gemisch wird unter vermindertem Druck eingeengt, mit 50 ml 2n-Natronlauge alkalisch gestellt und mit Diäthyläther ausgeschüttelt. Die ätherischen Lösungen werden mit gesättiger Kochsalzlösung gewaschen und über Magnesiumsulfat getrocknet. Nach Entfernen des Lösungsmittel erhält man rohes 2-(6-Methoxyindan-1-yl)äthanol als gelbliches, viskoses Oel.

5,0 g (26,0 mMol) rohes 2-(6-Methoxyindan-1-yl)äthanol werden in 50 ml Dichlormethan gelöst und unter Eiskühlung tropfenweise mit 3,6 g (31 mMol) Methansulfonsäurechlorid und 3,1 g (31 mMol) Triäthylamin versetzt. Das Gemisch wird 3 Stunden bei Raumtemperatur gerührt, vom ausgefallenen Triäthylammoniumchlorid abfiltriert und das Filtrat mit Eiswasser ausgeschüttelt. Die organischen Phasen werden mit gesättigter Kochsalzlösung gewaschen, über Magnesiumsulfat getrocknet und unter vermindertem Druck bei 40° eingedampft. Man erhält Methansulfonsäure-2-(6-methoxyindan-1-yl)äthylester, der ohne weitere Reinigung eingesetzt werden kann.

Beispiel 30: Eine Lösung von 4,05 g (15 mMol) von 4-Hydroxy-1-[(5-methoxybenzocyclobuten-1-yl)-methyl]-1,2,5,6-tetrahydro-pyridin-3-carbonsäurenitril-hydrochlorid in 100 ml 95%igem Aethanol wird mit 1,5 ml konzentrierter Salzsäure versetzt und 15 Stunden zum Rückfluss erhitzt. Nach dem Erkalten wird unter verminderten Druck auf etwa 30 ml eingeengt und diese Lösung in ein Gemisch von 5n-Salzsäure und 20

ml Toluol eingegossen, worauf beim Rühren und Abkühlen 4-Hydroxy-1-[(5-methoxybenzocyclobuten-1-yl)-methyl]-1,2,5,6-tetrahydropyridin-3-carbonsäureäthylester-bzw. 1-[(5-Methoxybenzocyclobuten-1-yl)methyl]-4-oxo-piperidin-3-carbonsäuräthylester-hydrochlorid vom Smp. 181-182° C auskristallisiert.

Das Ausgangsmaterial kann man z.B. folgendermassen herstellen:

In Analogie zu Beispiel 1 kann man ausgehend von 3.6 g (22 mMol) 5-Methoxybenzocyclobuten-1-ylmethylamin und 2.4 ml (22 mMol) Acrylsäureäthylester in 70 ml Toluol bei Raumtemperatur 3-(5-Methoxybenzocyclobuten-1-ylmethylamino)propionsäureäthylester-hydrochlorid vom Smp. 129-130° herstellen.

30,16 g (0.1 Mol) 3-(5-Methoxybenzocyclobuten-1-ylmethylamino)propionsäureäthylester werden in 250 ml Aethanol mit 10,5 g (0,1 Mol) Triäthylamin und 5,84 g (0,11 Mol) Acrylnitril versetzt und 15 Stunden bei Raumtemperatur gerührt. Dann wird im Wasserstrahlvakuum eingeengt, der Rückstand in Diäthyläther aufgenommen und mit Eiswasser neutralgewaschen. Die ätherische Lösung wird über Kaliumcarbonat getrocknet, filtriert und eingedampft; man erhält 3-{N-(2-Cyanoäthyl)-N-[(5-methoxybenzocyclobuten-1-yl)-methyl]amino}propionsäureäthylester als gelbes Oel.

Man versetzt eine Suspension von 5,73 g Natriumhydrid (etwa 55%-ig in Mineralöl) in 100 ml Tetrahydrofuran unter einer Stickstoffatmosphäre tropfenweise mit einer Lösung von 13,07 g (41.3 mMol) 3-{N-(2-Cyanoäthyl)-N-[(5-methoxybenzocyclobuten-1-yl)methyl]-amino}-propionsäureäthylester in 200 ml Tetrahydrofuran und rührt 16 Stunden bei Raumtemperatur. Nach Zusatz von 70 ml 2n-Schwefelsäure erhält man eine gelbe Lösung. Diese versetzt man mit 300 ml Diäthyläther und 100 ml Wasser, wobei 2 Schichten entstehen. Die wässrige Schicht wird dreimal mit je 100 ml Diäthyläther extrahiert. Die vereinigten organischen Phasen werden über Natriumsulfat getrocknet, unter vermindertem Druck auf etwa 100 ml eingeengt und in ein Gemisch von 80 ml 5n-Salzsäure und 20 ml Toluol eingegossen, worauf beim Rühren und Abkühlen 4-Hydroxy-1-[(5-methoxybenzocyclobuten-1-yl)methyl]-1,2,5,6-tetrahydro-pyridin-3-carbonsäurenitril-hydrochlorid bzw. 1-[(5-Methoxybenzocyclobuten-1-yl)-methyl]-4-oxo-piperidin-3-carbonsäurenitril-hydrochlorid auskristallisiert.

Beispiel 31: Eine Lösung von 10,2 g (0.04 Mol) 1-[(5-Methoxybenzocyclobuten-1-yl)methyl]-1,2,5,6-tetrahydropyridin-3-carbonsäurenitril in 200 ml 96%-igem Aethanol wird mit 4 ml konzentrierter Schwefelsäure versetzt und 16 Stunden zum Rückfluss erhitzt. Nach dem Erkalten wird unter vermindertem Druck auf 50 ml eingeengt, der Rückstand in 200 ml Dichlormethan und 100 ml Wasser verteilt und mit gesättigter Natriumhydrogencarbonatlösung neutralisiert. Die organische Phase wird abgetrennt und die wässrige Phase noch einmal mit 100 ml Dichlormethan nachgeschüttelt. Die vereinigten organischen Phasen werden über Natriumsulfat getrocknet, filtriert und eingedampft. Das Rohprodukt wird an 250 g Kieselgel chromatographisch gereinigt, mit äthanolischer Salzsäure ins Hydrochlorid überführt und aus Aethanol/Diäthyläther kristallisiert. Man erhält 1-[(5-Methoxybenzocyclobuten-1-yl)methyl]-1,2,5,6-tetrahydro-pyridin-3-carbonsäureäthylester-hydrochlorid vom Smp. 204,5-205,5°.

Das Ausgangsmaterial kann man z.B. folgendermassen herstellen:

42,2 g (0,1 Mol) Methansulfonsäure(5-methoxybenzocyclobuten-1-ylmethyl)ester, 11,9 g (0,11 Mol) 1,2,5,6-Tetrahydropyridin-3-carbonsäurenitril und 15 g N-Aethyl-N,N-diisopropyl-amin werden unter Stickstoff in 250 ml Dimethylformamid gelöst und 16 Stunden gerührt. Man engt unter vermindertem Druck auf etwa 100 ml ein, fügt 300 ml Wasser hinzu und schüttelt dreimal mit je 150 ml Dichlormethan aus. Die vereinigten organischen Phasen werden über Natriumsulfat getrocknet, filtriert und eingedampft. Der Rückstand wird durch Chromatographie an 500 g Kieselgel mit Toluol/Aethylacetat (19:1) als Laufmittel gereinigt. Man erhält 1-[(5-Methoxybenzocyclobuten-1-yl)methyl]-1,2,5,6-tetrahydro-pyridin-3-carbonsäurenitril als gelbes Oel.

Beispiel 32: Eine Lösung von 19,1 g (50 mMol) eines Gemisches von cis- und trans-4-Brom-1-[(5-methoxybenzocyclobuten-1-yl)methyl]piperidin-3-carbonsäureäthylester und 8,2 g (0,1 Mol) wasserfreiem Natriumacetat in 100 ml Eisessig wird 16 Stunden zum Rückfluss erhitzt. Nach dem Abkühlen engt man unter vermindertem Druck ein, verteilt zwischen Dichlormethan und wässriger Natriumcarbonatlösung, trocknet die vereinigten organischen Phasen über Natriumsulfat und dampft ein. Das erhaltene, ölige Gemisch von cis- und trans-4-Acetoxy-1-[(5-methoxybenzocyclobuten-1-yl)methyl]piperidin-3-carbonsäureäthylester wird durch Chromatographie an 600 g Kieselgel (60, Merck) mit Trichlormethan/Methanol (98:2) als Laufmittel gereinigt. Das trans-Isomere wird zuerst, das cis-Isomere zuletzt isoliert. Von letzterem wird mit ätherischer Salzsäurelösung das Hydrochlorid bereitet. Sein Smp. beträgt 202-203° C.

Das Ausgangsmaterial kann man z.B. folgendermassen herstellen:

Unter Rühren werden 30,14 g (0,1 Mol) 1-[(5-Methoxybenzocyclobuten-1-yl)methyl]-1,2,5,6-tetrahydro-pyridin-3-carbonsäureäthylester portionsweise in 150 ml einer 33%igen Lösung von Bromwasserstoff in Eisessig eingetragen. Nach 18 Stunden Rühren bei Raumtemperatur wird unter vermindertem Druck eingeengt und zwischen Dichlormethan und wässriger Natriumcarbonatlösung verteilt. Die organische

Phase wird über Natriumsulfat getrocknet und das Lösungsmittel unter vermindertem Druck abgedampft. Man erhält ein Gemisch von cis- und trans-4-Brom-1-[(5-methoxybenzocyclobuten-1-yl)methyl]-piperidin-3-carbonsäureäthylester als gelbes Oel.

Beispiel 33: Man versetzt eine Suspension von 4,8 g Natriumhydrid (50%ige Suspension in Mineralöl) in 100 ml trockenem Tetrahydrofuran mit einer Lösung von 11 g (0,1 Mol) Benzylalkohol in 100 ml Tetrahydrofuran und erhitzt nach Abklingen der Gasreaktion zum Rückfluss. Nach Erkalten fügt man tropfenweise eine Lösung von 30,14 g (0,1 Mol) 1-[(5-Methoxybenzocyclobuten-1-yl)methyl]-1,2,5,6-tetrahydropyridin-3-carbonsäureäthylester in 200 ml Tetrahydrofuran hinzu und erhitzt erneut 5 Stunden zum Rückfluss. Nach Erkalten wird das Lösungsmittel eingedampft. Man erhält ein Gemisch von cis- und trans-4-Benzyloxy-1-[(5-methoxybenzocyclobuten-1-yl)methyl]-piperidin-3-carbonsäureäthylester als Oel.

Beispiel 34: 10,24 g (25 mMol) eines Gemisches von cis- und trans-4-Benzyloxy-1-[(5-methoxybenzocyclobuten-1-yl)methyl]piperidin-3-carbonsäureäthylester werden in 150 ml 95 % Ethanol gelöst, mit 2 g 10 % Palladium auf Kohle versetzt und in einer Parr-Apparatur 12 Stunden bei Raumtemperatur hydriert. Das Reaktionsgemisch wird über Diatomeenerde filtriert und zur Trockne eingedampft. Der Eindampfrückstand wird über 400 g Kieselgel mit Toluol/Essigester 19:1 als Laufmittel chromatographiert. Zunächst wird trans-4-Hydroxy-1-[(5-methoxybenzocyclobuten-1-yl)methyl]piperidin-3-carbonsäureäthylester und dann cis-4-Hydroxy-1-[(5-methoxybenzocyclobuten-1-yl)methyl]piperidin-3-carbonsäureäthylester isoliert. (Smp. des Hydrochlorids: 156-160°).

Beispiel 35: In eine Lösung von 25,68 g (50 mMol) 1-[(5-Methoxybenzocyclobuten-1-yl)methyl]-4-äthoxy-3-äthoxycarbonyl-pyridiniumtosylat in 250 ml 96%-igen Aethanol wird in einer Stickstoffatmosphäre bei 0° innerhalb von 30 Minuten 3,3 g Natriumborhydrid eingetragen. Nach 1 Stunde Rühren bei 0° und 2 Stunden bei Raumtemperatur wird die Lösung unter vermindertem Druck eingedampft, der Rückstand mit 70 ml Wasser versetzt und dreimal mit je 100 ml Dichlormethan extrahiert. Die organischen Phasen werden vereinigt, über Magnesiumsulfat getrocknet und eingedampft. Der Rückstand wird mit einem Gemisch von 80 ml 5n-Salzsäure und 20 ml Toluol versetzt, worauf beim Rühren und Abkühlen 4-Hydroxy-1-[(5-methoxybenzocyclobuten-1-yl)methyl]-1,2,4,5-tetrahydro-pyridin-3-carbonsäureäthylester-hydrochlorid bzw. 1-[(5-Methoxybenzocyclobuten-1-yl)methyl]-4-oxo-piperidin-3-carbonsäureäthylester-hydrochlorid vom Smp. 181-182° C auskristallisieren.

Das Ausgangsmaterial kann z.B. folgendermassen hergestellt werden:

Man versetzt eine Lösung von 31,84 g (0,1 Mol) p-Toluolsulfonsäure(5-methoxybenzocyclobuten-1-yl)-methylester in 150 ml 95%-igem Aethanol mit 19,52 g 4-Aethoxynikotinsäureäthylester und rührt 3 Tage bei Raumtemperatur. Nach Abdestillieren des Lösungsmittels unter vermindertem Druck wird 1-[(5-Methoxybenzocyclobuten-1-yl)methyl]-3-äthoxycarbonyl-4-äthoxy-pyridinium-tosylat als hellgelber Schaum erhalten.

Beispiel 36: Man versetzt eine Lösung von 2,43 g (45 mMol) Natriummethanolat in 15 ml Dimethylformamid mit einer Lösung von 12,78 g (40 mMol) 3-[N-(5-Methoxybenzocyclobuten-1-ylmethyl)-N-(3-oxopropyl)-amino]propionsäureäthylester in 100 ml Dimethylformamid und rührt 3 Stunden bei 40° C. Hierauf wird das Lösungsmittel unter vermindertem Druck abgedampft und der Rückstand durch Chromatographie an 400 g Kieselgel mit Dichlormethan/Methanol (95:5) in die Komponenten aufgetrennt. Zuerst wird trans-4-Hydroxy-1-[(5-methoxybenzocyclobuten-1-yl)methyl]-piperidin-3-carbonsäureäthylester und hierauf cis-4-Hydroxy-1-[(5-methoxybenzocyclobuten-1-yl)methyl]-piperidin-3-carbonsäureäthylester, dessen Hydrochlorid einen Smp. von 156-160° C aufweist, isoliert.

Das Ausgangsmaterial kann z.B. folgendermassen hergestellt werden:

30,16 g (0,1 Mol) 3(5-Methoxybenzocyclobuten-1-ylmethylamino)propionsäureäthylester-hydrochlorid werden in 250 ml Dioxan mit 10,5 g (0,1 Mol) Triäthylamin und 20 g (0.11 Mol) 2-(2-Bromäthyl)-1,3-dioxolan 20 Stunden bei 80° gerührt. Nach dem Erkalten wird das Lösungsmittel bei vermindertem Druck abgedampft, der Rückstand in Diäthyläther aufgenommen und mit Eiswasser neutral gewaschen. Die ätherische Lösung wird über Kaliumcarbonat getrocknet und eingedampft, worauf man 3-[N-(1,3-Dioxolan-2-yl)-N-(5-methoxybenzocyclobuten-1-ylmethyl)-amino]propionsäureäthylester als gelbes Oel isoliert.

Eine Lösung von 21,8 g (60 mMol) desselben in 300 ml Dichlormethan wird mit 200 g Kieselgel, welches nach J.M. Conia et al., Synthesis 1978, 63 mit 10 % wässriger Oxalsäurelösung imprägniert wurde, versetzt und die Suspension 3 Stunden bei Raumtemperatur gerührt. Nach Abfiltrieren wascht man mit 5 % wässriger Natriumhydrogencarbonatlösung, trocknet die organische Phase mit Natriumsulfat und dampft unter vermindertem Druck ein. Der Eindampfrückstand wird zur Reinigung über 600 g Kieselgel mit Toluol/Aethylacetat (19:1) als Laufmittel chromatographiert. Man erhält 3-[N-(5-Methoxybenzocyclobuten-1-ylmethyl)-N-(3-oxopropyl)-amino]propionsäureäthylester als gelbes Oel.

Beispiel 37: 2 g (5,7 mMol) 4-Hydroxy-1-[(5-methoxybenzocyclobuten-1-yl)methyl]-1,2,5,6-tetrahydro-pyridin-3-carbonsäureäthylester-hydrochlorid werden in 80 ml Dichlormethan bei -80 °C unter Argon vorgelegt. Unter Rühren wird 1,08 ml (11,3 mMol) Bortribromid zudosiert. Nach 30 Minuten bei -80 °C wird das Reaktionsgemisch unter Rühren innerhalb von 2 Stunden allmählich auf +5 °C erwärmt. Dann wird vorsichtig mit 40 ml Aethanol versetzt und anschliessend im Wasserstrahlvakuum eingeengt. Der Rückstand wird in 150 ml Aethanol gelöst und erneut im Wasserstrahlvakuum eingedampft. Dies wird noch zweimal wiederholt. Der erhaltene Rückstand wird dann aus Aethanol/Diäthyläther kristallisiert. Man erhält 4-Hydroxy-1-[(5-hydroxybenzocyclobuten-1-yl)methyl]-1,2,5,6-tetrahydro-pyridin-3-carbonsäureäthylester- bzw. 1-[(5-Hydroxybenzocyclobuten-1-yl)methyl]-4-oxo-piperidin-3-carbonsäureäthylester-hydrobromid vom Smp. 192-194 °C.

Beispiel 38: In Analogie zu Beispiel 1 kann man ausgehend von 24 g (72 mMol) N-(Benzocyclobuten-1-ylmethyl)imino-di(3-propionsäure)diäthylester und 4,7 g (94 mMol) Natriumhydrid (50 % Dispersion in Mineralöl) in 650 ml Toluol 4-Hydroxy-1-[(benzocyclobuten-1-yl)methyl]-1,2,5,6-tetrahydro-pyridin-3-carbonsäureäthylester-hydrochlorid bzw. 1-[(Benzocyclobuten-1-yl)methyl]-4-oxo-piperidin-3-carbonsäureäthylester-hydrochlorid vom Smp. 182-183 °C herstellen.

Das Ausgangsmaterial, N-[(Benzocyclobuten-1-yl)methyl]imino-di(3-propionsäure)diäthylester (Hydrochlorid: Smp. 87-89 °) kann man ausgehend von 15 g (114 mMol) Benzocyclobuten-1-ylmethylamin und 37 ml (340 mMol) Acrylsäureäthylester in 200 ml Aethanol analog wie in Beispiel 1 beschrieben herstellen.

Beispiel 39: In Analogie zu Beispiel 1 kann man ausgehend von 1,9 g (39 mMol) Natriumhydrid (50 % Dispersion in Mineralöl) und 11 g (30 mMol) N-(5-Methoxy-1-methyl-benzocyclobuten-1-ylmethyl)-imino-di-(3-propionsäure)diäthylester-hydrochlorid in 280 ml Toluol 4-Hydroxy-1-[(5-methoxy-1-methyl-benzocyclobuten-1-yl)methyl]-1,2,5,6-tetrahydropyridin-3-carbonsäureäthylester-bzw. 1-[(5-Methoxy-1-methyl-benzocyclobuten-1-yl)methyl]-4-oxo-piperidin-3-carbonsäureäthylester-hydrochlorid vom Smp. 108 °C herstellen.

N-(5-Methoxy-1-methyl-benzocyclobuten-1-ylmethyl)imino-di(3-propionsäure)diäthylester-hydrochlorid vom Smp. 122-125 ° kann analog wie in Beispiel 1 beschrieben ausgehend von 10 g (56 mMol) 5-Methoxy-1-methylbenzocyclobuten-1-ylmethylamin und 18 ml (169 mMol) Acrylsäureäthylester in 100 ml Aethanol erhalten werden.

Das Ausgangsmaterial kann z.B. folgendermassen erhalten werden:

5 g (29 mMol) 5-Methoxy-1-methyl-benzocyclobuten-1-carbonsäurenitril werden in 50 ml Aethanol gelöst und mit 5 g (290 mMol) flüssigem Ammoniak und 1 g Raney-Nickel versetzt. Dann wird das Reaktionsgemisch 1 Stunde bei 90 °C und $10^7$ Pa hydriert. Das abgekühlte Reaktionsgemisch wird über Diatomeenerde filtriert und zur Trockne eingedampft. Der Rückstand wird in Aethanol aufgenommen, mit Salzsäure in das Hydrochlorid überführt und durch Zugabe von Diäthyläther kristallisiert. Man erhält so 5-Methoxy-1-methyl-benzocyclobuten-1-ylmethylammoniumchlorid vom Smp. 159-160 °C.

Beispiel 40: Analog zu Beispiel 3 erhält man cis-4-Hydroxy-1-[(benzocyclobuten-1-yl)methyl]-piperidin-3-carbonsäureäthylester-hydrochlorid vom Smp. 147-148 °, indem man 3,2 g (10 mMol) 4-Hydroxy-1-[-(benzocyclobuten-1-yl)methyl]-1,2,5,6-tetrahydro-pyridin-3-carbonsäureäthylesterhydrochlorid unter Zusatz von 400 mg Platinoxid in 100 ml Aethanol bei $4 \cdot 10^5$ Pa hydriert.

Beispiel 41: In Analogie zu Beispiel 1 kann man ausgehend von 5,9 g (16 mMol) N-(Benzocyclobuten-1-yl)imino-di(3-propionsäure)diäthylester und 1 g einer 50%-igen Suspension von Natriumhydrid in Toluol 4-Hydroxy-1-(benzocyclobuten-1-yl)-1,2,5,6-tetrahydropyridin-3-carbonsäureäthylester-hydrochlorid bzw. 1-(Benzocyclobuten-1-yl)-4-oxo-piperidin-3-carbonsäureäthylester-hydrochlorid vom Smp. 208-209 °C herstellen.

Das Ausgangsmaterial, N-(Benzocyclobuten-1-yl)imino-di(3-propionsäure)diäthylester, kann in analoger Weise wie in Beispiel 1 beschrieben hergestellt werden; Rf = 0.3, Hexan/Essigsäureäthylester (4:1), wobei man von 3,5 g (29 mMol) Benzocyclobuten-1-ylamin und 9,6 ml (88 mMol) Acrylsäureäthylester in 100 ml Aethanol ausgeht.

Beispiel 42: 2 g (5,6 mMol) cis-4-Hydroxy-1-[(5-methoxybenzocyclobuten-1-yl)methyl]-piperidin-3-carbonsäureäthylester-hydrochlorid und 20 ml Essigsäureanhydrid werden in 100 ml Toluol 18 Stunden unter Rückfluss erhitzt. Dann werden zum abgekühlten Reaktionsgemisch 200 ml Eiswasser zugegeben und 30 Minuten verrührt. Mit 2n-Natronlauge wird auf pH 10 alkalisch gestellt und dann die Toluol-Phase abgetrennt. Die Wasserphase wird zweimal mit Dichlormethan extrahiert. Die organischen Phasen werden vereinigt, über Natriumsulfat getrocknet und eingedampft.

Das so erhaltene Rohprodukt wird über die 10-fache Menge Kieselgel mit Diäthyläther als Laufmittel chromatographisch gereinigt. Das gereinigte Produkt wird ins Hydrochlorid überführt und aus Aethanol/Diäthyläther kristallisiert. Man erhält cis-4-Acetyloxy-1-[(5-methoxybenzocyclobuten-1-yl)methyl]-piperidin-3-carbonsäureäthylester-hydrochlorid vom Smp. 202-203°.

Beispiel 43: Analog zu Beispiel 5 kann man ausgehend von 9.2 g (32 mMol) cis-4-Hydroxy-1-[-(benzocyclobuten-1-yl)methyl]-piperidin-3-carbonsäureäthylester, 9 ml (64 mMol) Triäthylamin, 4,3 g (35 mMol) 4-Dimethylaminopyridin, 2,7 ml (35 mMol) Methansulfonsäurechlorid in 100 ml Dichlormethan und nachfolgende Behandlung mit 10 g Kaliumhydroxid in 140 ml Aethanol bei Raumtemperatur 1-[-(Benzocyclobuten-1-yl)methyl]-1,2,5,6-tetrahydro-pyridin-3-carbonsäureäthylester-hydrochlorid vom Smp. 157-158° herstellen.

Beispiel 44: 3,8 g (13 mMol) trans-4-Hydroxy-1-[(benzocyclobuten-1-yl)methyl]-piperidin-3-carbonsäureäthylester, 3,6 ml (26 mMol) Triäthylamin und 1,8 g (14 mMol) 4-Dimethylaminopyridin werden in 100 ml Dichlormethan vorgelegt. Dazu wird bei Raumtemperatur 1,1 ml Methansulfonsäurechlorid gegeben. Man rührt 30 Stunden nach, verdünnt das Reaktionsgemisch mit Dichlormethan, wäscht 3-mal mit Wasser und trocknet über Natriumsulfat. Das Rohprodukt wird chromatographisch an Kieselgel gereinigt. Das gereinigte Produkt wird mit äthanolischer Salzsäure in das Hydrochlorid überführt und durch Zugabe von Diäthyläther auskristallisiert.

Man erhält trans-1-[(Benzocyclobuten-1-yl)methyl]-4-methansulfonyloxy-piperidin-3-carbonsäureäthylester-hydrochlorid vom Smp. 145-146°C.

Das Ausgangsmaterial wird in Analogie zu Beispiel 4 hergestellt, wobei man von 8 g (28 mMol) 4-Hydroxy-1-[(benzocyclobuten-1-yl)methyl]-1,2,5,6-tetrahydropyridin-3-carbonsäureäthylester und 0,55 g (14 mMol) Natriumborhydrid in 160 ml 50%-igem wässrigem Aethanol ausgeht.

Beispiel 45: 1 g (2,8 mMol) cis-4-Hydroxy-1-[(5-methoxybenzocyclobuten-1-yl)methyl]-piperidin-3-carbonsäureäthylester-hydrochlorid und 20 ml 25%-iger wässriger Ammoniak werden mit 5 ml Aethanol bei 60°C im Bombenrohr 8 Stunden verrührt. Das Reaktionsgemisch wird dann mit Wasser verdünnt und zweimal mit Trichlormethan extrahiert. Die Extrakte werden über Natriumsulfat getrocknet und eingedampft. Das erhaltene Rohprodukt wird in Methanol gelöst, mit Methansulfonsäure auf pH 3 titriert und durch Zugabe von Diäthyläther auskristallisiert. Man erhält 4-Hydroxy-1-[(5-methoxybenzocyclobuten-1-yl)methyl]-piperidin-3-carbonsäureamidmethansulfonat vom Smp. 167-177°C.

Beispiel 46: Analog zu Beispiel 1 kann man ausgehend von 1 g (20 mMol) Natriumhydrid (50%-ige Dispersion in Mineralöl) und 5,7 g (15,5 mMol) N-[(5-Chlorbenzocyclobuten-1-yl)methyl]imino-di(3-propionsäure)diäthylester in 180 ml Toluol 4-Hydroxy-1-[(5-chlorbenzocyclobuten-1-yl)methyl]-1,2,5,6-tetrahydro-pyridin-3-carbonsäureäthylester-hydrochlorid bzw. 1-[(5-Chlorbenzocyclobuten-1-yl)methyl]-4-oxo-piperidin-3-carbonsäureäthylester-hydrochlorid vom Smp. 161-163°C herstellen.

Das Ausgangsmaterial, N-[(5-Chlor-benzocyclobuten-1-yl)methyl]imino-di(3-propionsäure)diäthylester-hydrochlorid vom Smp. 97-99°, kann analog wie in Beispiel 1 beschrieben ausgehend von 4 g (24 mMol) 5-Chlorbenzocyclobuten-1-ylmethylamin und 7,8 ml (72 mMol) Acrylsäureäthylester in 80 ml Aethanol hergestellt werden.

Das dazu benötigte 5-Chlorbenzocyclobuten-1-ylmethylamin, Smp. des Hydrochlorids: 240-241°, kann in Analogie zu Beispiel 6 ausgehend von 5 g (31 mMol) 5-Chlor-benzocyclobuten-1-carbonsäurenitril und 4,6 ml (46 mMol) Boran/Dimethylsulfid (10-molar in Tetrahydrofuran) in 100 ml Tetrahydrofuran hergestellt werden.

Beispiel 47: In Analogie zu Beispiel 1 kann ausgehend von 1,7 g (36 mMol) Natriumhydrid (50%-ige Dispersion in Mineralöl) und 10 g (27 mMol) N-[(4-Methoxybenzocyclobuten-1-yl)methyl]imino-di(3-propionsäure)diäthylester in 220 ml Toluol 4-Hydroxy-1-[(4-methoxybenzocyclobuten-1-yl)methyl]-1,2,5,6-tetrahydro-pyridin-3-carbonsäureäthylester-hydrochlorid bzw. 1-[(4-Methoxybenzocyclobuten-1-yl)methyl]-4-oxo-piperidin-3-carbonsäureäthylester-hydrochlorid vom Smp. 149-151°C hergestellt werden.

Das Ausgangsmaterial kann in Analogie zu Beispiel 1 wie folgt hergestellt werden:

Aus 6,2 g (38 mMol) 4-Methoxybenzocyclobuten-1-ylmethylamin und 12 ml (112 mMol) Acrylsäureäthylester erhält man N-[(4-Methoxybenzocyclobuten-1-yl)methyl]imino-di(3-propionsäure)diäthylester als viskoses Oel; Rf = 0,3, Hexan/Essigsäureäthylester (4:1).

Das dazu benötigte 4-Methoxybenzocyclobuten-1-ylmethylamin, Smp. des Hydrochlorides: 208-210°, kann analog wie in Beispiel 6 beschrieben aus 13 g (80 mMol) 4-Methoxybenzocyclobuten-1-carbonsäurenitril und 12 ml (12 mMol) Boran/Dimethylsulfid (10-molar in Tetrahydrofuran) in 70 ml Tetrahydrofuran hergestellt werden.

Beispiel 48: Analog zu Beispiel 1 kann man ausgehend von 1,1 g (23 mMol) Natriumhydrid (50%-ige Dispersion in Mineralöl) und 6,6 g (17,5 mMol) N-[(5-Methoxy-3-methyl-benzocyclobuten-1-yl)methyl]imino-di(3-propionsäure)diäthylester in 230 ml Toluol 4-Hydroxy-1-[(5-methoxy-3-methylbenzocyclobuten-1-yl)-methyl]-1,2,5,6-tetrahydro-pyridin-3-carbonsäureäthylester-hydrochlorid bzw. 1-[(5-Methoxy-3-methyl-benzocyclobuten-1-yl)methyl]-4-oxo-piperidin-3-carbonsäureäthylester-hydrochlorid vom Smp. 160-162° herstellen.

Das Ausgangsmaterial kann in Analogie zu Beispiel 1 wie folgt hergestellt werden:
Aus 4,1 g (23 mMol) 5-Methoxy-3-methyl-benzocyclobuten-1-ylmethylamin und 7,5 ml (69,5 mMol) Acrylsäureäthylester erhält man N-[(5-Methoxy-3-methyl-benzocyclobuten-1-yl)methyl]imino-di(3-propionsäure)-diäthylester als viskoses Oel; Rf = 0,32, Hexan/Essigsäureäthylester (4:1).

Das Ausgangsmaterial kann in Analogie zu Beispiel 6 durch Hydrierung von 4,4 g (25 mMol) 5-Methoxy-3-methyl-benzocyclobuten-1-carbonsäurenitril mit 0,5 g Raney-Nickel und 5 g Ammoniak in 50 ml Aethanol hergestellt werden; Smp. des Hydrochlorides: 228-229°.

Beispiel 49: In Analogie zu Beispiel 1 kann man ausgehend von 110 mg (2,3 mMol) Natriumhydrid (50%-ige Dispersion in Mineralöl) und 580 mg (1,5 mMol) N-[2-(5-Methoxy-benzocyclobuten-1-yliden)äthyl]-imino-di(3-propionsäure)diäthylester in 60 ml Toluol 4-Hydroxy-1-[2-(5-methoxybenzocyclobuten-1-yliden)-äthyl]-1,2,5,6-tetrahydro-pyridin-3-carbonsäureäthylester-hydrochlorid bzw. 1-[2-(5-Methoxybenzocyclobuten-1-yliden)äthyl]-4-oxo-piperidin-3-carbonsäureäthylester-hydrochlorid vom Smp. 167-169° herstellen.

Das Ausgangsmaterial kann wie folgt hergestellt werden:
10 g (63 mMol) 5-Methoxybenzocyclobuten-1-carbonsäurenitril werden bei -60°C unter Argon in 200 ml Tetrahydrofuran vorgelegt. Unter Rühren werden 75 ml (75 mMol) einer 1-molaren Lösung von Diisobutylaluminiumhydrid in Hexan langsam zugetropft. Dann lässt man das Reaktionsgemisch langsam auf Raumtemperatur erwärmen und lässt weitere 4 Stunden rühren. Bei Raumtemperatur wird dann 500 ml gesättigte Ammoniumchlorid-Lösung zugesetzt und 40 Minuten nachgerührt. Man versetzt mit 220 ml 5%-iger Schwefelsäure und extrahiert zweimal mit Diäthyläther. Die vereinigten organischen Phasen werden getrocknet und eingeengt. Das Rohprodukt wird an der zehnfachen Menge Kieselgel chromatographisch gereinigt. Man erhält 5-Methoxybenzocyclobuten-1-carboxaldehyd, Rf = 0.23, Hexan/Essigsäureäthylester (9:1).

5,5 g (34 mMol) 5-Methoxybenzocyclobuten-1-carboxaldehyd und 13,8 g (42 mMol) Caesiumcarbonat werden in 55 ml Dioxan unter Argon vorgelegt. Unter Rühren wird 8,7 g (39 mMol) Phosphonoessigsäuretriäthylester zugegeben und anschliessend 2 Stunden auf 80° erwärmt. Dann wird das Reaktionsgemisch eingeengt, in Dichlormethan aufgenommen und mit Wasser gewaschen. Die organische Phase wird über Natriumsulfat getrocknet und eingedampft. Das so erhaltene Rohprodukt wird an Kieselgel chromatographisch gereinigt. Man erhält 3-(5-Methoxybenzocyclobuten-1-yl)acrylsäureäthylester; Rf = 0,5, Hexan/Diäthyläther (4:1).

7,3 g (31,5 mMol) 3-(5-Methoxybenzocyclobuten-1-yl)-acrylsäureäthylester werden in 140 ml 50 % wässrigem Aethanol mit 7 g (125 mMol) Kaliumhydroxid 5 Stunden bei 50° verrührt. Dann wird das Reaktionsgemisch mit konzentrierter Salzsäure auf pH 1 gestellt und dreimal mit Dichlormethan extrahiert. Die vereinigten organischen Extrakte werden über Natriumsulfat getrocknet und eingedampft. Das Rohprodukt wird an Kieselgel chromatographisch gereinigt und dann aus Diäthyläther/Hexan kristallisiert. Man erhält so 3-(5-Methoxybenzocyclobuten-1-yliden)propionsäure vom Smp. 134-137°.

2,1 g (10 mMol) 3-(5-Methoxybenzocyclobuten-1-yliden)propionsäure und 1,4 ml (10 mMol, Triäthylamin werden in 20 ml Toluol unter Argon vorgelegt. Dazu gibt man 2,2 ml (10 mMol) Diphenylphosphorylazid und erwärmt unter Rühren 30 Minuten auf 80°. Man lässt auf Raumtemperatur abkühlen, gibt 2,9 ml (20 mMol) 2-Trimethylsilyläthanol hinzu und erwärmt 7 Stunden auf 80°. Dann wird das Reaktionsgemisch am Rotationsverdampfer zur Trockene eingeengt. Der ölige Rückstand wird in Diäthyläther aufgenommen, mit Wasser und 2n-Natronlauge gewaschen, über Natriumsulfat getrocknet und eingedampft. Das Rohprodukt wird an einer Kieselgelsäule chromatographisch gereinigt, mit 10 ml (10 mMol) einer 1-molaren Lösung von Tetrabutylammoniumfluorid in Tetrahydrofuran 2 Stunden bei 50° verrührt und dann zur Trockne eingedampft. Der Rückstand wird mit n-Pentan/Wasser (1:1) kräftig verrührt. Die n-Pentan-Phase wird abgetrennt und verworfen. Die Wasser-Phase wird mit Ammoniumchlorid auf pH 8 gestellt, worauf kristallines Produkt ausfällt. Dieses wird abgenutscht. Die Mutterlauge wird mit Kochsalz gesättigt, worauf weiteres Produkt auskristallisiert. Die Kristallisate werden vereinigt, in Trichlormethan/Methanol (1:1) aufgenommen, über Natriumsulfat getrocknet und eingedampft. Der kristalline Rückstand wird mit Diäthyläther verrührt und anschliessend filtriert. Man erhält 2-(5-Methoxybenzocyclobuten-1-yliden)äthylamin vom Smp. 238-240°.

580 mg (3,3 mMol) 2-(5-Methoxybenzocyclobuten-1-yliden)äthylamin werden in analoger Weise wie in Beispiel 1 beschrieben in 20 ml Aethanol mit 1,1 ml (9,9 mMol) Acrylsäureäthylester zu N-[2-(5-Methoxybenzocyclobuten-1-yliden)äthyl]imino-di(3-propionsäure)diäthylester; Rf = 0,14, Hexan/Essigsäureäthylester (4:1) umgesetzt.

Beispiel 50: In analoger Weise wie in Beispiel 1 beschrieben erhält man ausgehend von N-(5-Methoxyindan-2-yl)methyl]imino-di(3-propionsäure)diäthylester den 4-Hydroxy-1-[(5-methoxyindan-2-yl)-methyl]-1,2,5,6-tetrahydropyridin- bzw. 1-[(5-Methoxyindan-2-yl)methyl]-4-oxo-piperidin-3-carbonsäureäthylester und dessen Hydrochlorid.

Das Ausgangsmaterial kann man z.B. ausgehend von 5-Methoxyindan-1-on durch α-Metallierung und Umsetzung mit Chlorameisensäuremethylester zum 5-Methoxy-1-oxo-indan-2-carbonsäuremethylester, Hydrierung desselben zu 5-Methoxyindan-2-carbonsäuremethylester, Reduktion mit Lithiumaluminiumhydrid zum 5-Methoxyindan-2-methanol, Ueberführung desselben mittels Methansulfonsäurechlorid/Natriumazid und erneute Reduktion mit Lithiumaluminiumhydrid und Umsetzung des erhaltenen 5-Methoxyindan-2-ylmethylamins mit der doppeltmolaren Menge Acrylsäureäthylester erhalten.

Beispiel 51: In analoger Weise wie in Beispiel 29 beschrieben erhält man durch Umsetzung von Methansulfonsäure(5-methoxyindan-2-ylmethyl)ester, erhältlich aus 5-Methoxyindan-2-methanol (vergl. Beispiel 50), mit Guvacolin-hydrobromid und N-Aethyl-N,N-diisopropyl-amin den 1-[(5-Methoxyindan-2-yl)-methyl]-1,2,5,6-tetrahydropyridin-3-carbonsäuremethylester und sein Hydrochlorid.

Beispiel 52: In analoger Weise wie in Beispiel 1 beschrieben erhält man ausgehend von 5-Methoxyindan-2-ylamin, erhältlich ausgehend von 5-Methoxyindan-2-carbonsäuremethylester über 5-Methoxyindan-2-carbonsäureazid und Curtius-Abbau, und Acrylsäuremethylester den 4-Hydroxy-1-(5-methoxyindan-2-yl)-1,2,5,6-tetrahydro-pyridin- bzw. 1-(5-Methoxyindan-2-yl)-4-oxo-piperidin-3-carbonsäuremethylester und sein Hydrochlorid und aus diesem analog wie in den Beispielen 27 und 28 beschrieben 1-(5-Methoxyindan-2-yl)-1,2,5,6-tetrahydro-pyridin-3-carbonsäuremethylester und sein Hydrochlorid.

Beispiel 53: Durch katalytische Hydrierung von 1-[(5-Methoxybenzocyclobuten-1-yl)methyl]-1,2,5,6-tetrahydro-pyridin-3-carbonsäureäthylester erhält man 1-[(5-Methoxybenzocyclobuten-1-yl)methyl]piperidin-3-carbonsäureäthylester und durch Verseifung desselben 1-[(5-Methoxybenzocyclobuten-1-yl)methyl]-piperidin-3-carbonsäure sowie jeweils deren Hydrochloride.

Beispiel 54: Eine Lösung von 7,27 g (30 mMol) Methansulfonsäure-(5-methoxybenzocyclobuten-1-yl)-methylester in 60 ml Dimethylformamid wird zuerst mit 5,66 g (36 mMol) Piperidin-3-carbonsäureäthylester und anschliessend mit 9,70 g (75 mMol) N-Aethyl-N,N-diisopropyl-amin versetzt. Das Gemisch wird 18 Stunden bei 60° gerührt und nach Erkalten im Hochvakuum eingedampft. Der Rückstand wird in Diäthyläther aufgenommen, mit Wasser gewaschen und die organische Phase mit 2n-Salzsäure extrahiert. Die Salzsäureextrakte werden vereinigt, kalt mit konzentrierter Natronlauge alkalisch gestellt und mit Dichlormethan extrahiert. Die vereinigten organischen Phasen werden über Natriumsulfat getrocknet und unter verminderdem Druck zur Trockne eingedampft. Man erhält 1-[(5-Methoxybenzocyclobuten-1-yl)methyl]-piperidin-3-carbonsäureäthylester als gelbes Oel.

Das hieraus mit Salzsäure in Diäthyläther hergestellte Hydrochlorid kristallisiert aus Aethanol/Diäthyläther mit dem Smp. 193-195° (Zers.).

Beispiel 55: Eine Lösung von 5,10 g (15 mMol) 1-[(5-Methoxybenzocyclobuten-1-yl)methyl]piperidin-3-carbonsäureäthylester-hydrochlorid in 60 ml Aethanol wird mit 31.5 ml (31.5 mMol) n-Natronlauge versetzt. Nach 5 Minuten Rühren bei Raumtemperatur werden 40 ml Wasser zugegeben, das Gemisch auf 50°-60° erwärmt und 30 Minuten bei dieser Temperatur gehalten. Nach Erkalten wird die Lösung mit 15 ml konzentrierter Salzsäure versetzt und unter vermindertem Druck eingedampft. Der Rückstand wird in 150 ml heissem Aceton aufgenommen und vom unlöslichen Natriumchlorid getrennt. Nach Konzentrieren der Acetonlösung auf 50 ml kristallisiert das 1-[(5-Methoxybenzocyclobuten-1-yl)methyl]piperidin-3-carbonsaure-hydrochlorid vom Smp. 195°-198° aus.

Beispiel 56: Tabletten, enthaltend 25 mg Wirkstoff, z.B. 4-Hydroxy-1-[(5-methoxybenzocyclobuten-1-yl)-methyl]-1,2,5,6-tetrahydro-pyridin-3-carbonsäureäthylester-hydrochlorid bzw. 1-[(5-Methoxybenzocyclobuten-1-yl)methyl]-4-oxo-piperidin-3-carbonsäureäthylester-hydrochlorid, können folgendermassen hergestellt werden:

| Bestandteile (für 1000 Tabletten) | |
|---|---|
| Wirkstoff | 25,0 g |
| Lactose | 100,7 g |
| Weizenstärke | 7,5 g |
| Polyethylenglykol 6000 | 5,0 g |
| Talkum | 5,0 g |
| Magnesiumstearat | 1,8 g |
| entmineralisiertes Wasser | q.s. |

Herstellung: Sämtliche festen Ingredienzien werden zunächst durch ein Sieb mit 0,6 mm Maschenweite getrieben. Dann wird der Wirkstoff, die Lactose, das Talkum, das Magnesiumstearat und die Hälfte der Stärke vermischt. Die andere Hälfte der Stärke werden in 40 ml Wasser suspendiert und diese Suspension zu einer siedenden Lösung des Polyethylenglykols in 100 ml Wasser hinzugegeben. Der erhaltene Stärkekleister wird zu der Hauptmenge hinzugegeben und das Gemisch, wenn nötig unter Hinzufügen von Wasser, granuliert. Das Granulat wird über Nacht bei 35° getrocknet, durch ein Sieb mit 1,2 mm Maschenweite getrieben und zu beidseitig konkaven Tabletten von etwa 6 mm Durchmesser verpresst.

Beispiel 57: Tabletten, enthaltend 50 mg des Wirkstoffs, z.B. 4-Hydroxy-1-[(5-methoxybenzocyclobuten-1-yl)methyl]-1,2,5,6-tetrahydro-pyridin-3-carbonsäureäthylester-hydrochlorid bzw. 1-[(5-Methoxybenzocyclobuten-1-yl)methyl]-4-oxo-piperidin-3-carbonsäureäthylester-hydrochlorid, werden wie folgt hergestellt:

| Zusammensetzung (für 10 000 Tabletten) | |
|---|---|
| Wirkstoff | 500,00 g |
| Lactose | 140,80 g |
| Kartoffelstärke | 274,70 g |
| Stearinsäure | 10,00 g |
| Talk | 50,00 g |
| Magnesiumstearat | 2,50 g |
| Kolloidales Siliciumdioxid | 32,00 g |
| Aethanol | q.s. |

Ein Gemisch des Wirkstoffs, der Lactose und 194,70 g Kartoffelstärke wird mit einer äthanolischen Lösung der Stearinsäure befeuchtet und durch ein Sieb granuliert. Nach dem Trocknen mischt man die restliche Kartoffelstärke, den Talk, das Magnesiumstearat und das kolloidale Siliciumdioxid zu und presst die Mischung zu Tabletten von je 0,1 g Gewicht, die gewünschtenfalls mit Teilkerben zur feineren Anpassung der Dosierung versehen sein können.

In analoger Weise können 100 mg Wirkstoff eingearbeitet werden.

Beispiel 58: Kapseln, enthaltend 0,025 g des Wirkstoffs, z.B. 4-Hydroxy-1-[(5-methoxybenzocyclobuten-1-yl)methyl]-1,2,5,6-tetrahydro-pyridin-3-carbonsäureäthylester-hydrochlorid bzw. 1-[(5-Methoxybenzocyclobuten-1-yl)methyl]-4-oxo-piperidin-3-carbonsäureäthylester-hyrdrochlorid, können wie folgt hergestellt werden:

| Zusammensetzung (für 1000 Kapseln) | |
|---|---|
| Wirkstoff | 25,00 g |
| Lactose | 249,00 g |
| Gelatine | 2,00 g |
| Maisstärke | 10,00 g |
| Talk | 15,00 g |
| Wasser | q.s. |

Man mischt den Wirkstoff mit der Lactose, befeuchtet die Mischung gleichmässig mit einer wässrigen Lösung der Gelatine und granuliert sie durch ein Sieb mit einer Maschenweite von 1,2 bis 1,5 mm. Das Granulat mischt man mit der getrockneten Maisstärke und dem Talk und füllt Portionen von 300 mg in Hartgelatinekapseln (Grösse 1) ab.

Beispiel 59: In analoger Weise wie in den Beispielen 56 bis 58 beschrieben können auch pharmazeutische Präparate, enthaltend eine andere Verbindung der Formel I oder ein Tautomeres und/oder ein pharmazeutisch verwendbares Salz davon, beispielsweise gemäss einem der Beispiele 1 bis 55, hergestellt werden.

## Patentansprüche

### Patentansprüche für folgende Vertragsstaaten : BE, CH, DE, FR, GB, IT, LI, LU, NL, SE

**1.** Hydropyridin-Derivate der Formel

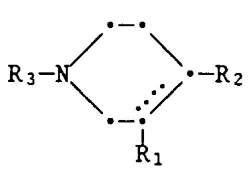

(I),

worin $R_1$ Carboxy, Niederalkoxycarbonyl, Carbamoyl, N-Niederalkylcarbamoyl oder N,N-Diniederalkylcarbamoyl bedeutet, $R_2$ Wasserstoff, Hydroxy, Niederalkoxy, gegebenenfalls durch Niederalkyl, Niederalkoxy und/oder Halogen substituiertes Phenylniederalkoxy, Niederalkanoyloxy, Benzoyloxy, Pyridoyloxy, Niederalkansulfonyloxy, Niederalkoxycarbonyloxy, Phenylniederalkoxycarbonyloxy, Amino, Niederalkanoylamino, Benzoylamino, Pyridoylamino, Niederalkansulfonylamino, Niederalkoxycarbonylamino oder Phenylniederalkoxycarbonylamino bedeutet, $R_3$ einen Rest der Formeln R- (Ia), R-$alk_1$- (Ib) oder R'=$alk_2$- (Ic) bedeutet, worin R einen unsubstituierten oder im Benzoteil ein- oder mehrfach durch Hydroxy, Niederalkoxy, Niederalkanoyloxy, Halogen, Niederalkyl und/oder Trifluormethyl und/oder in $\alpha$-Stellung zum Benzoteil durch Niederalkyl substituierten, über ein gesättigtes C-Atom gebundenen Benzocycloalkenylrest mit insgesamt 8 bis 12 Ring-C-Atomen bedeutet, R' einen unsubstituierten oder im Benzoteil ein- oder mehrfach durch Hydroxy, Niederalkoxy, Niederalkanoyloxy, Halogen, Niederalkyl und/oder Trifluormethyl substituierten Benzocycloalkenylidenrest mit insgesamt 8 bis 12 Ring-C-Atomen bedeutet, $alk_1$ Niederalkylen oder Niederalkyliden ist, $alk_2$ Niederalkyl-$\omega$-yliden bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, wobei "niedere" Gruppen jeweils 1 bis und mit 4 Kohlenstoffatome aufweisen können, und ihre Tautomeren und/oder Salze.

2. Verbindungen der Formel I gemäss Anspruch 1, worin $R_3$ eine Gruppe Ia oder Ib ist, R einen unsubstituierten oder im Benzoteil ein- oder mehrfach durch Hydroxy, Niederalkoxy, Niederalkanoyloxy, Halogen, Niederalkyl und/oder Trifluormethyl und/oder in $\alpha$-Stellung zum Benzoteil durch Niederalkyl substituierten Benzocyclobuten-1-ylrest bedeutet, $R_1$ Carboxy, Niederalkoxycarbonyl, Carbamoyl, N-Niederalkylcarbamoyl oder N,N-Diniederalkylcarbamoyl bedeutet, $R_2$ Wasserstoff, Hydroxy, Niederalkoxy, Phenylniederalkoxy, Niederalkanoyloxy, Benzoyloxy, Pyridoyloxy, Niederalkoxycarbonyloxy, Phenylniederalkoxycarbonyloxy, Amino, Niederalkanoylamino, Benzoylamino, Pyridoylamino, Niederalkoxycarbonylamino oder Phenylniederalkoxycarbonylamino bedeutet, $alk_1$ Niederalkylen oder Niederalkyliden bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, wobei "niedere" Gruppen jeweils 1 bis und mit 4 Kohlenstoffatome aufweisen können, und ihre Tautomeren und/oder Salze.

3. Verbindungen der Formel I gemäss Anspruch 1, worin $R_3$ eine Gruppe Ia, Ib oder Ic ist, R einen unsubstituierten oder im Benzoteil ein- oder mehrfach durch Hydroxy, Niederalkoxy, Niederalkanoyloxy und/oder Halogen und/oder in $\alpha$-Stellung zum Benzoteil durch Niederalkyl substituierten Benzocyclobuten-1-ylrest, Indan-1-yl- oder Indan-2-ylrest oder 1,2,3,4-Tetrahydronaphth-1-yl- oder 1,2,3,4-Tetrahydronaphth-2-ylrest bedeutet, R' einen unsubstituierten oder im Benzoteil ein- oder mehrfach durch Hydroxy, Niederalkoxy, Niederalkanoyloxy und/oder Halogen substituierten Benzocyclobuten-1-ylidenrest bedeutet, $R_1$ Carboxy, Niederalkoxycarbonyl oder Carbamoyl bedeutet, $R_2$ Wasserstoff, Hydroxy, Niederalkoxy, Niederalkanoyloxy, Niederalkansulfonyloxy, gegebenenfalls im Phenylteil durch Niederalkyl, Niederalkoxy und/oder Halogen substituiertes Phenylniederalkoxy oder Amino bedeutet, $alk_1$ Niederalkylen oder Niederalkyliden, das die beiden Ringsysteme durch 1 bis und mit 3 C-Atome bzw. durch 1 C-Atom verbindet, bedeutet, $alk_2$ Niederalkyl-$\omega$-yliden, das die beiden Ringsysteme durch 2 oder 3 C-Atome verbindet, darstellt und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, wobei "niedere" Gruppen jeweils 1 bis und mit 4 Kohlenstoffatome aufweisen können, und ihre Tautomeren und/oder Salze.

4. Verbindungen der Formel I gemäss Anspruch 1, worin $R_3$ eine Gruppe Ia oder Ib darstellt, R einen unsubstituierten oder im Benzoteil durch Niederalkoxy mit bis und mit 4 C-Atomen, Niederalkyl mit bis und mit 4 C-Atomen oder durch Halogen der Atomnummer bis und mit 35 monosubstituierten oder durch Niederalkyl mit bis und mit 4 C-Atomen oder durch Niederalkoxy mit bis und mit 4 C-Atomen sowie Niederalkyl mit bis und mit 4 C-Atomen disubstituierten Benzocyclobuten-1-ylrest, Indan-1-ylrest, Indan-2-ylrest oder 1,2,3,4-Tetrahydronaphth-1-ylrest bedeutet, $R_1$ Niederalkoxycarbonyl mit 1 bis und mit 4 C-Atomen im Niederalkoxyteil oder Carbamoyl bedeutet, $R_2$ Wasserstoff oder Hydroxy bedeutet, $alk_1$ für Niederalkylen, welches die Ringsysteme durch 1 bis und mit 3 C-Atome überbrückt, mit bis und mit 3 C-Atomen steht und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und ihre Tautomeren und/oder Salze.

5. Verbindungen der Formel I gemäss Anspruch 2, worin $R_3$ eine Gruppe der Formel Ib darstellt, R einen unsubstituierten oder im Benzoteil durch Niederalkoxy mit bis und mit 4 C-Atomen substituierten Benzocyclobuten-1-ylrestbedeutet, $R_1$ Niederalkoxycarbonyl mit 1 bis und mit 4 C-Atomen im Niederalkoxyteil oder Carbamoyl bedeutet, $R_2$ Wasserstoff oder Hydroxy bedeutet, $alk_1$ für Niederalkylen, welches die Ringsysteme durch 1 bis und mit 3 C-Atome überbrückt, mit bis und mit 3 C-Atomen steht und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, und ihre Tautomeren und/oder Salze.

6. 4-Hydroxy-1-[(5-methoxybenzocyclobuten-1-yl)methyl]-1,2,5,6-tetrahydro-pyridin-3-carbonsäureäthylester bzw. 1-[(5-Methoxybenzocyclobuten-1-yl)methyl]-4-oxo-piperidin-3-carbonsäureäthylester oder ein Salz davon gemäss Anspruch 2.

7. cis-4-Hydroxy-1-[(5-methoxybenzocyclobuten-1-yl)methyl]-piperidin-3-carbonsäureäthylester oder ein Salz davon gemäss Anspruch 2.

8. trans-4-Hydroxy-1-[2-(5-methoxybenzocyclobuten-1-yl)äthyl]-piperidin-3-carbonsäureäthylester oder ein Salz davon gemäss Anspruch 2.

9. 4-Amino-1-[(5-methoxybenzocyclobuten-1-yl)methyl]-1,2,5,6-tetrahydro-pyridin-3-carbonsäureäthylester bzw. 1-[(5-Methoxybenzocyclobuten-1-yl)methyl]-4-imino-piperidin-3-carbonsäureäthylester oder ein Salz davon gemäss Anspruch 2.

10. 4-Hydroxy-1-[2-(5-methoxybenzocyclobuten-1-yl)äthyl]-1,2,5,6-tetrahydro-pyridin-3-carbonsäuremethylester bzw. 1-[2-(5-Methoxybenzocyclobuten-1-yl)äthyl]-4-oxo-piperidin-3-carbonsäuremethylester oder ein Salz davon gemäss Anspruch 2.

11. 4-Hydroxy-1-[(benzocyclobuten-1-yl)methyl]-1,2,5,6-tetrahydro-pyridin-3-carbonsäureäthylester bzw. 1-[(Benzocyclobuten-1-yl)methyl]-4-oxo-piperidin-3-carbonsäureäthylester oder ein Salz davon gemäss Anspruch 1.

12. 1-[(6-Methoxyindan-1-yl)methyl]-1,2,5,6-tetrahydro-pyridin-3-carbonsäuremethylester oder ein Salz davon gemäss Anspruch 1.

13. 4-Hydroxy-1-[(5-methoxy-1-methyl-benzocyclobuten-1-yl)methyl]-1,2,5,6-tetrahydro-pyridin-3-carbonsäureäthylester bzw. 1-[(5-Methoxy-1-methyl-benzocyclobuten-1-yl)methyl]-4-oxo-piperidin-3-carbonsäureäthylester oder ein Salz davon gemäss Anspruch 1.

14. 4-Hydroxy-1-(benzocyclobuten-1-yl)-1,2,5,6-tetrahydro-pyridin-3-carbonsäureäthylester bzw. 1-(Benzocyclobuten-1-yl)-4-oxo-piperidin-3-carbonsäureäthylester oder ein Salz davon gemäss Anspruch 1.

15. 1-[(7-Methoxy-1,2,3,4-tetrahydro-naphth-2-yl)methyl]-1,2,5,6-tetrahydro-pyridin-3-carbonsäuremethylester oder ein Salz davon gemäss Anspruch 1.

16. 4-Hydroxy-1-[(4-methoxybenzocyclobuten-1-yl)methyl]-1,2,5,6-tetrahydro-pyridin-3-carbonsäureäthylester bzw. 1-[4-Methoxybenzocyclobuten-1-yl)methyl]-4-oxo-piperidin-3-carbonsäureäthylester oder ein Salz davon gemäss Anspruch 1.

17. 4-Hydroxy-1-[(7-methoxy-1,2,3,4-tetrahydro-naphth-2-yl)methyl]-1,2,5,6-tetrahydro-pyridin-3-carbonsäuremethylester bzw. 1-[(7-Methoxy-1,2,3,4-tetrahydro-naphth-2-yl)methyl]-4-oxo-piperidin-3-carbonsäuremethylester oder ein Salz davon gemäss Anspruch 1.

18. 4-Hydroxy-1-(6-methoxyindan-1-yl)-1,2,5,6-tetrahydro-pyridin-3-carbonsäureäthylester bzw. 1-(6-Methoxyindan-1-yl)-4-oxo-piperidin-3-carbonsäureäthylester oder ein Salzdavon gemäss Anspruch 1.

19. 4-Hydroxy-1-(7-methoxy-1,2,3,4-tetrahydro-naphth-2-yl)-1,2,5,6-tetrahydro-pyridin-3-carbonsäuremethylester bzw. 1-(7-Methoxy-1,2,3,4-tetrahydro-naphth-2-yl)-4-oxo-piperidin-3-carbonsäuremethylester oder ein Salz davon gemäss Anspruch 1.

20. Eine Verbindung gemäss einem der Ansprüche 1 bis 19 oder ein Tautomeres und/oder ein pharmazeutisch verwendbares Salz davon zur Anwendung in einem Verfahren zur therapeutischen Behandlung des menschlichen oder tierischen Körpers.

21. Eine Verbindung gemäss einem der Ansprüche 2 und 5 bis 10 oder ein Tautomeres und/oder ein pharmazeutisch verwendbares Salz davon zur Anwendung in einem Verfahren zur therapeutischen Behandlung des menschlichen oder tierischen Körpers.

22. Eine Verbindung gemäss einem der Ansprüche 1 bis 21 oder ein Tautomeres und/oder ein pharmazeutisch verwendbares Salz davon zur Anwendung als Nootropikum.

23. Eine Verbindung gemäss einem der Ansprüche 2,5 bis 10 und 21 oder ein Tautomeres und/oder ein pharmazeutisch verwendbares Salz davon zur Anwendung als Nootropikum.

**24.** Ein pharmazeutisches Präparat, als Wirkstoff enthaltend eine Verbindung gemäss einem der Ansprüche 1 bis 23 oder ein Tautomeres und/oder ein pharmazeutisch verwendbares Salz davon, gegebenenfalls neben üblichen pharmazeutischen Hilfsstoffen.

**25.** Ein pharmazeutisches Präparat, als Wirkstoff enthaltend eine Verbindung gemäss einem der Ansprüche 2,5 bis 10,21 und 23 oder ein Tautomeres und/oder ein pharmazeutisch verwendbares Salz davon, gegebenenfalls neben üblichen pharmazeutischen Hilfsstoffen.

**26.** Ein nootrop wirksames pharmazeutisches Präparat gemäss Anspruch 24 oder 25, dadurch gekennzeichnet, dass man einen nootrop wirksamen Wirkstoff wählt.

**27.** Ein nootrop wirksames pharmazeutisches Präparat gemäss Anspruch 25, dadurch gekennzeichnet, dass man einen nootrop wirksamen Wirkstoff wählt.

**28.** Verfahren zur Herstellung einer Verbindung der Formel I gemäss einem der Ansprüche 1 bis 19 oder eines Tautomeren und/oder Salzes davon, dadurch gekennzeichnet, dass man
a) eine Verbindung der Formel

$R_3$-$X_1$ (IIa)

oder ein Salz davon, worin $X_1$ Hydroxy oder reaktionsfähiges verestertes Hydroxy bedeutet, mit einer Verbindung der Formel

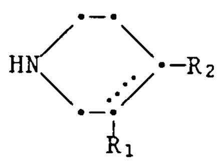

(IIb)

oder einem Tautomeren und/oder Salz davon umsetzt oder
b) in einer Verbindung der Formel

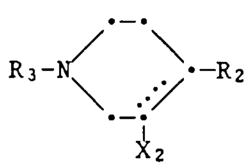

(III)

oder einem Tautomeren und/oder Salz davon, worin $X_2$ Cyano, Halogencarbonyl, gegebenenfalls durch $C_1$-$C_4$-Alkyl substituiertes Amidino, $C_1$-$C_4$-Alkanoyloxycarbonyl, $C_1$-$C_4$-Alkoxycarbonyloxycarbonyl, Carboximidoyl, $C_1$-$C_4$-Alkoxycarbimidoyl, Halogencarbimidoyl, Tri-$C_1$-$C_4$-alkoxymethyl oder Trihalogenmethyl bedeutet, $X_2$ durch Solvolysein $R_1$ überführt oder
c) zur Herstellung von Verbindungen der Formel I, worin $R_2$ Hydroxy oder Amino bedeutet und worin $R_1$ insbesondere $C_1$-$C_4$-Alkoxycarbonyl bedeutet, oder ihrer Tautomeren und/oder Salze eine Verbindung der Formel

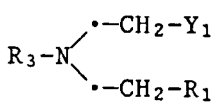

(IV),

worin $Y_1$ eine Gruppe der Formel -CH=$R_2'$ ,-C($Y_2$)=$R_2$'oder Cyano und $R_2'$ Oxo oder Imino darstellt, wobei $Y_2$ $C_1$-$C_4$-Alkoxy darstellt, oder ein Salz davon cyclisiert oder

d) zur Herstellung von Verbindungen der Formel I, worin $R_2$ Hydroxy oder Amino bedeutet und die gestrichelte Linie zum Ausdruck bringen soll, dass zwischen den Kohlenstoffatomen, welche die Reste $R_1$ und $R_2$ tragen, eine Doppelbindung vorliegt, und worin $R_1$ insbesondere $C_1$-$C_4$-Alkoxycarbonyl bedeutet, oder ihrer Tautomeren und/oder Salze eine Verbindung der Formel

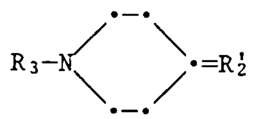

(Va),

worin $R_2'$ Oxo oder Imino ist, oder ein Tautomeres oder Salz davon mit einer Verbindung der Formel

$X_3$-$R_1$ (Vb),

worin $X_3$ Halogen oder $C_1$-$C_4$-Alkoxy bedeutet, oder einem Salz davon umsetzt oder

e) zur Herstellung von Verbindungen der Formel I, worin $R_2$ von Wasserstoff verschieden ist, oder ihrer Tautomeren und/oder Salze in einer Verbindung der Formel

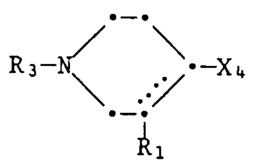

(VI)

oder einem Salz davon, worin $X_4$ Halogen oder eine Diazoniumgruppe der Formel $-N_2^{\oplus}A^{\ominus}$, worin $A^{\ominus}$ das Anion einer Mineralsäure ist, bedeutet, $X_4$ durch Solvolyse in $R_2$ überführt oder

f) insbesondere zur Herstellung von Verbindungen der Formel I, worin $R_2$ Wasserstoff bedeutet, oder ihrer Tautomeren und/oder Salze in einer Verbindung der Formel

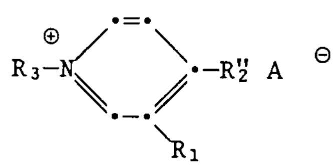

(VII),

worin $A^{\ominus}$ für das Anion einer Säure steht, und $R_2''$ Wasserstoff bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, ein verfahrensgemäss erhältliches Isomerengemisch in die Komponenten auftrennt, ein verfahrensgemäss erhältliches Enantiomeren- bzw. Diastereomerengemisch in die Enantiomeren bzw. Diastereomeren aufspaltet und/oder eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

**29.** Verfahren zur Herstellung eines pharmazeutischen Präparates gemäss Anspruch 24, dadurch gekennzeichnet, dass man den Wirkstoff, gegebenenfalls unter Beimischung von üblichen pharmazeutischen Hilfsstoffen, zu einem pharmazeutischen Präparat verarbeitet.

**30.** Verfahren gemäss Anspruch 29 zur Herstellung eines nootrop wirksamen pharmazeutischen Präparates gemäss Anspruch 26, dadurch gekennzeichnet, dass man einen nootrop wirksamen Wirkstoff wählt.

**31.** Verwendung einer Verbindung gemäss einem der Ansprüche 1 bis 23 oder eines Tautomeren und/oder eines pharmazeutisch verwendbaren Salzes davon zur Herstellung eines pharmazeutischen Präparates.

**32.** Verwendung gemäss Anspruch 31 zur Herstellung eines Nootropikums.

**33.** Verwendung einer Verbindung gemäss einem der Ansprüche 1 bis 23 oder eines Tautomeren und/oder eines pharmazeutisch verwendbaren Salzes davon zur Herstellung eines pharmazeutischen Präparates auf nicht-chemischem Wege.

**Patentansprüche für folgenden Vertragsstaat : AT**

**1.** Verfahren zur Herstellung neuer Hydropyridin-Derivate der Formel

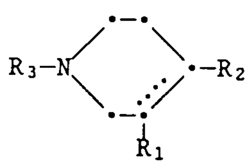

(I),

worin $R_1$ Carboxy, Niederalkoxycarbonyl, Carbamoyl, N-Niederalkylcarbamoyl oder N,N-Diniederalkylcarbamoyl bedeutet, $R_2$ Wasserstoff, Hydroxy, Niederalkoxy, gegebenenfalls durch Niederalkyl, Niederalkoxy und/oder Halogen substituiertes Phenylniederalkoxy, Niederalkanoyloxy, Benzoyloxy, Pyridoyloxy, Niederalkansulfonyloxy, Niederalkoxycarbonyloxy, Phenylniederalkoxycarbonyloxy, Amino, Niederalkanoylamino, Benzoylamino, Pyridoylamino, Niederalkansulfonylamino, Niederalkoxycarbonylamino oder Phenylniederalkoxycarbonylamino bedeutet, $R_3$ einen Rest der Formeln R- (Ia), R-alk$_1$- (Ib) oder R'=alk$_2$- (Ic) bedeutet, worin R einen unsubstituierten oder im Benzoteil ein- oder mehrfach durch Hydroxy, Niederalkoxy, Niederalkanoyloxy, Halogen, Niederalkyl und/oder Trifluormethyl und/oder in $\alpha$-Stellung zum Benzoteil durch Niederalkyl substituierten, über ein gesättigtes C-Atom gebundenen Benzocycloalkenylrest mit insgesamt 8 bis 12 Ring-C-Atomen bedeutet, R' einen unsubstituierten oder im Benzoteil ein- oder mehrfach durch Hydroxy, Niederalkoxy, Niederalkanoyloxy, Halogen, Niederalkyl und/oder Trifluormethyl substituierten Benzocycloalkenylidenrest mit insgesamt 8 bis 12 Ring-C-Atomen bedeutet, alk$_1$ Niederalkylen oder Niederalkyliden ist, alk$_2$ Niederalkyl-$\omega$-yliden bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, wobei "niedere" Gruppen jeweils 1 bis und mit 4 Kohlenstoffatome aufweisen können, oder ihrer Tautomeren und/oder Salze, dadurch gekennzeichnet, dass man

a) eine Verbindung der Formel

$R_3$-$X_1$ (IIa)

oder ein Salz davon, worin $X_1$ Hydroxy oder reaktionsfähiges verestertes Hydroxy bedeutet, mit einer Verbindung der Formel

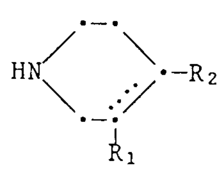

(IIb)

oder einem Tautomeren und/oder Salz davon umsetzt oder

b) in einer Verbindung der Formel

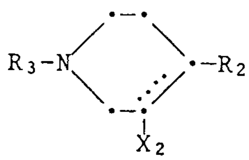

(III)

oder einem Tautomeren und/oder Salz davon, worin $X_2$ Cyano, Halogencarbonyl, gegebenenfalls durch $C_1$-$C_4$-Alkyl substituiertes Amidino, $C_1$-$C_4$-Alkanoyloxycarbonyl, $C_1$-$C_4$-Alkoxycarbonyloxycarbonyl, Carboximidoyl, $C_1$-$C_4$-Alkoxycarbimidoyl, Halogencarbimidoyl, Tri-$C_1$-$C_4$-alkoxymethyl oder Trihalogenmethyl bedeutet, $X_2$ durch Solvolyse in $R_1$ überführt oder

c) zur Herstellung von Verbindungen der Formel I, worin $R_2$ Hydroxy oder Amino bedeutet und worin $R_1$ insbesondere $C_1$-$C_4$-Alkoxycarbonyl bedeutet, oder ihrer Tautomeren und/oder Salze eine Verbindung der Formel

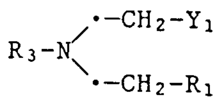

(IV),

worin $Y_1$ eine Gruppe der Formel $-CH=R_2'$ ,$-C(Y_2)=R_2'$oder Cyano und $R_2''$ Oxo oder Imino darstellt, wobei $Y_2$ $C_1$-$C_4$-Alkoxy darstellt, oder ein Salz davon cyclisiert oder

d) zur Herstellung von Verbindungen der Formel I, worin $R_2$ Hydroxy oder Amino bedeutet und die gestrichelte Linie zum Ausdruck bringen soll, dass zwischen den Kohlenstoffatomen, welche die Reste $R_1$ und $R_2$ tragen, eine Doppelbindung vorliegt, und worin $R_1$ insbesondere $C_1$-$C_4$-Alkoxycarbonyl bedeutet, oder ihrer Tautomeren und/oder Salze eine Verbindung der Formel

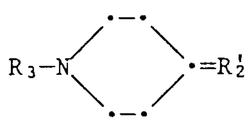

(Va),

worin $R_2'$ Oxo oder Imino ist, oder ein Tautomeres oder Salz davon mit einer Verbindung der Formel

$X_3$-$R_1$ (Vb),

worin $X_3$ Halogen oder $C_1$-$C_4$-Alkoxy bedeutet, oder einem Salz davon umsetzt oder

e) zur Herstellung von Verbindungen der Formel I, worin $R_2$ von Wasserstoff verschieden ist, oder ihrer Tautomeren und/oder Salze in einer Verbindung der Formel

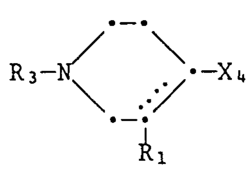

(VI)

oder einem Salz davon, worin $X_4$ Halogen oder eine Diazoniumgruppe der Formel $-N_2^{\oplus}A^{\ominus}$, worin $A^{\ominus}$ das Anion einer Mineralsäure ist, bedeutet, $X_4$ durch Solvolyse in $R_2$ überführt oder

f) insbesondere zur Herstellung von Verbindungen der Formel I, worin $R_2$ Wasserstoff bedeutet, oder ihrer Tautomeren und/oder Salze in einer Verbindung der Formel

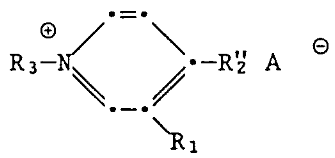

(VII),

worin $A^{\ominus}$ für das Anion einer Säure steht, und $R_2''$ Wasserstoff bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, ein verfahrensgemäss erhältliches Isomerengemisch in die Komponenten auftrennt, ein verfahrensgemäss erhältliches Enantiomeren- bzw. Diastereomerengemisch in die Enantiomeren bzw. Diastereomeren aufspaltet und/oder eine verfahrensgemäss erhältliche freie Verbindung der Formel I in ein Salz überführt oder ein verfahrensgemäss erhältliches Salz in die freie Verbindung der Formel I oder in ein anderes Salz umwandelt.

**2.** Verfahren gemäss Anspruch 1 zur Herstellung von Verbindungen der Formel I, worin $R_3$ eine Gruppe Ia oder Ib ist, Reinen unsubstituierten oder im Benzoteil ein- oder mehrfach durch Hydroxy, Niederalkoxy, Niederalkanoyloxy, Halogen, Niederalkyl und/oder Trifluormethyl und/oder in $\alpha$-Stellung zum Benzoteil durch Niederalkyl substituierten Benzocyclobuten-1-ylrest bedeutet, $R_1$ Carboxy, Niederalkoxycarbonyl, Carbamoyl, N-Niederalkylcarbamoyl oder N,N-Diniederalkylcarbamoyl bedeutet, $R_2$ Wasserstoff, Hydroxy, Niederalkoxy, Phenylniederalkoxy, Niederalkanoyloxy, Benzoyloxy, Pyridoyloxy, Niederalkoxycarbonyloxy, Phenylniederalkoxycarbonyloxy, Amino, Niederalkanoylamino, Benzoylamino, Pyridoylamino, Niederalkoxycarbonylamino oder Phenylniederalkoxycarbonylamino bedeutet, $alk_1$ Niederalkylen oder Niederalkyliden bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, wobei "niedere" Gruppen jeweils 1 bis und mit 4 Kohlenstoffatome aufweisen können, oder ihrer Tautomeren und/oder Salze.

**3.** Verfahren gemäss Anspruch 1 zur Herstellung von Verbindungen der Formel I, worin $R_3$ eine Gruppe Ia, Ib oder Ic ist, R einen unsubstituierten oder im Benzoteil ein- oder mehrfach durch Hydroxy, Niederalkoxy, Niederalkanoyloxy und/oder Halogen und/oder in $\alpha$-Stellung zum Benzoteil durch Niederalkyl substituierten Benzocyclobuten-1-ylrest, Indan-1-yl- oder Indan-2-ylrest oder 1,2,3,4-Tetrahydronaphth-1-yl- oder 1,2,3,4-Tetrahydronaphth-2-ylrest bedeutet, R' einen unsubstituierten oder im Benzoteil ein- oder mehrfach durch Hydroxy, Niederalkoxy, Niederalkanoyloxy und/oder Halogen substituierten Benzocyclobuten-1-ylidenrest bedeutet, $R_1$ Carboxy, Niederalkoxycarbonyl oder Carbamoyl bedeutet, $R_2$ Wasserstoff, Hydroxy, Niederalkoxy, Niederalkanoyloxy, Niederalkansulfonyloxy, gegebenenfalls im Phenylteil durch Niederalkyl, Niederalkoxy und/oder Halogen substituiertes Phenylniederalkoxy oder Amino bedeutet, $alk_1$ Niederalkylen oder Niederalkyliden, das die beiden Ringsysteme durch 1 bis und mit 3 C-Atome bzw. durch 1 C-Atom verbindet, bedeutet, $alk_2$ Niederalkyl-$\omega$-yliden, das die beiden Ringsysteme durch 2 oder 3 C-Atome verbindet, darstellt und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, wobei "niedere" Gruppen jeweils 1 bis und mit 4 Kohlenstoffatome aufweisen können, oder ihrer Tautomeren und/oder Salze.

**4.** Verfahren gemäss Anspruch 1 zur Herstellung von Verbindungen der Formel I, worin $R_3$ eine Gruppe Ia oder Ib darstellt, R einen unsubstituierten oder im Benzoteil durch Niederalkoxy mit bis und mit 4 C-Atomen, Niederalkyl mit bis und mit 4 C-Atomen oder durch Halogen der Atomnummer bis und mit 35 monosubstituierten oder durch Niederalkyl mit bis und mit 4 C-Atomen oder durch Niederalkoxy mit bis und mit 4 C-Atomen sowie Niederalkyl mit bis und mit 4 C-Atomen disubstituierten Benzocyclobuten-1-ylrest, Indan-1-ylrest, Indan-2-ylrest oder 1,2,3,4-Tetrahydronaphth-1-ylrest bedeutet, $R_1$ Niederalkoxycarbonyl mit 1 bis und mit 4 C-Atomen im Niederalkoxyteil oder Carbamoyl bedeutet, $R_2$ Wasserstoff oder Hydroxy bedeutet, $alk_1$ für Niederalkylen, welches die Ringsysteme durch 1 bis und mit 3 C-Atome überbrückt, mit bis und mit 3 C-Atomen steht und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, oder ihrer Tautomeren und/oder Salze.

**5.** Verfahren gemäss Anspruch 1 zur Herstellung von Verbindungen der Formel I, worin $R_3$ eine Gruppe der Formel Ib darstellt, R einen unsubstituierten oder im Benzoteil durch Niederalkoxy mit bis und mit 4 C-Atomen substituierten Benzocyclobuten-1-ylrest bedeutet, $R_1$ Niederalkoxycarbonyl mit 1 bis und mit 4 C-Atomen im Niederalkoxyteil oder Carbamoyl bedeutet, $R_2$ Wasserstoff oder Hydroxy bedeutet, $alk_1$ für Niederalkylen, welches die Ringsysteme durch 1 bis und mit 3 C-Atome überbrückt, mit bis und mit 3 C-Atomen steht und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, oder ihrer Tautomeren und/oder Salze.

**6.** Verfahren gemäss Anspruch 1 zur Herstellung von 4-Hydroxy-1-[(5-methoxybenzocyclobuten-1-yl)-methyl]-1,2,5,6-tetrahydro-pyridin-3-carbonsäureäthylester bzw. 1-[(5-Methoxybenzocyclobuten-1-yl)-methyl]-4-oxo-piperidin-3-carbonsäureäthylester oder eines Salzes davon.

**7.** Verfahren gemäss Anspruch 2 zur Herstellung von cis-4-Hydroxy-1-[(5-methoxybenzocyclobuten-1-yl)-methyl]-piperidin-3-carbonsäureäthylester oder eines Salzes davon.

**8.** Verfahren gemäss Anspruch 2 zur Herstellung von trans-4-Hydroxy-1-[2-(5-methoxybenzocyclobuten-1-yl)äthyl]-piperidin-3-carbonsäureäthylester oder eines Salzes davon.

**9.** Verfahren gemäss Anspruch 2 zur Herstellung von 4-Amino-1-[(5-methoxybenzocyclobuten-1-yl)-methyl]-1,2,5,6-tetrahydro-pyridin-3-carbonsäureäthylester bzw. 1-[(5-Methoxybenzocyclobuten-1-yl)-methyl]-4-imino-piperidin-3-carbonsäureäthylester odereines Salzes davon.

**10.** Verfahren gemäss Anspruch 2 zur Herstellung von 4-Hydroxy-1-[2-(5-methoxybenzocyclobuten-1-yl)-äthyl]-1,2,5,6-tetrahydro-pyndin-3-carbonsäuremethylester bzw. 1-[2-(5-Methoxybenzocyclobuten-1-yl)-äthyl]-4-oxo-piperidin-3-carbonsäuremethylester oder eines Salzes davon.

**11.** Verfahren gemäss Anspruch 1 zur Herstellung von 4-Hydroxy-1-[(benzocyclobuten-1-yl)methyl]-1,2,5,6-tetrahydro-pyridin-3-carbonsäureäthylester bzw. 1-[(Benzocyclobuten-1-yl)methyl]-4-oxo-piperidin-3-carbonsäureäthylester oder eines Salzes davon.

**12.** Verfahren gemäss Anspruch 1 zur Herstellung von 1[(6-Methoxyindan-1-yl)methyl]-1,2,5,6-tetrahydro-pyridin-3-carbonsäuremethylester oder eines Salzes davon.

**13.** Verfahren gemäss Anspruch 1 zur Herstellung von 4-Hydroxy-1-[(5-methoxy-1-methyl-benzocyclobuten-1-yl)methyl]-1,2,5,6-tetrahydro-pyridin-3-carbonsäureäthylester bzw. 1-[(5-Methoxy-1-methyl-benzocyclobuten-1-yl)methyl]-4-oxo-piperidin-3-carbonsäureäthylester oder eines Salzes davon.

**14.** Verfahren gemäss Anspruch 1 zur Herstellung von 4-Hydroxy-1-(benzocyclobuten-1-yl)-1,2,5,6-tetrahydro-pyridin-3-carbonsäureäthylester bzw. 1-(Benzocyclobuten-1-yl)-4-oxo-piperidin-3-carbonsäureäthylester oder eines Salzes davon.

**15.** Verfahren gemäss Anspruch 1 zur Herstellung von 1-[(7-Methoxy-1,2,3, 4-tetrahydro-naphth-2-yl)-methyl]-1,2,5,6-tetrahydro-pyridin-3-carbonsäuremethylester oder eines Salzes davon.

**16.** Verfahren gemäss Anspruch 1 zur Herstellung von 4-Hydroxy-1-[(4-methoxybenzocyclobuten-1-yl)-methyl]-1,2,5,6-tetrahydro-pyridin-3-carbonsäureäthylester bzw. 1-[(4-Methoxybenzocyclobuten-1-yl)-methyl]-4-oxo-piperidin-3-carbonsäureäthylester oder eines Salzes davon.

**17.** Verfahren gemäss Anspruch 1 zur Herstellung von 4-Hydroxy-1-[(7-methoxy-1,2,3,4-tetrahydro-naphth-2-yl)methyl]-1,2,5,6-tetrahydro-pyridin-3-carbonsäuremethylester bzw. 1-[(7-Methoxy-1,2,3,4-teträhydro-naphth-2-yl)methyl]-4-oxo-piperidin-3-carbonsäureme thylester oder eines Salzes davon.

**18.** Verfahren gemäss Anspruch 1 zur Herstellung von 4-Hydroxy-1-(6-methoxyindan-1-yl)-1,2,5,6-tetrahydro-pyridin-3-carbonsäureäthylester bzw. 1-(6-Methoxyindan-1-yl)-4-oxo-piperidin-3-carbonsäureäthylester oder eines Salzes davon.

**19.** Verfahren gemäss Anspruch 1 zur Herstellung von 4-Hydroxy-1-(7-methoxy-1,2,3,4-tetrahydro-naphth-2-yl)-1,2,5,6-tetrahydro-pyridin-3-carbonsäuremethylester bzw. 1-(7-Methoxv-1,2,3,4-tetrahydro-naphth-2-yl)-4-oxo-piperidin-3-carbonsäuremethylester oder eines Salzes davon.

**20.** Verfahren zur Herstellung eines pharmazeutischen Präparats, als Wirkstoff enthaltend eine Verbindung, erhältlich gemäss einem der Ansprüche 1 bis 19, oder ein Tautomeres und/oder ein pharmazeutisch verwendbares Salz davon, dadurch gekennzeichnet, dass man den Wirkstoff mit üblichen pharmazeutischen Hilfsstoffen vermischt.

**21.** Verfahren zur Herstellung eines pharmazeutischen Präparats, als Wirkstoff enthaltend eine Verbindung, erhältlich gemäss einem der Ansprüche 2 und 5 bis 10, oder ein Tautomeres und/oder ein pharmazeutisch verwendbares Salz davon, dadurch gekennzeichnet, dass man den Wirkstoff mit üblichen pharmazeutischen Hilfsstoffen vermischt.

**22.** Verfahren zur Herstellung eines nootrop wirksamen pharmazeutischen Präparats gemäss Anspruch 20 oder 21, dadurch gekennzeichnet, dass man einen nootrop wirksamen Wirkstoff wählt.

**23.** Verfahren zur Herstellung eines nootrop wirksamen pharmazeutischen Präparats gemäss Anspruch 21, dadurch gekennzeichnet, dass man einen nootrop wirksamen Wirkstoff wählt.

**24.** Verfahren zur Herstellung eines pharmazeutischen Präparats, dadurch gekennzeichnet, dass man
a) eine Verbindung der Formel

$R_3$-$X_1$ (IIa)

oder ein Salz davon, worin $X_1$ Hydroxy oder reaktionsfähiges verestertes Hydroxy bedeutet, mit einer Verbindung der Formel

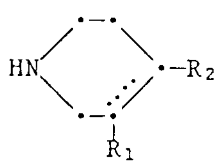

(IIb)

oder einem Tautomeren und/oder Salz davon umsetzt oder
b) in einer Verbindung der Formel

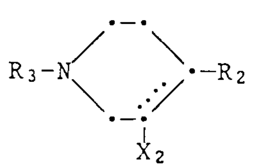

(III)

oder einem Tautomeren und/oder Salz davon, worin $X_2$ Cyano, Halogencarbonyl, gegebenenfalls durch $C_1$-$C_4$-Alkyl substituiertes Amidino, $C_1$-$C_4$-Alkanoyloxycarbonyl, $C_1$-$C_4$-Alkoxycarbonyloxycarbonyl, Carboximidoyl, $C_1$-$C_4$-Alkoxycarbimidoyl, Halogencarbimidoyl, Tri-$C_1$-$C_4$-alkoxymethyl oder Trihalogenmethyl bedeutet, $X_2$ durch Solvolyse in $R_1$ überführt oder
c) zur Herstellung von Verbindungen der Formel I, worin $R_2$ Hydroxy oder Amino bedeutet und worin $R_1$ insbesondere $C_1$-$C_4$-Alkoxycarbonyl bedeutet, oder ihrer Tautomeren und/oder Salze eine Verbindung der Formel

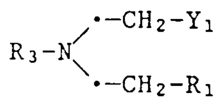

(IV),

worin $Y_1$ eine Gruppe der Formel $-CH=R_2'$, $-C(Y_2)=R_2'$ oder Cyano und $R_2'$ Oxo oder Imino darstellt, wobei $Y_2$ $C_1$-$C_4$-Alkoxy darstellt, oder ein Salz davon cyclisiert oder
d) zur Herstellung von Verbindungen der Formel I, worin $R_2$ Hydroxy oder Amino bedeutet und die gestrichelte Linie zum Ausdruck bringen soll, dass zwischen den Kohlenstoffatomen, welche die Reste $R_1$ und $R_2$ tragen, eine Doppelbindung vorliegt, und worin $R_1$ insbesondere $C_1$-$C_4$-Alkoxycarbonyl bedeutet, oder ihrer Tautomeren und/oder Salze eine Verbindung der Formel

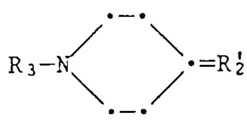

(Va),

worin $R_2'$ Oxo oder Imino ist, oder ein Tautomeres oder Salz davon mit einer Verbindung der Formel

$X_3$-$R_1$ (Vb),

worin $X_3$ Halogen oder $C_1$-$C_4$-Alkoxy bedeutet, oder einem Salz davon umsetzt oder
e) zur Herstellung von Verbindungen der Formel I, worin $R_2$ von Wasserstoff verschieden ist, oder ihrer Tautomeren und/oder Salze in einer Verbindung der Formel

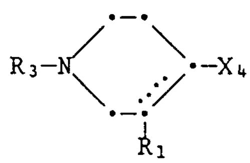

(VI)

oder einem Salz davon, worin $X_4$ Halogen oder eine Diazoniumgruppe der Formel $-N_2^{\oplus}A^{\ominus}$, worin $A^{\ominus}$ das Anion einer Mineralsäure ist, bedeutet, $X_4$ durch Solvolyse in $R_2$ überführt oder
f) insbesondere zur Herstellung von Verbindungen der Formel I, worin $R_2$ Wasserstoff bedeutet, oder ihrer Tautomeren und/oder Salze in einer Verbindung der Formel

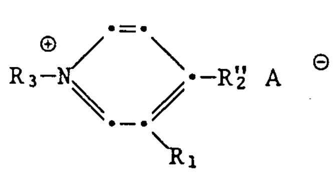

(VII),

worin $A^{\ominus}$ für das Anion einer Säure steht, und $R_2''$ Wasserstoff bedeutet, die überschüssigen Doppelbindungen zu Einfachbindungen reduziert und jeweils eine gegebenenfalls vorhandene Schutzgruppe abspaltet und jeweils gewünschtenfalls eine verfahrensgemäss oder auf andere Weise erhältliche Verbindung der Formel I in eine andere Verbindung der Formel I überführt, ein verfahrensgemäss erhältliches Isomerengemisch in die Komponenten auftrennt, ein verfahrensgemäss erhältliches Enantiomeren- bzw. Diastereomerengemisch in die Enantiomeren bzw. Diastereomeren aufspaltet und/oder eine verfahrensgemäss erhältliche freie Verbindung der Formel I in ein pharmazeutisch verwendbares Salz überführt oder ein verfahrensgemäss erhältliches pharmazeutisch verwendbares Salz in die freie Verbindung der Formel I oder in ein anderes pharmazeutisch verwendbares Salz umwandelt und ein auf diese Weise erhältliches Hydropyridin-Derivat der Formel

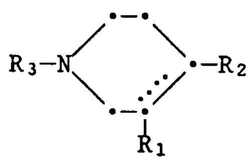

(I),

worin $R_1$ Carboxy, Niederalkoxycarbonyl, Carbamoyl, N-Niederalkylcarbamoyl oder N,N-Diniederalkylcarbamoyl bedeutet, $R_2$ Wasserstoff, Hydroxy, Niederalkoxy, gegebenenfalls durch Niederalkyl, Niederalkoxy und/oder Halogen substituiertes Phenylniederalkoxy, Niederalkanoyloxy, Benzoyloxy, Pyridoyloxy, Niederalkansulfonyloxy, Niederalkoxycarbonyloxy, Phenylniederalkoxycarbonyloxy, Amino, Niederalkanoylamino, Benzoylamino, Pyridoylamino, Niederalkansulfonylamino, Niederalkoxycarbonylamino oder Phenylniederalkoxycarbonylamino bedeutet, $R_3$ einen Rest der Formeln R- (Ia), R-alk$_1$- (Ib) oder R'=alk$_2$- (Ic) bedeutet, worin R einen unsubstituierten oder im Benzoteil ein- oder mehrfach durch Hydroxy, Niederalkoxy, Niederalkanoyloxy, Halogen, Niederalkyl und/oder Trifluormethyl und/oder in $\alpha$-Stellung zum Benzoteil durch Niederalkyl substituierten, über ein gesättigtes C-Atom gebundenen Benzocycloalkenylrest mit insgesamt 8 bis 12 Ring-C-Atomen bedeutet, R' einen unsubstituierten oder im Benzoteil ein- oder mehrfach durch Hydroxy, Niederalkoxy, Niederalkanoyloxy, Halogen, Niederalkyl und/oder Trifluormethyl substituierten Benzocycloalkenylidenrest mit insgesamt 8 bis 12 Ring-C-Atomen bedeutet, alk$_1$ Niederalkylen oder Niederalkyliden ist, alk$_2$ Niederalkyl-$\omega$-yliden bedeutet und die gestrichelte Linie zum Ausdruck bringen soll, dass zwischen den Kohlenstoffatomen, welche die Reste $R_1$ und $R_2$ tragen, eine Einfach- oder eine Doppelbindung vorliegt, wobei "niedere" Gruppen jeweils 1 bis und mit 4 Kohlenstoffatome aufweisen können, oder ein auf diese Weise erhältliches Tautomeres und/oder pharmazeutisch verwendbares Salz davon unter Beimischung von üblichen pharmazeutischen Hilfsstoffen zu einem pharmazeutischen Präparat verarbeitet.

**25.** Verfahren gemäss Anspruch 24 zur Herstellung eines nootrop wirksamen pharmazeutischen Präparates, dadurch gekennzeichnet, dass man einen nootrop wirksamen Wirkstoff wählt.

**26.** Verwendung einer Verbindung, erhältlich gemäss einem der Ansprüche 1 bis 19, oder eines Tautomeren und/oder eines pharmazeutisch verwendbaren Salzes davon zur Herstellung eines pharmazeutischen Präparates.

**27.** Verwendung gemäss Anspruch 26 zur Herstellung eines Nootropikums.

**28.** Verwendung einer Verbindung, erhältlich gemäss einem der Ansprüche 1 bis 19, oder eines Tautomeren und/oder eines pharmazeutisch verwendbaren Salzes davon zur Herstellung eines pharmazeutischen Präparates auf nicht-chemischem Wege.

## Claims

### Claims for the following Contracting States : BE, CH, DE, FR, GB, IT, LI, LU, NL, SE

**1.** A hydropyridine derivative of formula

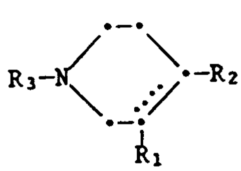

(I)

wherein $R_1$ is carboxy, lower alkoxycarbonyl, carbamoyl, N-lower alkylcarbamoyl or N,N-di-lower alkylcarbamoyl, $R_2$ is hydrogen, hydroxy, lower alkoxy, unsubstituted or lower alkyl-, lower alkoxy- and/or halo-substituted phenyl-lower alkoxy, lower alkanoyloxy, benzoyloxy, pyridoyloxy, lower alkanesulfonyloxy, lower alkoxycarbonyloxy, phenyl-lower alkoxycarbonyloxy, amino, lower alkanoylamino, benzoylamino, pyridoylamino, lower alkanesulfonylamino, lower alkoxycarbonylamino or

phenyl-lower alkoxycarbonylamino, $R_3$ is a radical of the formula R- (Ia), R-$alk_1$- (Ib) or R'=$alk_2$- (Ic) wherein R is a benzocycloalkenyl radical having a total of from 8 to 12 ring carbon atoms which is bonded via a saturated carbon atom and which is unsubstituted or is mono- or poly-substituted in the benzo moiety by hydroxy, lower alkoxy, lower alkanoyloxy, halogen, lower alkyl and/or by trifluoromethyl and/or substituted in the $\alpha$-position with respect to the benzo moiety by lower alkyl, R' is a benzocycloalkenylidene radical having a total of from 8 to 12 ring carbon atoms which is unsubstituted or is mono- or poly-substituted in the benzo moiety by hydroxy, lower alkoxy, lower alkanoyloxy, halogen, lower alkyl and/or by trifluoromethyl, $alk_1$ is lower alkylene or lower alkylidene, $alk_2$ is lower alkyl-$\omega$-ylidene and the dotted line is intended to show that there is a single bond or a double bond between the carbon atoms carrying the radicals $R_1$ and $R_2$, wherein "lower" groups may each contain from 1 up to and including 4 carbon atoms, or a tautomer and/or salt thereof.

**2.** A compound of formula I according to claim 1, wherein $R_3$ is a group Ia or Ib, R is a benzocyclobuten-1-yl radical which is unsubstituted or is mono- or poly-substituted in the benzo moiety by hydroxy, lower alkoxy, lower alkanoyloxy, halogen, lower alkyl and/or by trifluoromethyl and/or substituted in the $\alpha$-position with respect to the benzo moiety by lower alkyl, $R_1$ is carboxy, lower alkoxycarbonyl, carbamoyl, N-lower alkylcarbamoyl or N,N-di-lower alkylcarbamoyl, $R_2$ is hydrogen, hydroxy, lower alkoxy, phenyl-lower alkoxy, lower alkanoyloxy, benzoyloxy, pyridoyloxy, lower alkoxycarbonyloxy, phenyl-lower alkoxycarbonyloxy, amino, lower alkanoylamino, benzoylamino, pyridoylamino, lower alkoxycarbonylamino or phenyl-lower alkoxycarbonylamino, $alk_1$ is lower alkylene or lower alkylidene and the dotted line is intended to show that there is a single bond or a double bond between the carbon atoms carrying the radicals $R_1$ and $R_2$, wherein "lower" groups may each contain from 1 up to and including 4 carbon atoms, or a tautomer and/or salt thereof.

**3.** A compound of formula I according to claim 1, wherein $R_3$ is a group Ia, Ib or Ic, R is a benzocyclobuten-1-yl radical, indan-1-yl or indan-2-yl radical or 1,2,3,4-tetrahydronaphth-1-yl or 1,2,3,4-tetrahydronaphth-2-yl radical each of which is unsubstituted or is mono- or poly-substituted in the benzo moiety by hydroxy, lower alkoxy, lower alkanoyloxy and/or by halogen, and/or substituted in the $\alpha$-position with respect to the benzo moiety by lower alkyl, R' is a benzocyclobuten-1-ylidene radical which is unsubstituted or is mono- or poly-substituted in the benzo moiety by hydroxy, lower alkoxy, lower alkanoyloxy and/or by halogen, $R_1$ is carboxy, lower alkoxycarbonyl or carbamoyl, $R_2$ is hydrogen, hydroxy, lower alkoxy, lower alkanoyloxy, lower alkanesulfonyloxy, phenyl-lower alkoxy which is unsubstituted or is substituted in the phenyl moiety by lower alkyl, lower alkoxy and/or by halogen, or is amino, $alk_1$ is lower alkylene or lower alkylidene which links the two ring systems by from 1 up to and including 3 carbon atoms or by 1 carbon atom, respectively, $alk_2$ is lower alkyl-$\omega$-ylidene which links the two ring systems by 2 or 3 carbon atoms, and the dotted line is intended to show that there is a single bond or a double bond between the carbon atoms carrying the radicals $R_1$ and $R_2$, wherein "lower" groups may each contain from 1 up to and including 4 carbon atoms, or a tautomer and/or salt thereof.

**4.** A compound of formula I according to claim 1, wherein $R_3$ is a group Ia or Ib, R is a benzocyclobuten-1-yl radical, indan-1-yl radical, indan-2-yl radical or 1,2,3,4-tetrahydronaphth-1-yl radical each of which is unsubstituted or is mono-substituted in the benzo moiety by lower alkoxy having up to and including 4 carbon atoms, lower alkyl having up to and including 4 carbon atoms or by halogen having an atomic number of up to and including 35, or di-substituted in the benzo moiety by lower alkyl having up to and including 4 carbon atoms or by lower alkoxy having up to and including 4 carbon atoms and lower alkyl having up to and including 4 carbon atoms, $R_1$ is lower alkoxycarbonyl having from 1 up to and including 4 carbon atoms in the lower alkoxy moiety or is carbamoyl, $R_2$ is hydrogen or hydroxy, $alk_1$ is lower alkylene having up to and including 3 carbon atoms which bridges the ring systems by from 1 up to and including 3 carbon atoms, and the dotted line is intended to show that there is a single bond or a double bond between the carbon atoms carrying the radicals $R_1$ and $R_2$, or a tautomer and/or salt thereof.

**5.** A compound of formula I according to claim 2, wherein $R_3$ is a group of formula Ib, R is a benzocyclobuten-1-yl radical which is unsubstituted or is substituted in the benzo moiety by lower alkoxy having up to and including 4 carbon atoms, $R_1$ is lower alkoxycarbonyl having from 1 up to and including 4 carbon atoms in the lower alkoxy moiety or is carbamoyl, $R_2$ is hydrogen or hydroxy, $alk_1$ is lower alkylene having up to and including 3 carbon atoms which bridges the ring systems by from 1 up

to and including 3 carbon atoms, and the dotted line is intended to show that there is a single bond or a double bond between the carbon atoms carrying the radicals $R_1$ and $R_2$, or a tautomer and/or salt thereof.

**6.** 4-Hydroxy-1-[(5-methoxybenzocyclobuten-1-yl)methyl]-1,2,5,6-tetrahydro-pyridine-3-carboxylic acid ethyl ester alias 1-[(5-methoxybenzocyclobuten-1-yl)methyl]-4-oxo-piperidine-3-carboxylic acid ethyl ester or a salt thereof according to claim 2.

**7.** cis-4-Hydroxy-1-[(5-methoxybenzocyclobuten-1-yl)methyl]-piperidine-3-carboxylic acid ethyl ester or a salt thereof according to claim 2.

**8.** trans-4-Hydroxy-1-[2-(5-methoxybenzocyclobuten-1-yl)ethyl]-piperidine-3-carboxylic acid ethyl ester or a salt thereof according to claim 2.

**9.** 4-Amino-1-[(5-methoxybenzocyclobuten-1-yl)methyl]-1,2,5,6-tetrahydro-pyridine-3-carboxylic acid ethyl ester alias 1-[(5-methoxybenzocyclobuten-1-yl)methyl]-4-imino-piperidine-3-carboxylic acid ethyl ester or a salt thereof according to claim 2.

**10.** 4-Hydroxy-1-[2-(5-methoxybenzocyclobuten-1-yl)ethyl]-1,2,5,6-tetrahydro-pyridine-3-carboxylic acid methyl ester alias 1-[2-(5-methoxybenzocyclobuten-1-yl)ethyl]-4-oxo-piperidine-3-carboxylic acid methyl ester or a salt thereof according to claim 2.

**11.** 4-Hydroxy-1-[(benzocyclobuten-1-yl)methyl]-1,2,5,6-tetrahydro-pyridine-3-carboxylic acid ethyl ester alias 1-[(benzocyclobuten-1-yl)methyl]-4-oxo-piperidine-3-carboxylic acid ethyl ester or a salt thereof according to claim 1.

**12.** 1-[(6-Methoxyindan-1-yl)methyl]-1,2,5,6-tetrahydro-pyridine-3-carboxylic acid methyl ester or a salt thereof according to claim 1.

**13.** 4-Hydroxy-1-[(5-methoxy-1-methyl-benzocyclobuten-1-yl)methyl]-1,2,5,6-tetrahydro-pyridine-3-carboxylic acid ethyl ester alias 1-[(5-methoxy-1-methyl-benzocyclobuten-1-yl)methyl]-4-oxo-piperidine-3-carboxylic acid ethyl ester or a salt thereof according to claim 1.

**14.** 4-Hydroxy-1-(benzocyclobuten-1-yl)-1,2,5,6-tetrahydro-pyridine-3-carboxylic acid ethyl ester alias 1-(benzocyclobuten-1-yl)-4-oxo-piperidine-3-carboxylic acid ethyl ester or a salt thereof according to claim 1.

**15.** 1-[(7-Methoxy-1,2,3,4-tetrahydro-naphth-2-yl)methyl]-1,2,5,6-tetrahydro-pyridine-3-carboxylic acid methyl ester or a salt thereof according to claim 1.

**16.** 4-Hydroxy-1-[(4-methoxybenzocyclobuten-1-yl)methyl]-1,2,5,6-tetrahydro-pyridine-3-carboxylic acid ethyl ester alias 1-[(4-methoxybenzocyclobuten-1-yl)methyl]-4-oxo-piperidine-3-carboxylic acid ethyl ester or a salt thereof according to claim 1.

**17.** 4-Hydroxy-1-[(7-methoxy-1,2,3,4-tetrahydro-naphth-2-yl)methyl]-1,2,5,6-tetrahydro-pyridine-3-carboxylic acid methyl ester alias 1-[(7-methoxy-1,2,3,4-tetrahydro-naphth-2-yl)methyl]-4-oxo-piperidine-3-carboxylic acid methyl ester or a salt thereof according to claim 1.

**18.** 4-Hydroxy-1-(6-methoxyindan-1-yl)-1,2,5,6-tetrahydro-pyridine-3-carboxylic acid ethyl ester alias 1-(6-methoxyindan-1-yl)-4-oxo-piperidine-3-carboxylic acid ethyl ester or a salt thereof according to claim 1.

**19.** 4-Hydroxy-1-(7-methoxy-1,2,3,4-tetrahydro-naphth-2-yl)-1,2,5,6-tetrahydro-pyridine-3-carboxylic acid methyl ester alias 1-(7-methoxy-1,2,3,4-tetrahydro-naphth-2-yl)-4-oxo-piperidine-3-carboxylic acid methyl ester or a salt thereof according to claim 1.

**20.** A compound according to any one of claims 1 to 19 or a tautomer and/or a pharmaceutically acceptable salt thereof for use in a method for the therapeutic treatment of the human or animal body.

**21.** A compound according to any one of claims 2 and 5 to 10 or a tautomer and/or a pharmaceutically acceptable salt thereof for use in a method for the therapeutic treatment of the human or animal body.

**22.** A compound according to any one of claims 1 to 21 or a tautomer and/or a pharmaceutically acceptable salt thereof for use as a nootropic.

**23.** A compound according to any one of claims 2, 5 to 10 and 21 or a tautomer and/or a pharmaceutically acceptable salt thereof for use as a nootropic.

**24.** A pharmaceutical composition comprising as active ingredient a compound according to any one of claims 1 to 23 or a tautomer and/or a pharmaceutically acceptable salt thereof, where appropriate together with customary pharmaceutical excipients.

**25.** A pharmaceutical composition comprising as active ingredient a compound according to any one of claims 2, 5 to 10, 21 and 23 or a tautomer and/or a pharmaceutically acceptable salt thereof, where appropriate together with customary pharmaceutical excipients.

**26.** A nootropically active pharmaceutical composition according to claim 24 or claim 25, wherein a nootropically active active ingredient is selected.

**27.** A nootropically active pharmaceutical composition according to claim 25, wherein a nootropically active active ingredient is selected.

**28.** A process for the preparation of a compound of formula I according to any one of claims 1 to 19 or a tautomer and/or salt thereof, wherein
a) a compound of formula

$R_3$-$X_1$ (IIa),

or a salt thereof, wherein $X_1$ is hydroxy or reactive esterified hydroxy, is reacted with a compound of formula

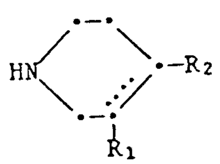

(IIb)

or with a tautomer and/or salt thereof, or
b) in a compound of formula

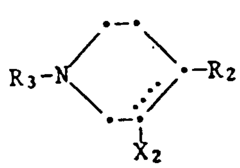

(III),

or in a tautomer and/or salt thereof, wherein $X_2$ is cyano, halocarbonyl, unsubstituted or $C_1$-$C_4$alkyl-substituted amidino, $C_1$-$C_4$alkanoyloxycarbonyl, $C_1$-$C_4$alkoxycarbonyloxycarbonyl, carboximidoyl, $C_1$-$C_4$alkoxycarbimidoyl, halocarbimidoyl, tri-$C_1$-$C_4$alkoxymethyl or trihalomethyl, $X_2$ is converted by solvolysis into $R_1$, or

c) for the preparation of a compound of formula I wherein $R_2$ is hydroxy or amino and wherein $R_1$ is especially $C_1$-$C_4$alkoxycarbonyl, or a tautomer and/or salt thereof, a compound of formula

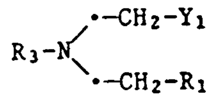

(IV),

wherein $Y_1$ is a group of formula $-CH=R_2'$, $-C(Y_2)=R_2'$ or cyano, and $R_2'$ is oxo or imino, $Y_2$ being $C_1$-$C_4$alkoxy, or a salt thereof, is cyclised, or
d) for the preparation of a compound of formula I wherein $R_2$ is hydroxy or amino and the dotted line is intended to show that there is a double bond between the carbon atoms carrying the radicals $R_1$ and $R_2$, and wherein $R_1$ is especially $C_1$-$C_4$alkoxycarbonyl, or a tautomer and/or salt thereof, a compound of formula

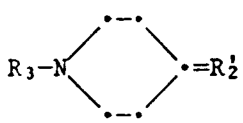

(Va),

wherein $R_2'$ is oxo or imino, or a tautomer or salt thereof, is reacted with a compound of formula

$X_3$-$R_1$ (Vb),

wherein $X_3$ is halogen or $C_1$-$C_4$alkoxy, or with a salt thereof, or
e) for the preparation of a compound of formula I wherein $R_2$ is other than hydrogen, or a tautomer and/or salt thereof, in a compound of formula

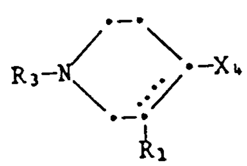

(VI),

or in a salt thereof, wherein $X_4$ is halogen or a diazonium group of formula $-N_2^{\oplus}A^{\ominus}$ wherein $A^{\ominus}$ is the anion of a mineral acid, $X_4$ is converted by solvolysis into $R_2$, or
f) especially for the preparation of a compound of formula I wherein $R_2$ is hydrogen, or a tautomer and/or salt thereof, in a compound of formula

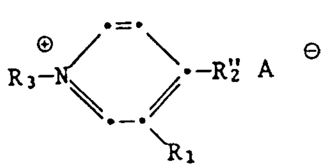

(VII),

wherein $A^{\ominus}$ is the anion of an acid and $R_2''$ is hydrogen, the excess double bonds are reduced to single bonds and, in each case, a protecting group which may be present is removed and, in each case, if desired, a compound of formula I obtainable according to the process or in a different manner is converted into a different compound of formula I, an isomeric mixture obtainable according to the process is separated into its components, an enantiomeric or diastereoisomeric mixture obtainable according to the process is split into the enantiomers or diastereoisomers, respectively, and/or a free compound of formula I obtainable according to the process is converted into a salt or a salt obtainable according to the process is converted into a free compound of formula I or into a different salt.

**29.** A process for the preparation of a pharmaceutical composition according to claim 24, wherein the active ingredient is processed to form a pharmaceutical composition, where appropriate with the admixture of customary pharmaceutical excipients.

**30.** A process according to claim 29 for the preparation of a nootropically active pharmaceutical composition according to claim 26, wherein a nootropically active active ingredient is selected.

**31.** The use of a compound according to any one of claims 1 to 23, or a tautomer and/or a pharmaceutically acceptable salt thereof, for the preparation of a pharmaceutical composition.

**32.** The use according to claim 31 for the preparation of a nootropic.

**33.** The use of a compound according to any one of claims 1 to 23, or a tautomer and/or a pharmaceutically acceptable salt thereof, for the preparation of a pharmaceutical composition by non-chemical means.

**Claims for the following Contracting State : AT**

**1.** A process for the preparation of a novel hydropyridine derivative of formula

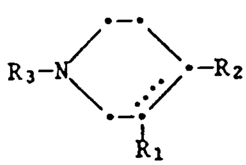

(I)

wherein $R_1$ is carboxy, lower alkoxycarbonyl, carbamoyl, N-lower alkylcarbamoyl or N,N-di-lower alkylcarbamoyl, $R_2$ is hydrogen, hydroxy, lower alkoxy, unsubstituted or lower alkyl-, lower alkoxy- and/or halo-substituted phenyl-lower alkoxy, lower alkanoyloxy, benzoyloxy, pyridoyloxy, lower alkanesulfonyloxy, lower alkoxycarbonyloxy, phenyl-lower alkoxycarbonyloxy, amino, lower alkanoylamino, benzoylamino, pyridoylamino, lower alkanesulfonylamino, lower alkoxycarbonylamino or phenyl-lower alkoxycarbonylamino, $R_3$ is a radical of the formula R- (Ia), R-$alk_1$- (Ib) or R'=$alk_2$- (Ic) wherein R is a benzocycloalkenyl radical having a total of from 8 to 12 ring carbon atoms which is bonded via a saturated carbon atom and which is unsubstituted or is mono- or poly-substituted in the benzo moiety by hydroxy, lower alkoxy, lower alkanoyloxy, halogen, lower alkyl and/or by trifluoromethyl and/or substituted in the $\alpha$-position with respect to the benzo moiety by lower alkyl, R' is a benzocycloalkenylidene radical having a total of from 8 to 12 ring carbon atoms which is unsubstituted or is mono- or poly-substituted in the benzo moiety by hydroxy, lower alkoxy, lower alkanoyloxy, halogen, lower alkyl and/or by trifluoromethyl, $alk_1$ is lower alkylene or lower alkylidene, $alk_2$ is lower alkyl-$\omega$-ylidene and the dotted line is intended to show that there is a single bond or a double bond between the carbon atoms carrying the radicals $R_1$ and $R_2$, wherein "lower" groups may each contain from 1 up to and including 4 carbon atoms, or a tautomer and/or salt thereof, wherein

a) a compound of formula

$R_3$-$X_1$ (IIa),

or a salt thereof, wherein $X_1$ is hydroxy or reactive esterified hydroxy, is reacted with a compound of formula

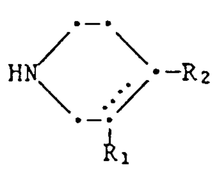

(IIb)

or with a tautomer and/or salt thereof, or

b) in a compound of formula

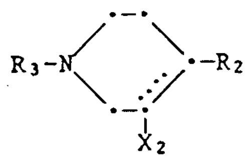

(III),

or in a tautomer and/or salt thereof, wherein $X_2$ is cyano, halocarbonyl, unsubstituted or $C_1$-$C_4$alkyl-substituted amidino, $C_1$-$C_4$alkanoyloxycarbonyl, $C_1$-$C_4$alkoxycarbonyloxycarbonyl, carboximidoyl, $C_1$-$C_4$alkoxycarbimidoyl, halocarbimidoyl, tri-$C_1$-$C_4$alkoxymethyl or trihalomethyl, $X_2$ is converted by solvolysis into $R_1$, or

c) for the preparation of a compound of formula I wherein $R_2$ is hydroxy or amino and wherein $R_1$ is especially $C_1$-$C_4$alkoxycarbonyl, or a tautomer and/or salt thereof, a compound of formula

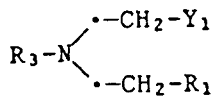

(IV),

wherein $Y_1$ is a group of formula $-CH=R_2'$, $-C(Y_2)=R_2'$ or cyano, and $R_2'$ is oxo or imino, $Y_2$ being $C_1$-$C_4$alkoxy, or a salt thereof, is cyclised, or

d) for the preparation of a compound of formula I wherein $R_2$ is hydroxy or amino and the dotted line is intended to show that there is a double bond between the carbon atoms carrying the radicals $R_1$ and $R_2$, and wherein $R_1$ is especially $C_1$-$C_4$alkoxycarbonyl, or a tautomer and/or salt thereof, a compound of formula

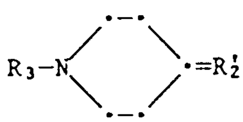

(Va),

wherein $R_2'$ is oxo or imino, or a tautomer or salt thereof, is reacted with a compound of formula

$X_3$-$R_1$ (Vb),

wherein $X_3$ is halogen or $C_1$-$C_4$alkoxy, or with a salt thereof, or

e) for the preparation of a compound of formula I wherein $R_2$ is other than hydrogen, or a tautomer and/or salt thereof, in a compound of formula

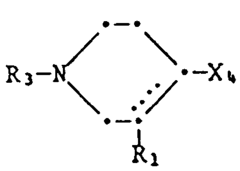

(VI),

or in a salt thereof, wherein $X_4$ is halogen or a diazonium group of formula $-N_2^{\oplus}A^{\ominus}$ wherein $A^{\ominus}$ is the anion of a mineral acid, $X_4$ is converted by solvolysis into $R_2$, or

f) especially for the preparation of a compound of formula I wherein $R_2$ is hydrogen, or a tautomer and/or salt thereof, in a compound of formula

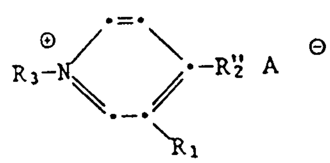

(VII),

wherein $A^{\ominus}$ is the anion of an acid and $R_2''$ is hydrogen, the excess double bonds are reduced to single bonds and, in each case, a protecting group which may be present is removed and, in each case, if desired, a compound of formula I obtainable according to the process or in a different manner is converted into a different compound of formula I, an isomeric mixture obtainable according to the process is separated into its components, an enantiomeric or diastereoisomeric mixture obtainable according to the process is split into the enantiomers or diastereoisomers, respectively, and/or a free compound of formula I obtainable according to the process is converted into a salt or a salt obtainable according to the process is converted into a free compound of formula I or into a different salt.

**2.** A process according to claim 1 for the preparation of a compound of formula I wherein $R_3$ is a group Ia or Ib, R is a benzocyclobuten-1-yl radical which is unsubstituted or is mono- or poly-substituted in the benzo moiety by hydroxy, lower alkoxy, lower alkanoyloxy, halogen, lower alkyl and/or by trifluoromethyl and/or substituted in the $\alpha$-position with respect to the benzo moiety by lower alkyl, $R_1$ is carboxy, lower alkoxycarbonyl, carbamoyl, N-lower alkylcarbamoyl or N,N-di-lower alkylcarbamoyl, $R_2$ is hydrogen, hydroxy, lower alkoxy, phenyl-lower alkoxy, lower alkanoyloxy, benzoyloxy, pyridoyloxy, lower alkoxycarbonyloxy, phenyl-lower alkoxycarbonyloxy, amino, lower alkanoylamino, benzoylamino, pyridoylamino, lower alkoxycarbonylamino or phenyl-lower alkoxycarbonylamino, $alk_1$ is lower alkylene or lower alkylidene and the dotted line is intended to show that there is a single bond or a double bond between the carbon atoms carrying the radicals $R_1$ and $R_2$, wherein "lower" groups may each contain from 1 up to and including 4 carbon atoms, or a tautomer and/or salt thereof.

**3.** A process according to claim 1 for the preparation of a compound of formula I wherein $R_3$ is a group Ia, Ib or Ic, R is a benzocyclobuten-1-yl radical, indan-1-yl or indan-2-yl radical or 1,2,3,4-tetrahydronaphth-1-yl or 1,2,3,4-tetrahydronaphth-2-yl radical each of which is unsubstituted or is mono- or poly-substituted in the benzo moiety by hydroxy, lower alkoxy, lower alkanoyloxy and/or by halogen, and/or substituted in the $\alpha$-position with respect to the benzo moiety by lower alkyl, R' is a benzocyclobuten-1-ylidene radical which is unsubstituted or is mono-or poly-substituted in the benzo moiety by hydroxy, lower alkoxy, lower alkanoyloxy and/or by halogen, $R_1$ is carboxy, lower alkoxycarbonyl or carbamoyl, $R_2$ is hydrogen, hydroxy, lower alkoxy, lower alkanoyloxy, lower alkanesulfonyloxy, phenyl-lower alkoxy which is unsubstituted or is substituted in the phenyl moiety by lower alkyl, lower alkoxy and/or by halogen, or is amino, $alk_1$ is lower alkylene or lower alkylidene which links the two ring systems by from 1 up to and including 3 carbon atoms or by 1 carbon atom, respectively, $alk_2$ is lower alkyl-$\omega$-ylidene which links the two ring systems by 2 or 3 carbon atoms, and the dotted line is intended to show that there is a single bond or a double bond between the carbon atoms carrying the radicals $R_1$ and $R_2$, wherein "lower" groups may each contain from 1 up to and including 4 carbon atoms, or a tautomer and/or salt thereof.

**4.** A process according to claim 1 for the preparation of a compound of formula I wherein $R_3$ is a group Ia or Ib, R is a benzocyclobuten-1-yl radical, indan-1-yl radical, indan-2-yl radical or 1,2,3,4-tetrahydronaphth-1-yl radical each of which is unsubstituted or is mono-substituted in the benzo moiety by lower alkoxy having up to and including 4 carbon atoms, lower alkyl having up to and including 4 carbon atoms or by halogen having an atomic number of up to and including 35, or di-substituted in the benzo moiety by lower alkyl having up to and including 4 carbon atoms or by lower alkoxy having up to and including 4 carbon atoms and lower alkyl having up to and including 4 carbon atoms, $R_1$ is lower alkoxycarbonyl having from 1 up to and including 4 carbon atoms in the lower alkoxy moiety or is carbamoyl, $R_2$ is hydrogen or hydroxy, $alk_1$ is lower alkylene having up to and including 3 carbon

atoms which bridges the ring systems by from 1 up to and including 3 carbon atoms, and the dotted line is intended to show that there is a single bond or a double bond between the carbon atoms carrying the radicals $R_1$ and $R_2$, or a tautomer and/or salt thereof.

**5.** A process according to claim 1 for the preparation of a compound of formula I wherein $R_3$ is a group of formula Ib, R is a benzocyclobuten-1-yl radical which is unsubstituted or is substituted in the benzo moiety by lower alkoxy having up to and including 4 carbon atoms, $R_1$ is lower alkoxycarbonyl having from 1 up to and including 4 carbon atoms in the lower alkoxy moiety or is carbamoyl, $R_2$ is hydrogen or hydroxy, $alk_1$ is lower alkylene having up to and including 3 carbon atoms which bridges the ring systems by from 1 up to and including 3 carbon atoms, and the dotted line is intended to show that there is a single bond or a double bond between the carbon atoms carrying the radicals $R_1$ and $R_2$, or a tautomer and/or salt thereof.

**6.** A process according to claim 1 for the preparation of 4-hydroxy-1-[(5-methoxybenzocyclobuten-1-yl)-methyl]-1,2,5,6-tetrahydro-pyridine-3-carboxylic acid ethyl ester alias 1-[(5-methoxybenzocyclobuten-1-yl)methyl]-4-oxo-piperidine-3-carboxylic acid ethyl ester or a salt thereof.

**7.** A process according to claim 2 for the preparation of cis-4-hydroxy-1-[(5-methoxybenzocyclobuten-1-yl)methyl]piperidine-3-carboxylic acid ethyl ester or a salt thereof.

**8.** A process according to claim 2 for the preparation of trans-4-hydroxy-1-[2-(5-methoxybenzocyclobuten-1-yl)ethyl]-piperidine-3-carboxylic acid ethyl ester or a salt thereof.

**9.** A process according to claim 2 for the preparation of 4-amino-1-[(5-methoxybenzocyclobuten-1-yl)-methyl]-1,2,5,6-tetrahydro-pyridine-3-carboxylic acid ethyl ester alias 1-[(5-methoxybenzocyclobuten-1-yl)methyl]-4-imino-piperidine-3-carboxylic acid ethyl ester or a salt thereof.

**10.** A process according to claim 2 for the preparation of 4-hydroxy-1-[2-(5-methoxybenzocyclobuten-1-yl)-ethyl]-1,2,5,6-tetrahydro-pyridine-3-carboxylic acid methyl ester alias 1-[2-(5-methoxybenzocyclobuten-1-yl)ethyl]-4-oxo-piperidine-3-carboxylic acid methyl ester or a salt thereof.

**11.** A process according to claim 1 for the preparation of 4-hydroxy-1-[(benzocyclobuten-1-yl)methyl]-1,2,5,6-tetrahydro-pyridine-3-carboxylic acid ethyl ester alias 1-[(benzocyclobuten-1-yl)methyl]-4-oxo-piperidine-3-carboxylic acid ethyl ester or a salt thereof.

**12.** A process according to claim 1 for the preparation of 1-[(6-methoxyindan-1-yl)methyl]-1,2,5,6-tetrahydro-pyridine-3-carboxylic acid methyl ester or a salt thereof.

**13.** A process according to claim 1 for the preparation of 4-hydroxy-1-[(5-methoxy-1-methyl-benzocyclobuten-1-yl)methyl]-1,2,5,6-tetrahydro-pyridine-3-carboxylic acid ethyl ester alias 1-[(5-methoxy-1-methyl-benzocyclobuten-1-yl)methyl]-4-oxo-piperidine-3-carboxylic acid ethyl ester or a salt thereof.

**14.** A process according to claim 1 for the preparation of 4-hydroxy-1-(benzocyclobuten-1-yl)-1,2,5,6-tetrahydro-pyridine-3-carboxylic acid ethyl ester alias 1-(benzocyclobuten-1-yl)-4-oxo-piperidine-3-carboxylic acid ethyl ester or a salt thereof.

**15.** A process according to claim 1 for the preparation of 1-[(7-methoxy-1,2,3,4-tetrahydro-naphth-2-yl)-methyl]-1,2,5,6-tetrahydro-pyridine-3-carboxylic acid methyl ester or a salt thereof.

**16.** A process according to claim 1 for the preparation of 4-hydroxy-1-[(4-methoxybenzocyclobuten-1-yl)-methyl]-1,2,5,6-tetrahydro-pyridine-3-carboxylic acid ethyl ester alias 1-[(4-methoxybenzocyclobuten-1-yl)methyl]-4-oxo-piperidine-3-carboxylic acid ethyl ester or a salt thereof.

**17.** A process according to claim 1 for the preparation of 4-hydroxy-1-[(7-methoxy-1,2,3,4-tetrahydro-naphth-2-yl)methyl]-1,2,5,6-tetrahydro-pyridine-3-carboxylic acid methyl ester alias 1-[(7-methoxy-1,2,3,4-tetrahydro-naphth-2-yl)methyl]-4-oxo-piperidine-3-carboxylic acid methyl ester or a salt thereof.

**18.** A process according to claim 1 for the preparation of 4-hydroxy-1-(6-methoxyindan-1-yl)-1,2,5,6-tetrahydro-pyridine-3-carboxylic acid ethyl ester alias 1-(6-methoxyindan-1-yl)-4-oxo-piperidine-3-carboxylic acid ethyl ester or a salt thereof.

**19.** A process according to claim 1 for the preparation of 4-hydroxy-1-(7-methoxy-1,2,3,4-tetrahydro-naphth-2-yl)-1,2,5,6-tetrahydro-pyridine-3-carboxylic acid methyl ester alias 1-(7-methoxy-1,2,3,4-tetrahydro-naphth-2-yl)-4-oxo-piperidine-3-carboxylic acid methyl ester or a salt thereof.

**20.** A process for the preparation of a pharmaceutical composition comprising as active ingredient a compound obtainable according to any one of claims 1 to 19, or a tautomer and/or a pharmaceutically acceptable salt thereof, wherein the active ingredient is mixed with customary pharmaceutical excipients.

**21.** A process for the preparation of a pharmaceutical composition comprising as active ingredient a compound obtainable according to any one of claims 2 and 5 to 10, or a tautomer and/or a pharmaceutically acceptable salt thereof, wherein the active ingredient is mixed with customary pharmaceutical excipients.

**22.** A process for the preparation of a nootropically active pharmaceutical composition according to claim 20 or claim 21, wherein a nootropically active active ingredient is selected.

**23.** A process for the preparation of a nootropically active pharmaceutical composition according to claim 21, wherein a nootropically active active ingredient is selected.

**24.** A process for the preparation of a pharmaceutical composition, wherein

a) a compound of formula

$R_3$-$X_1$ (IIa),

or a salt thereof, wherein $X_1$ is hydroxy or reactive esterified hydroxy, is reacted with a compound of formula

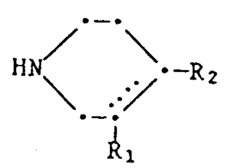

(IIb)

or with a tautomer and/or salt thereof, or

b) in a compound of formula

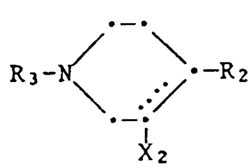

(III),

or in a tautomer and/or salt thereof, wherein $X_2$ is cyano, halocarbonyl, unsubstituted or $C_1$-$C_4$alkyl-substituted amidino, $C_1$-$C_4$alkanoyloxycarbonyl, $C_1$-$C_4$alkoxycarbonyloxycarbonyl, carboximidoyl, $C_1$-$C_4$alkoxycarbimidoyl, halocarbimidoyl, tri-$C_1$-$C_4$alkoxymethyl or trihalomethyl, $X_2$ is converted by solvolysis into $R_1$, or

c) for the preparation of a compound of formula I wherein $R_2$ is hydroxy or amino and wherein $R_1$ is especially $C_1$-$C_4$alkoxycarbonyl, or a tautomer and/or salt thereof, a compound of formula

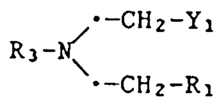

(IV),

wherein $Y_1$ is a group of formula $-CH=R_2'$, $-C(Y_2)=R_2'$ or cyano, and $R_2'$ is oxo or imino, $Y_2$ being $C_1$-$C_4$alkoxy, or a salt thereof, is cyclised, or

d) for the preparation of a compound of formula I wherein $R_2$ is hydroxy or amino and the dotted line is intended to show that there is a double bond between the carbon atoms carrying the radicals $R_1$ and $R_2$, and wherein $R_1$ is especially $C_1$-$C_4$alkoxycarbonyl, or a tautomer and/or salt thereof, a compound of formula

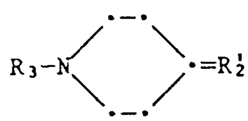

(Va),

wherein $R_2'$ is oxo or imino, or a tautomer or salt thereof, is reacted with a compound of formula

$X_3$-$R_1$ (Vb),

wherein $X_3$ is halogen or $C_1$-$C_4$alkoxy, or with a salt thereof, or

e) for the preparation of a compound of formula I wherein $R_2$ is other than hydrogen, or a tautomer and/or salt thereof, in a compound of formula

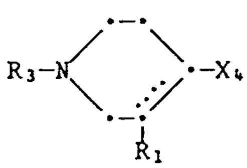

(VI),

or in a salt thereof, wherein $X_4$ is halogen or a diazonium group of formula $-N_2^{\oplus}A^{\ominus}$ wherein $A^{\ominus}$ is the anion of a mineral acid, $X_4$ is converted by solvolysis into $R_2$, or

f) especially for the preparation of a compound of formula L wherein $R_2$ is hydrogen, or a tautomer and/or salt thereof, in a compound of formula

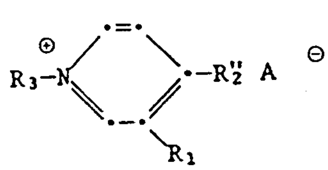

(VII),

wherein $A^{\ominus}$ is the anion of an acid and $R_2''$ is hydrogen, the excess double bonds are reduced to single bonds and, in each case, a protecting group which may be present is removed and, in each case, if desired, a compound of formula I obtainable according to the process or in a different manner is converted into a different compound of formula I, an isomeric mixture obtainable according to the process is separated into its components, an enantiomeric or diastereoisomeric mixture obtainable according to the process is split into the enantiomers or diastereoisomers, respectively, and/or a free compound of formula I obtainable according to the process is converted into a pharmaceutically acceptable salt or a pharmaceutically acceptable salt obtainable according to

the process is converted into a free compound of formula I or into a different pharmaceutically acceptable salt, and a hydropyridine derivative obtainable in that manner of formula

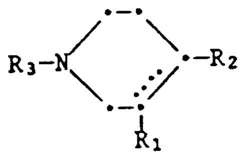

(I)

wherein $R_1$ is carboxy, lower alkoxycarbonyl, carbamoyl, N-lower alkylcarbamoyl or N,N-di-lower alkylcarbamoyl, $R_2$ is hydrogen, hydroxy, lower alkoxy, unsubstituted or lower alkyl-, lower alkoxy- and/or halo-substituted phenyl-lower alkoxy, lower alkanoyloxy, benzoyloxy, pyridoyloxy, lower alkanesulfonyloxy, lower alkoxycarbonyloxy, phenyl-lower alkoxycarbonyloxy, amino, lower alkanoylamino, benzoylamino, pyridoylamino, lower alkanesulfonylamino, lower alkoxycarbonylamino or phenyl-lower alkoxycarbonylamino, $R_3$ is a radical of the formula R- (Ia), R-alk$_1$- (Ib) or R' = alk$_2$- (Ic) wherein R is a benzocycloalkenyl radical having a total of from 8 to 12 ring carbon atoms which is bonded via a saturated carbon atom and which is unsubstituted or is mono- or poly-substituted in the benzo moiety by hydroxy, lower alkoxy, lower alkanoyloxy, halogen, lower alkyl and/or by trifluoromethyl and/or substituted in the $\alpha$-position with respect to the benzo moiety by lower alkyl, R' is a benzocycloalkenylidene radical having a total of from 8 to 12 ring carbon atoms which is unsubstituted or is mono- or poly-substituted in the benzo moiety by hydroxy, lower alkoxy, lower alkanoyloxy, halogen, lower alkyl and/or by trifluoromethyl, alk$_1$ is lower alkylene or lower alkylidene, alk$_2$ is lower alkyl-$\omega$-ylidene and the dotted line is intended to show that there is a single bond or a double bond between the carbon atoms carrying the radicals $R_1$ and $R_2$, wherein "lower" groups may each contain from 1 up to and including 4 carbon atoms, or a tautomer and/or a pharmaceutically acceptable salt thereof obtainable in that manner is processed to form a pharmaceutical composition with the admixture of customary pharmaceutical excipients.

**25.** A process according to claim 24 for the preparation of a nootropically active pharmaceutical composition, wherein a nootropically active active ingredient is selected.

**26.** The use of a compound obtainable according to any one of claims 1 to 19, or a tautomer and/or a pharmaceutically acceptable salt thereof, for the preparation of a pharmaceutical composition.

**27.** The use according to claim 26 for the preparation of a nootropic.

**28.** The use of a compound obtainable according to any one of claims 1 to 19, or a tautomer and/or a pharmaceutically acceptable salt thereof, for the preparation of a pharmaceutical composition by non-chemical means.

**Revendications**

**Revendications pour les Etats contractants suivants : BE, CH, DE, FR, GB, IT, LI, LU, NL, SE**

**1.** Hydropyridines substituées de formule

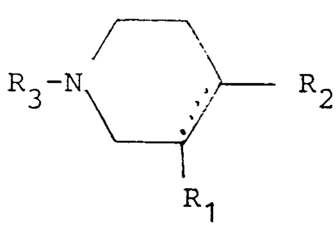

(I),

dans laquelle $R_1$ représente un groupe carboxy, (alcoxy inférieur)-carbonyle, carbamoyle, N-(alkyle inférieur)-carbamoyle ou N,N-di-(alkyle inférieur)-carbamoyle, $R_2$ représente l'hydrogène, un groupe

hydroxy, alcoxy inférieur, un groupe phényl-alcoxy inférieur éventuellement substitué par des groupes alkyle inférieurs, alcoxy inférieurs et/ou des halogènes, un groupe alcanoyloxy inférieur, benzoyloxy, pyridoyloxy, alcane-sulfonyloxy inférieur, (alcoxy inférieur)-carbonyloxy, phényl-(alcoxy inférieur)-carbonyloxy, amino, alcanoylamino inférieur, benzoylamino, pyridoylamino, alcane-sulfonylamino inférieur, (alcoxy inférieur)-carbonylamino ou phényl-(alcoxy inférieur)-carbonylamino, $R_3$ représente un groupe de formule R- (Ia), R-$alk_1$- (Ib) ou R'=$alk_2$- (Ic) dans lesquels R représente un groupe benzocycloalcényle relié par l'intermédiaire d'un atome de carbone saturé, non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle et/ou en position alpha de la partie benzo par un groupe alkyle inférieur, et qui contient au total de 8 à 12 atomes de carbone cycliques, R' représente un groupe benzocycloalcénylidène non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle, et qui contient au total de 8 à 12 atomes de carbone cycliques, $alk_1$ représente un groupe alkylène inférieur ou alkylidène inférieur, $alk_2$ un groupe (alkyle inférieur)-oméga-ylidène,
et le trait interrompu indique qu'entre les atomes de carbone portant les groupes $R_1$ et $R_2$, il y a une liaison simple ou double, les groupes qualifiés d'"inférieurs" contenant de 1 à 4 atomes de carbone inclus, et leurs tautomères et/ou leurs sels.

**2.** Composés de formule I de la revendication 1, dans laquelle $R_3$ représente un groupe Ia ou Ib, R un groupe benzocyclobutène-1-yle non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle et/ou en position alpha de la partie benzo par un groupe alkyle inférieur, $R_1$ représente un groupe carboxy, (alcoxy inférieur)-carbonyle, carbamoyle, N-(alkyle inférieur)-carbamoyle ou N,N-di-(alkyle inférieur)-carbamoyle, $R_2$ représente l'hydrogène, un groupe hydroxy, alcoxy inférieur, phényl-alcoxy inférieur, alcanoyloxy inférieur, benzoyloxy, pyridoyloxy, (alcoxy inférieur)-carbonyloxy, phényl-(alcoxy inférieur)-carbonyloxy, amino, alcanoylamino inférieur, benzoylamino, pyridoylamino, (alcoxy inférieur)-carbonylamino ou phényl-(alcoxy inférieur)-carbonylamino, $alk_1$ représente un groupe alkylène inférieur ou alkylidène inférieur et le trait interrompu indique qu'entre les atomes de carbone portant les groupes $R_1$ et $R_2$ il y a une liaison simple ou double, les groupes qualifiés d'"inférieurs" contenant chacun 1 à 4 atomes de carbone, et leurs tautomères et/ou leurs sels.

**3.** Composés de formule I de la revendication 1, dans laquelle $R_3$ représente un groupe Ia, Ib ou Ic, R représente un groupe benzocyclobutène-1-yle non substitué ou substitué dans la partie benzo une ou plusieurs fois par des groupes hydroxy, alcoxy inférieurs, alcanoyloxy inférieurs et/ou des halogènes et/ou en position alpha de la partie benzo par un groupe alkyle inférieur, un groupe indane-1-yle ou indane-2-yle ou 1,2,3,4-tétrahydronaphto-1-yle ou 1,2,3,4-tétrahydronaphto-2-yle, R' représente un groupe benzocyclobutène-1-ylidène non substitué ou substitué dans la partie benzo une ou plusieurs fois par des groupes hydroxy, alcoxy inférieurs, alcanoyloxy inférieurs et/ou des halogènes, $R_1$ représente un groupe carboxy, (alcoxy inférieur)-carbonyle ou carbamoyle, $R_2$ représente l'hydrogène, un groupe hydroxy, alcoxy inférieur, alcanoyloxy inférieur, alcane-sulfonyloxy inférieur, phényl-alcoxy inférieur éventuellement substitué dans la partie phényle par des groupes alkyle inférieurs, alcoxy inférieurs et/ou des halogènes, ou un groupe amino, $alk_1$ représente un groupe alkylène inférieur ou alkylidène inférieur qui relie les deux systèmes cycliques respectivement par 1 à 3 atomes de carbone inclus ou par un atome de carbone, $alk_2$ représente un groupe (alkyle inférieur)-oméga-ylidène qui relie les deux systèmes cycliques par 2 ou 3 atomes de carbone, et le trait interrompu indique qu'entre les atomes de carbone portant les groupes $R_1$ et $R_2$ il y a une liaison simple ou double, les groupes qualifiés d'"inférieurs" contenant chacun de 1 à 4 atomes de carbone inclus, et leurs tautomères et/ou leurs sels.

**4.** Composés de formule I de la revendication 1, dans laquelle $R_3$ représente un groupe Ia ou Ib, R représente un groupe benzocyclobutène-1-yle non substitué ou mono-substitué dans la partie benzo par un groupe alcoxy inférieur contenant jusqu'à 4 atomes de carbone inclus, alkyle inférieur contenant jusqu'à 4 atomes de carbone inclus ou un halogène de numéro atomique allant jusqu'à 35 inclus, ou disubstitué par des groupes alkyle inférieurs contenant jusqu'à 4 atomes de carbone inclus ou par un groupe alcoxy inférieur contenant jusqu'à 4 atomes de carbone inclus et un groupe alkyle inférieur contenant jusqu'à 4 atomes de carbone inclus, un groupe indane-1-yle, indane-2-yle ou 1,2,3,4-tétrahydronaphto-1-yle, $R_1$ représente un groupe (alcoxy inférieur)-carbonyle contenant 1 à 4 atomes

de carbone inclus dans la partie alcoxy inférieur, ou un groupe carbamoyle, $R_2$ représente l'hydrogène ou un groupe hydroxy, $alk_1$ représente un groupe alkylène inférieur contenant jusqu'à 3 atomes de carbone inclus et qui relie les systèmes cycliques par un pont de 1 à 3 atomes de carbone inclus, et le trait interrompu indique qu'entre les atomes de carbone portant les groupes $R_1$ et $R_2$ il y a une liaison simple ou double, et leurs tautomères et/ou leurs sels.

**5.** Composés de formule I de la revendication 2, dans laquelle $R_3$ représente un groupe de formule Ib, R représente un groupe benzocyclobutène-1-yle non substitué ou substitué dans la partie benzo par un groupe alcoxy inférieur contenant jusqu'à 4 atomes de carbone inclus, $R_1$ représente un groupe (alcoxy inférieur)-carbonyle contenant de 1 à 4 atomes de carbone inclus dans la partie alcoxy inférieur, ou un groupe carbamoyle, $R_2$ représente l'hydrogène ou un groupe hydroxy, $alk_1$ représente un groupe alkylène inférieur contenant jusqu'à 3 atomes de carbone inclus et qui relie les systèmes cycliques par un pont de 1 à 3 atomes de carbone, et le trait interrompu indique qu'entre les atomes de carbone portant les groupes $R_1$ et $R_2$ il y a une liaison simple ou double, et leurs tautomères et/ou leurs sels.

**6.** Le 4-hydroxy-1-[(5-méthoxybenzocyclobutène-1-yl)-méthyl]-1,2,5,6-tétrahydro-pyridine-3-carboxylate d'éthyle ou 1-[(5-métboxybenzocyclobutène-1-yl)-méthyl]-4-oxo-pipéridine-3-carboxylate d'éthyle ou un sel de ce composé selon revendication 2.

**7.** Le cis-4-bydroxy-1-[(5-méthoxybenzocyclobutène-1-yl)-méthyl]-pipéridine-3-carboxylate d'éthyle ou un de ses sels selon revendication 2.

**8.** Le trans-4-hydroxy-1-[2-(5-méthoxybenzocyclobutène-1-yl)-éthyl]-pipéridine-3-carboxylate d'éthyle ou un de ses sels selon revendication 2.

**9.** Le 4-amino-1-[(5-méthoxybenzocyclobutène-1-yl)-méthyl]-1,2,5,6-tétrahydro-pyridine-3-carboxylate d'éthyle ou 1-[(5-méthoxybenzocyclobutène-1-yl)-méthyl]-4-ininopipéridine-3-carboxylate d'éthyle ou un de ses sels selon revendication 2.

**10.** Le 4-hydroxy-1-[2-(5-méthoxybenzocyclobutène-1-yl)-éthyl]-1,2,5,6-tétrahydro-pyridine-3-carboxylate de méthyle ou 1-[2-(5-méthoxybenzocyclobutène-1-yl)-éthyl]-4-oxo-pipéridine-3-carboxylate de méthyle ou un de ses sels selon revendication 2.

**11.** Le 4-hydroxy-1-[(benzocyclobutène-1-yl)-méthyl]-1,2,5,6-tétrahydro-pyridine-3-carboxylate d'éthyle ou 1-[(benzocyclobutène-1-yl)-méthyl]-4-oxo-pipéridine-3-carboxylate d'éthyle ou un de ses sels selon revendication 1.

**12.** Le 1-[(6-méthoxyindane-1-yl)-méthyl]-1,2,5,6-tétrahydro-pyridine-3-carboxylate de méthyle ou un de ses sels selon revendication 1.

**13.** Le 4-hydroxy-1-[(5-méthoxy-1-méthyl-benzocyclobutène-1-yl)-méthyl]-1,2,5,6-tétrahydro-pyridine-3-carboxylate d'éthyle ou 1-[(5-méthoxy-1-méthyl-benzocyclobutène-1-yl)-méthyl]-4-oxo-pipéridine-3-carboxylate d'éthyle ou un de ses sels selon revendication 1.

**14.** Le 4-hydroxy-1-(benzocyclobutène-1-yl)-1,2,5,6-tétrahydro-pyridine-3-carboxylate d'éthyle ou 1-(benzocyclobutène-1-yl)-4-oxo-pipéridine-3-carboxylate d'éthyle ou un de ses sels selon revendication 1.

**15.** Le 1-[(7-méthoxy-1,2,3,4-tétrahydronaphto-2-yl)-méthyl]-1,2,5,6-tétrahydro-pyridine-3-carboxylate de méthyle ou un de ses sels selon revendication 1.

**16.** Le 4-hydroxy-1-[(4-méthoxybenzocyclobutène-1-yl)-méthyl]-1,2,5,6-tétrahydro-pyridine-3-carboxylate d'éthyle ou 1-[4-méthoxybenzocyclobutène-1-yl)-méthyl]-4-oxo-pipéridine-3-carboxylate d'éthyle ou un de ses sels selon revendication 1.

**17.** Le 4-hydroxy-1-[(7-méthoxy-1,2,3,4-tétrahydronaphto-2-yl)-méthyl]-1,2,5,6-tétrahydro-pyridine-3-carboxylate de méthyle ou 1-[(7-méthoxy-1,2,3,4-tétrahydronaphto-2-yl)-méthyl]-4-oxo-pipéridine-3-carboxylate de méthyle ou un de ses sels selon revendication 1.

**18.** Le 4-hydroxy-1-(6-méthoxyindane-1-yl)-1,2,5,6-tétrahydro-pyridine-3-carboxylate d'éthyle ou 1-(6-méthoxyindane-1-yl)-4-oxo-pipéridine-3-carboxylate d'éthyle ou un de ses sels selon revendication 1.

**19.** Le 4-hydroxy-1-(7-méthoxy-1,2,3,4-tétrahydro-naphto-2-yl)-1,2,5,6-tétrahydro-pyridine-3-carboxylate de méthyle ou 1-(7-méthoxy-1,2,3,4-tétrahydronaphto-2-yl)-4-oxo-pipéridine-3-carboxylate de méthyle ou un de ses sels selon revendication 1.

**20.** Un composé selon l'une des revendications 1 à 19, ou un tautomère et/ou un sel acceptable pour l'usage pharmaceutique d'un tel composé pour l'utilisation dans un procédé pour le traitement thérapeutique de l'organisme humain ou animal.

**21.** Un composé selon l'une des revendications 2 et 5 à 10, ou un tautomère et/ou un sel acceptable pour l'usage pharmaceutique d'un tel composé pour l'utilisation dans un procédé pour le traitement thérapeutique de l'organisme humain ou animal.

**22.** Un composé selon l'une des revendications 1 à 21 ou un tautomère et/ou un sel acceptable pour l'usage pharmaceutique d'un tel composé pour l'utilisation en tant qu'agent nootropique.

**23.** Un composé selon l'une des revendications 2, 5 à 10 et 21, ou un tautomère et/ou un sel acceptable pour l'usage pharmaceutique d'un tel composé pour l'utilisation en tant qu'agent nootropique.

**24.** Une composition pharmaceutique contenant un composé selon l'une des revendications 1 à 23 ou un tautomère et/ou un sel acceptable pour l'usage pharmaceutique d'un tel composé, éventuellement avec des produits auxiliaires pharmaceutiques usuels.

**25.** Une composition pharmaceutique contenant en tant que substance active un composé selon l'une des revendications 2, 5 à 10, 21 et 23 ou un tautomère et/ou un sel acceptable pour l'usage pharmaceutique d'un tel composé, éventuellement avec des produits auxiliaires pharmaceutiques usuels.

**26.** Une composition pharmaceutique à activité nootrope selon la revendication 24 ou 25, caractérisée en ce que l'on choisit une substance active possédant une activité nootrope.

**27.** Une composition pharmaceutique à activité nootrope selon revendication 25, caractérisée en ce que l'on choisit une substance active possédant une activité nootrope.

**28.** Procédé de préparation d'un composé de formule I selon l'une des revendications 1 à 19, ou d'un tautomère et/ou d'un sel d'un tel composé, caractérisé en ce que :

a) on fait réagir un composé de formule

$R_3$-$X_1$ (IIa)

ou un sel d'un tel composé, dans lequel $X_1$ représente un groupe hydroxy ou un groupe hydroxy estérifié réactif, avec un composé de formule

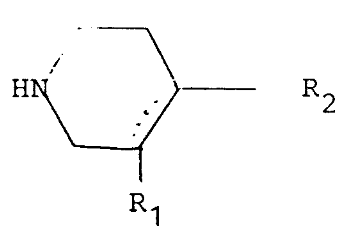

(IIb)

ou un tautomère et/ou un sel d'un tel composé, ou bien

b) dans un composé de formule

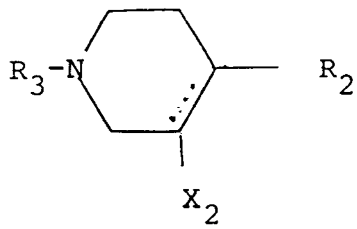

(III)

ou un tautomère et/ou un sel d'un tel composé, dans lequel $X_2$ représente un groupe cyano, halogénocarbonyle, amidino éventuellement substitué par un groupe alkyle en C 1-C 4, un groupe (alcanoyle en C 1-C 4)-oxycarbonyle, (alcoxy en C 1-C 4)-carbonyloxycarbonyle, carboximidoyle, (alcoxy en C 1-C 4)-carbimidoyle, halogénocarbimidoyle, tri-(alcoxy en C 1-C 4)-méthyle ou trihalogénométhyle, on convertit $X_2$ par solvolyse en $R_1$, ou bien

c) pour préparer les composés de formule I dans laquelle $R_2$ représente un groupe hydroxy ou amino et $R_1$ plus spécialement un groupe (alcoxy en C 1-C 4)-carbonyle, ou leurs tautomères et/ou leurs sels, on cyclise un composé de formule

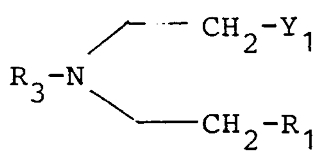

(IV),

dans laquelle $Y_1$ représente un groupe de formule $-CH=R'_2$, $-C(Y_2)=R'_2$ ou cyano et $R'_2$ représente un groupe oxo ou imino, et $Y_2$ un groupe alcoxy en C 1-C 4, ou un sel d'un tel composé, ou bien

d) pour préparer les composés de formule I dans laquelle $R_2$ représente un groupe hydroxy ou amino et le trait interrompu indique qu'entre les atomes de carbone portant les groupes $R_1$ et $R_2$, il y a une double liaison, et $R_1$ représente plus spécialement un groupe (alcoxy en C 1-C 4)-carbonyle, ou leurs tautomères et/ou leurs sels, on fait réagir un composé de formule

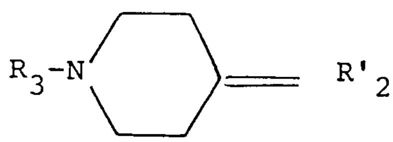

(Va),

dans laquelle $R'_2$ représente un groupe oxo ou imino, ou un tautomère ou sel d'un tel composé, avec un composé de formule

$X_3$-$R_1$ (Vb),

dans laquelle $X_3$ représente un halogène ou un groupe alcoxy en C 1-C 4, ou un sel d'un tel composé, ou bien

e) pour préparer les composés de formule I dans laquelle $R_2$ a une signification autre que l'hydrogène, ou leurs tautomères et/ou leurs sels, partant d'un composé de formule

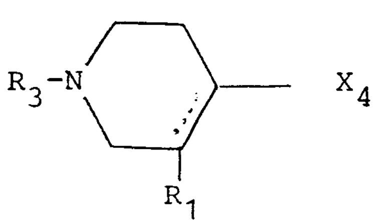

(VI),

ou d'un sel d'un tel composé, dans lequel $X_4$ représente un halogène ou un groupe diazonium de formule $-N_2^{\oplus} A^{\ominus}$ dans laquelle $A^{\ominus}$ est l'anion d'un acide minéral, on convertit $X_4$ par solvolyse en $R_2$, ou bien

f) spécialement pour préparer les composés de formule I dans laquelle $R_2$ représente l'hydrogène, ou leurs tautomères et/ou leurs sels, partant d'un composé de formule

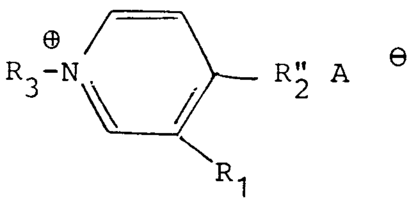

(VII),

dans laquelle $A^{\ominus}$ représente l'anion d'un acide et $R''_2$ représente l'hydrogène, on réduit les doubles liaisons en excès en liaisons simples et, dans tous les cas, on élimine un groupe protecteur éventuellement présent et dans tous les cas, si on le désire, on convertit un composé de formule I obtenu selon l'invention ou par un autre procédé en un autre composé de formule I, on sépare un mélange d'isomères obtenu conformément à l'invention en les composants, on résout un mélange d'énantiomères ou de diastéréoisomères obtenu conformément à l'invention en les énantiomères et diastéréoisomères respectivement et/ou on convertit un composé de formule I obtenu à l'état libre conformément à l'invention en un sel ou un sel obtenu conformément à l'invention en le composé libre de formule I ou en un autre sel.

**29.** Procédé pour préparer une composition pharmaceutique selon revendication 24, caractérisé en ce que l'on met la substance active, éventuellement avec adjonction de produits auxiliaires pharmaceutiques usuels, à l'état de comp osition pharmaceutique.

**30.** Procédé selon la revendication 29, pour préparer une composition pharmaceutique à activité nootrope selon revendication 26, caractérisé en ce que l'on choisit une substance active à activité nootrope.

**31.** Utilisation d'un composé selon l'une des revendications 1 à 23, ou d'un tautomère et/ou d'un sel acceptable pour l'usage pharmaceutique d'un tel composé, pour la préparation d'une composition pharmaceutique.

**32.** Utilisation selon la revendication 31, pour la préparation d'un médicament nootropique.

**33.** Utilisation d'un composé selon l'une des revendications 1 à 23, ou d'un tautomère et/ou d'un sel acceptable pour l'usage pharmaceutique d'un tel composé, pour la préparation d'une composition pharmaceutique par vole non chimique.

**Revendications pour l'Etat contractant suivant : AT**

**1.** Procédé de préparation de nouvelles hydropyridines substituées de formule

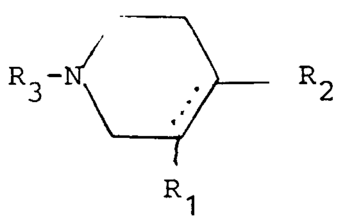

(I),

dans laquelle $R_1$ représente un groupe carboxy, (alcoxy inférieur)-carbonyle, carbamoyle, N-(alkyle inférieur)-carbamoyle ou N,N-di-(alkyle inférieur)-carbamoyle, $R_2$ représente l'hydrogène, un groupe

hydroxy, alcoxy inférieur, un groupe phényl-alcoxy inférieur éventuellement substitué par des groupes alkyle inférieurs, alcoxy inférieurs et/ou des halogènes, un groupe alcanoyloxy inférieur, benzoyloxy, pyridoyloxy, alcane-sulfonyloxy inférieur, (alcoxy inférieur)-carbonyloxy, phényl-(alcoxy inférieur)-carbonyloxy, amino, alcanoylamino inférieur, benzoylamino, pyridoylamino, alcane-sulfonylamino inférieur, (alcoxy inférieur)-carbonylamino ou phényl-(alcoxy inférieur)-carbonylamino, $R_3$ représente un groupe de formule R- (Ia), R-$alk_1$- (Ib) ou R'=$alk_2$- (Ic) dans lesquels R représente un groupe benzocycloalcényle relié par l'intermédiaire d'un atome de carbone saturé, non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle et/ou en position alpha de la partie benzo par un groupe alkyle inférieur, et qui contient au total de 8 à 12 atomes de carbone cycliques, R' représente un groupe benzocycloalcénylidène non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle, et qui contient au total de 8 à 12 atomes de carbone cycliques, $alk_1$ représente un groupe alkylène inférieur ou alkylidène inférieur, $alk_2$ un groupe (alkyle inférieur)-oméga-ylidène,
et le trait interrompu indique qu'entre les atomes de carbone portant les groupes $R_1$ et $R_2$, il y a une liaison simple ou double, les groupes qualifiés d'"inférieurs" contenant de 1 à 4 atomes de carbone inclus, et leurs tautomères et/ou leurs sels, caractérisé en ce que :

a) on fait réagir un composé de formule

$R_3$-$X_1$ (IIa)

ou un sel d'un tel composé, dans lequel $X_1$ représente un groupe hydroxy ou un groupe hydroxy estérifié réactif, avec un composé de formule

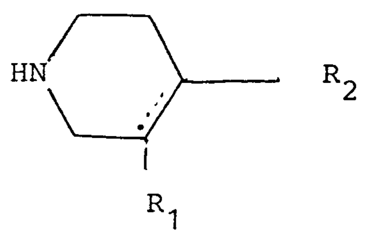

(IIb)

ou un tautomère et/ou un sel d'un tel composé, ou bien

b) dans un composé de formule

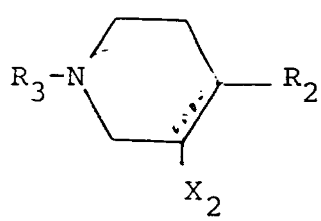

(III)

ou un tautomère et/ou un sel d'un tel composé, dans lequel $X_2$ représente un groupe cyano, halogénocarbonyle, amidino éventuellement substitué par un groupe alkyle en C 1-C 4, un groupe (alcanoyle en C 1-C 4)-oxycarbonyle, (alcoxy en C 1-C 4)-carbonyloxycarbonyle, carboximidoyle, (alcoxy en C 1-C 4)-carbimidoyle, halogénocarbimidoyle, tri-(alcoxy en C 1-C 4)-méthyle ou trihalogénométhyle, on convertit $X_2$ par solvolyse en $R_1$, ou bien

c) pour préparer les composés de formule I dans laquelle $R_2$ représente un groupe hydroxy ou amino et $R_1$ plus spécialement un groupe (alcoxy en C 1-C 4)-carbonyle, ou leurs tautomères et/ou leurs sels, on cyclise un composé de formule

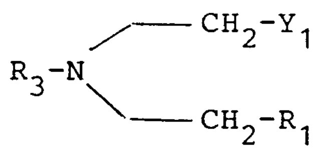

(IV),

dans laquelle $Y_1$ représente un groupe de formule $-CH=R'_2$, $-C(Y_2)=R'_2$ ou cyano et $R'_2$ représente un groupe oxo ou imino, et $Y_2$ un groupe alcoxy en C 1-C 4, ou un sel d'un tel composé, ou bien d) pour préparer les composés de formule I dans laquelle $R_2$ représente un groupe hydroxy ou amino et le trait interrompu indique qu'entre les atomes de carbone portant les groupes $R_1$ et $R_2$, il y a une double liaison, et $R_1$ représente plus spécialement un groupe (alcoxy en C 1-C 4)-carbonyle, ou leurs tautomères et/ou leurs sels, on fait réagir un composé de formule

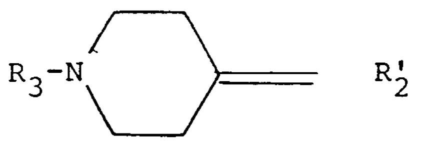

(Va),

dans laquelle $R'_2$ représente un groupe oxo ou imino, ou un tautomère ou sel d'un tel composé, avec un composé de formule

$X_3-R_1$ (Vb),

dans laquelle $X_3$ représente un halogène ou un groupe alcoxy en C 1-C 4, ou un sel d'un tel composé, ou bien
e) pour préparer les composés de formule I dans laquelle $R_2$ a une signification autre que l'hydrogène, ou leurs tautomères et/ou leurs sels, partant d'un composé de formule

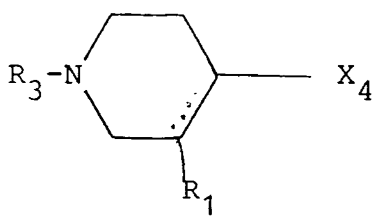

(VI),

ou d'un sel d'un tel composé, dans lequel $X_4$ représente un halogène ou un groupe diazonium de formule $-N_2{}^{\oplus} A^{\ominus}$ dans laquelle $A^{\ominus}$ est l'anion d'un acide minéral, on convertit $X_4$ par solvolyse en $R_2$, ou bien
f) spécialement pour préparer les composés de formule I dans laquelle $R_2$ représente l'hydrogène, ou leurs tautomères et/ou leurs sels, partant d'un composé de formule

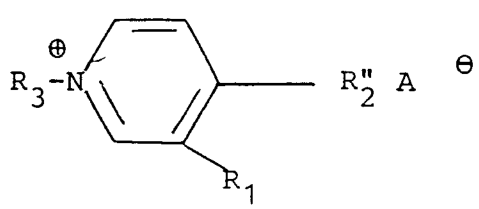

(VII),

dans laquelle $A^{\ominus}$ représente l'anion d'un acide et $R''_2$ représente l'hydrogène, on réduit les doubles liaisons en excès en liaisons simples et, dans tous les cas, on élimine un groupe protecteur éventuellement présent et dans tous les cas, si on le désire, on convertit un composé de formule I obtenu selon l'invention ou par un autre procédé en un autre composé de formule I, on sépare un

mélange d'isomères obtenu conformément à l'invention en les composants, on résout un mélange d'énantiomères ou de diastéréoisomères obtenu conformément à l'invention en les énantiomères et diastéréoisomères respectivement et/ou on convertit un composé de formule I obtenu à l'état libre conformément à l'invention en un sel ou un sel obtenu conformément à l'invention en le composé libre de formule I ou en un autre sel.

**2.** Procédé selon la revendication 1, pour préparer les composés de formule I dans laquelle $R_3$ représente un groupe Ia ou Ib, R un groupe benzocyclobutène-1-yle non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle et/ou en position alpha de la partie benzo par un groupe alkyle inférieur, $R_1$ représente un groupe carboxy, (alcoxy inférieur)-carbonyle, carbamoyle, N-(alkyle inférieur)-carbamoyle ou N,N-di-(alkyle inférieur)-carbamoyle, $R_2$ représente l'hydrogène, un groupe hydroxy, alcoxy inférieur, phényl-alcoxy inférieur, alcanoyloxy inférieur, benzoyloxy, pyridoyloxy, (alcoxy inférieur)-carbonyloxy, phényl-(alcoxy inférieur)-carbonyloxy, amino, alcanoylamino inférieur, benzoylamino, pyridoylamino, (alcoxy inférieur)-carbonylamino ou phényl-(alcoxy inférieur)-carbonylamino, $alk_1$ représente un groupe alkylène inférieur ou alkylidène inférieur et le trait interrompu indique qu'entre les atomes de carbone portant les groupes $R_1$ et $R_2$ il y a une liaison simple ou double, les groupes qualifiés d'"inférieurs" contenant chacun 1 à 4 atomes de carbone, et leurs tautomères et/ou leurs sels.

**3.** Procédé selon la revendication 1, pour préparer les composés de formule I dans laquelle $R_3$ représente un groupe Ia, Ib ou Ic, R représente un groupe benzocyclobutène-1-yle non substitué ou substitué dans la partie benzo une ou plusieurs fois par des groupes hydroxy, alcoxy inférieurs, alcanoyloxy inférieurs et/ou des halogènes et/ou en position alpha de la partie benzo par un groupe alkyle inférieur, un groupe indane-1-yle ou indane-2-yle ou 1,2,3,4-tétrahydronaphto-1-yle ou 1,2,3,4-tétrahydronaphto-2-yle, R' représente un groupe benzocyclobutène-1-ylidène non substitué ou substitué dans la partie benzo une ou plusieurs fois par des groupes hydroxy, alcoxy inférieurs, alcanoyloxy inférieurs et/ou des halogènes, $R_1$ représente un groupe carboxy, (alcoxy inférieur)-carbonyle ou carbamoyle, $R_2$ représente l'hydrogène, un groupe hydroxy, alcoxy inférieur, alcanoyloxy inférieur, alcane-sulfonyloxy inférieur, phényl-alcoxy inférieur éventuellement substitué dans la partie phényle par des groupes alkyle inférieurs, alcoxy inférieurs et/ou des halogènes, ou un groupe amino, $alk_1$ représente un groupe alkylène inférieur ou alkylidène inférieur qui relie les deux systèmes cycliques respectivement par 1 à 3 atomes de carbone inclus ou par un atome de carbone, $alk_2$ représente un groupe (alkyle inférieur)-oméga-ylidène qui relie les deux systèmes cycliques par 2 ou 3 atomes de carbone, et le trait interrompu indique qu'entre les atomes de carbone portant les groupes $R_1$ et $R_2$ il y a une liaison simple ou double, les groupes qualifiés d'"inférieurs" contenant chacun de 1 à 4 atomes de carbone inclus, et leurs tautomères et/ou leurs sels.

**4.** Procédé selon la revendication 1, pour préparer les composés de formule I dans laquelle $R_3$ représente un groupe Ia ou Ib, R représente un groupe benzocyclobutène-1-yle non substitué ou mono-substitué dans la partie benzo par un groupe alcoxy inférieur contenant jusqu'à 4 atomes de carbone inclus, alkyle inférieur contenant jusqu'à 4 atomes de carbone inclus ou un halogène de numéro atomique allant jusqu'à 35 inclus, ou di-substitué par des groupes alkyle inférieurs contenant jusqu'à 4 atomes de carbone inclus ou par un groupe alcoxy inférieur contenant jusqu'à 4 atomes de carbone inclus et un groupe alkyle inférieur contenant jusqu'à 4 atomes de carbone inclus, un groupe indane-1-yle, indane-2-yle ou 1,2,3,4-tétrahydronaphto-1-yle, $R_1$ représente un groupe (alcoxy inférieur)-carbonyle contenant 1 à 4 atomes de carbone inclus dans la partie alcoxy inférieur, ou un groupe carbamoyle, $R_2$ représente l'hydrogène ou un groupe hydroxy, $alk_1$ représente un groupe alkylène inférieur contenant jusqu'à 3 atomes de carbone inclus et qui relie les systèmes cycliques par un pont de 1 à 3 atones de carbone inclus, et le trait interrompu indique qu'entre les atomes de carbone portant les groupes $R_1$ et $R_2$ il y a une liaison simple ou double, et leurs tautomères et/ou leurs sels.

**5.** Procédé selon la revendication 1, pour préparer les composés de formule I dans laquelle $R_3$ représente un groupe de formule Ib, R représente un groupe benzocyclobutene-1-yle non substitué ou substitué dans la partie benzo par un groupe alcoxy inférieur contenant jusqu'à 4 atomes de carbone inclus, $R_1$ représente un groupe (alcoxy inférieur)-carbonyle contenant de 1 à 4 atomes de carbone inclus dans la partie alcoxy inférieur, ou un groupe carbamoyle, $R_2$ représente l'hydrogène ou un groupe hydroxy, $alk_1$ représente un groupe alkylène inférieur contenant jusqu'à 3 atomes de carbone inclus et qui relie

les systèmes cycliques par un pont de 1 à 3 atomes de carbone, et le trait interrompu indique qu'entre les atomes de carbone portant les groupes $R_1$ et $R_2$ il y a une liaison simple ou double, et leurs tautomères et/ou leurs sels.

**6.** Procédé selon revendication 1, pour préparer le 4-hydroxy-1-[-5-méthoxybenzocyclobutène-1-yl)-méthyl]-1,2,5,6-tétrahydro-pyridine-3-carboxylate d'éthyle ou 1-[(5-méthoxybenzocyclobutène-1-yl)-méthyl]-4-oxo-pipéridine-3-carboxylate d'éthyle ou un de ses sels.

**7.** Procédé selon revendication 2, pour préparer le cis-4-hydroxy-1-[(5-méthoxybenzocyclobutène-1-yl)-méthyl]-pipéridine-3-carboxylate d'éthyle ou un de ses sels.

**8.** Procédé selon revendication 2, pour préparer le trans-4-hydroxy-1-[2-(5-méthoxybenzocyclobutène-1-yl)-éthyl]-pipéridine-3-carboxylate d'éthyle ou un de ses sels.

**9.** Procédé selon revendication 2, pour préparer le 4-amino-1-[(5-méthoxybenzocyclobutène-1-yl)-méthyl]-1,2,5,6-tétrahydro-pyridine-3-carboxylate d'éthyle ou 1-[(5-méthoxybenzocyclobutène-1-yl)-méthyl]-4-imino-pipéridine-3-carboxylate d'éthyle ou un de ses sels.

**10.** Le 4-hydroxy-1-[2-(5-méthoxybenzocyclobutène-1-yl)-éthyl]-1,2,5,6-tétrahydro-pyridine-3-carboxylate de méthyle ou 1-[2-(5-méthoxybenzocyclobutène-1-yl)-éthyl]-4-oxo-pipéridine-3-carboxylate de méthyle ou un de ses sels.

**11.** Procédé selon revendication 1, pour préparer le 4-hydroxy-1-[(benzocyclobutène-1-yl)-méthyl]-1,2,5,6-tétrahydro-pyridine-3-carboxylate d'éthyle ou 1-[(benzocyclobutène-1-yl)-méthyl]-4-oxo-pipéridine-3-carboxylate d'éthyle ou un de ses sels.

**12.** Procédé selon revendication 1, pour préparer le 1-[(6-méthoxyindane-1-yl)-méthyl]-1,2,5,6-tétrahydro-pyridine-3-carboxylate de méthyle ou un de ses sels.

**13.** Procédé selon revendication 1, pour préparer le 4-hydroxy-1-[(5-méthoxy-1-méthyl-benzocyclobutène-1-yl)-méthyl]-1,2,5,6-tétrahydro-pyridine-3-carboxylate d'éthyle ou 1-[(5-méthoxy-1-méthyl-benzocyclobutène-1-yl)-méthyl]-4-oxo-pipéridine-3-carboxylate d'éthyle ou un de ses sels.

**14.** Procédé selon revendication 1, pour préparer le 4-hydroxy-1-(benzocyclobutène-1-yl)-1,2,5,6-tétrahydro-pyridine-3-carboxylate d'éthyle ou 1-(benzocyclobutène-1-yl)-4-oxo-pipéridine-3-carboxylate d'éthyle ou un de ses sels.

**15.** Procédé selon revendication 1, pour préparer le 1-[(7-méthoxy-1,2,3,4-tétrahydronaphto-2-yl)méthyl]-1,2,5,6-tétrahydro-pyridine-3-carboxylate de méthyle ou un de ses sels.

**16.** Procédé selon revendication 1, pour préparer le 4-hydroxy-1-[(4-méthoxybenzocyclobutène-1-yl)-méthyl]-1,2,5,6-tétrahydro-pyridine-3-carboxylate d'éthyle ou 1-[(4-méthoxybenzocyclobutène-1-yl)-méthyl]-4-oxo-pipéridine-3-carboxylate d'éthyle ou un de ses sels.

**17.** Procédé selon revendication 1, pour préparer le 4-hydroxy-1-[(7-méthoxy-1,2,3,4-tétrahydronaphto-2-yl)-méthyl]-1,2,5,6-tétrahydro-pyridine-3-carboxylate de méthyle ou 1-[(7-méthoxy-1,2,3,4-tétrahydronaphto-2-yl)-méthyl]-4-oxo-pipéridine-3-carboxylate de méthyle ou un de ses sels.

**18.** Procédé selon revendication 1, pour préparer le 4-hydroxy)1-(6-méthoxyindane-1-yl)-1,2,5,6-tétrahydro-pyridine-3-carboxylate d'éthyle ou 1-(6-méthoxyindane-1-yl)-4-oxo-pipéridine-3-carboxylate d'éthyle ou un de ses sels.

**19.** Procédé selon revendication 1, pour préparer le 4-hydroxy-1-(7-méthoxy-1,2,3,4-tétrahydronaphto-2-yl)-1,2,5,6-tétrahydro-pyridine-3-carboxylate de méthyle ou 1-(7-méthoxy-1,2,3,4-tétrahydronaphto-2-yl)-4-oxo-pipéridine-3-carboxylate de méthyle ou un de ses sels.

**20.** Procédé de préparation d'une composition pharmaceutique contenant en tant que substance active un composé obtenu selon une des revendications 1 à 19, ou un tautomère et/ou un sel acceptable pour l'usage pharmaceutique d'un tel composé, caractérisé en ce que l'on mélange la substance active avec des produits auxiliaires pharmaceutiques usuels.

**21.** Procédé de préparation d'une composition pharmaceutique contenant un composé obtenu selon l'une des revendications 2 et 5 à 10, ou un tautomère et/ou un sel acceptable pour l'usage pharmaceutique d'un tel composé, caractérisé en ce que l'on mélange la substance active avec des produits auxiliaires pharmaceutiques usuels.

**22.** Procédé de préparation d'une composition pharmaceutique à activité nootrope selon revendication 20 ou 21, caractérisé en ce que l'on choisit une substance active à activité nootrope.

**23.** Procédé de préparation d'une composition pharmaceutique à activité nootrope selon revendication 21, caractérisé en ce que l'on choisit une substance active à activité nootrope.

**24.** Procédé de préparation d'une composition pharmaceutique, caractérisé en ce que :

a) on fait réagir un composé de formule

$R_3$-$X_1$ (IIa)

ou un sel d'un tel composé, dans lequel $X_1$ représente un groupe hydroxy ou un groupe hydroxy estérifié réactif, avec un composé de formule

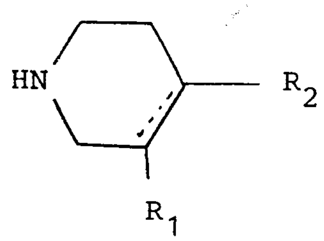

(IIb)

ou un tautomère et/ou un sel d'un tel composé, ou bien

b) dans un composé de formule

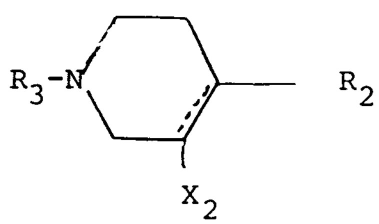

(III)

ou un tautomère et/ou un sel d'un tel composé, dans lequel $X_2$ représente un groupe cyano, halogénocarbonyle, amidino éventuellement substitué par un groupe alkyle en C 1-C 4, un groupe (alcanoyle en C 1-C 4)-oxycarbonyle, (alcoxy en C 1-C 4)-carbonyloxycarbonyle, carboximidoyle, (alcoxy en C 1-C 4)-carbimidoyle, halogénocarbimidoyle, tri-[alcoxy en C 1-C 4)-méthyle ou trihalogénométhyle, on convertit $X_2$ par solvolyse en $R_1$, ou bien

c) pour préparer les composés de formule I dans laquelle $R_2$ représente un groupe hydroxy ou amino et $R_1$ plus spécialement un groupe (alcoxy en C 1-C 4)-carbonyle, ou leurs tautomères et/ou leurs sels, on cyclise un composé de formule

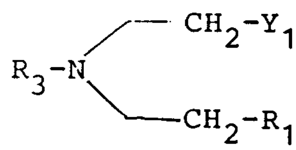 (IV)

dans laquelle $Y_1$ représente un groupe de formule $-CH=R'_2$, $-C(Y_2)=R'_2$ ou cyano et $R'_2$ représente un groupe oxo ou imino, et $Y_2$ un groupe alcoxy en C 1-C 4, ou un sel d'un tel composé, ou bien

d) pour préparer les composés de formule I dans laquelle $R_2$ représente un groupe hydroxy ou amino et le trait interrompu indique qu'entre les atomes de carbone portant les groupes $R_1$ et $R_2$, il y a une double liaison, et $R_1$ représente plus spécialement un groupe (alcoxy en C 1-C 4)-carbonyle, ou leurs tautomères et/ou leurs sels, on fait réagir un composé de formule

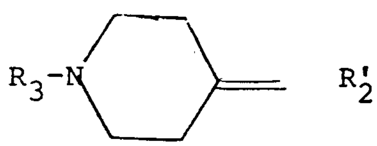 (Va)

dans laquelle $R_2'$ représente un groupe oxo ou imino, ou un tautomère ou sel d'un tel composé, avec un composé de formule

$X_3-R_1$ (Vb),

dans laquelle $X_3$ représente un halogène ou un groupe alcoxy en C 1-C 4, ou un set d'un tel composé, ou bien

e) pour préparer les composés de formule I dans laquelle $R_2$ a une signification autre que l'hydrogène, ou leurs tautomères et/ou leurs sels, partant d'un composé de formule

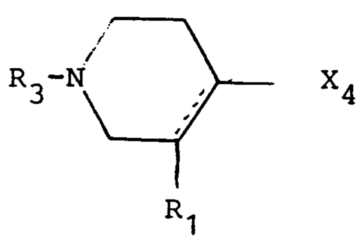 (VI),

ou d'un sel d'un tel composé, dans lequel $X_4$ représente un halogène ou un groupe diazonium de formule $-N_2^{\oplus} A^{\ominus}$ dans laquelle $A^{\ominus}$ est l'anion d'un acide minéral, on convertit $X_4$ par solvolyse en $R_2$, ou bien

f) spécialement pour préparer les composés de formule I dans laquelle $R_2$ représente l'hydrogène, ou leurs tautomères et/ou leurs sels, partant d'un composé de formule

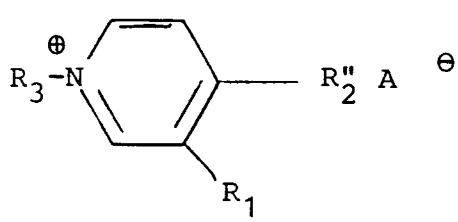 (VII),

dans laquelle $A^{\ominus}$ représente l'anion d'un acide et $R_2''$ représente l'hydrogène, on réduit les doubles liaisons en excès en liaisons simples et, dans tous les cas, on élimine un groupe protecteur

éventuellement présent et dans tous les cas, si on le désire, on convertit un composé de formule I obtenu selon l'invention ou par un autre procédé en un autre composé de formule I, on sépare un mélange d'isomères obtenu conformément à l'invention en les composants, on résout un mélange d'énantiomères ou de diastéréoisomères obtenu conformément à l'invention en les énantiomères et diastéréoisomères respectivement et/ou on convertit un composé de formule I obtenu à l'état libre conformément à l'invention en un sel ou un sel obtenu conformément à l'invention en le composé libre de formule I ou en un autre sel et on met à l'état de composition pharmaceutique, avec adjonction de produits auxiliaires pharmaceutiques usuels, une hydropyridine substituée ainsi obtenue, de formule

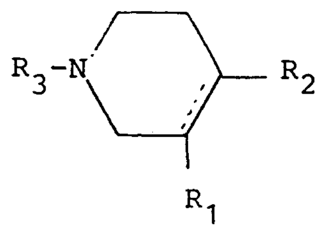

(I),

dans laquelle $R_1$ représente un groupe carboxy, (alcoxy inférieur)-carbonyle, carbamoyle, N-(alkyle inférieur)-carbamoyle ou N,N-di-(alkyle inférieur)-carbamoyle, $R_2$ représente l'hydrogène, un groupe hydroxy, alcoxy inférieur, un groupe phényl-alcoxy inférieur éventuellement substitué par des groupes alkyle inférieurs, alcoxy inférieurs et/ou des halogènes, un groupe alcanoyloxy inférieur, benzoyloxy, pyridoyloxy, alcane-sulfonyloxy inférieur, (alcoxy inférieur)-carbonyloxy, phényl-(alcoxy inférieur)-carbonyloxy, amino, alcanoylamino inférieur, benzoylamino, pyridoylamino, alcane-sulfonylamino inférieur, (alcoxy inférieur)-carbonylamino ou phényl-(alcoxy inférieur)-carbonylamino, $R_3$ représente un groupe de formule R- (Ia), R-$alk_1$- (Ib) ou R' = $alk_2$- (Ic) dans lesquels R représente un groupe benzocycloalcényle relié par l'intermédiaire d'un atome de carbone saturé, non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle et/ou en position alpha de la partie benzo par un groupe alkyle inférieur, et qui contient au total de 8 à 12 atomes de carbone cycliques, R' représente un groupe benzocycloalcénylidène non substitué ou substitué dans la partie benzo une ou plusieurs fois par des groupes hydroxy, alcoxy inférieurs, alcanoyloxy inférieurs, des halogènes, des groupes alkyle inférieurs et/ou trifluorométhyle, et qui contient au total de 8 à 12 atomes de carbone cycliques, $alk_1$ représente un groupe alkylène inférieur ou alkylidène inférieur, $alk_2$ un groupe (alkyle inférieur)-oméga-ylidène, et le trait interrompu indique qu'entre les atomes de carbone portant les groupes $R_1$ et $R_2$, il y a une liaison simple ou double, les groupes qualifiés d'"inférieurs" contenant de 1 à 4 atomes de carbone inclus, et leurs tautomères et/ou leurs sels.

**25.** Procédé selon revendication 24, pour préparer une composition pharmaceutique à activité nootrope, caractérisé en ce que l'on choisit une substance active à activité nootrope.

**26.** Utilisation d'un composé obtenu selon une dès revendications 1 à 19, ou d'un tautomère et/ou d'un sel acceptable pour l'usage pharmaceutique d'un tel composé pour la préparation d'une composition pharmaceutique.

**27.** Utilisation selon la revendication 26, pour la préparation d'un médicament nootropique.

**28.** Utilisation d'un composé obtenu selon l'une des revendications 1 à 19, ou d'un tautomère et/ou d'un sel acceptable pour l'usage pharmaceutique d'un tel composé, pour la préparation d'une composition pharmaceutique par voie non chimique.